(19) Europäisches Patentamt
European Patent Office
Office européen des brevets

(11) EP 4 108 765 A1

(12) EUROPEAN PATENT APPLICATION
published in accordance with Art. 153(4) EPC

(43) Date of publication:
**28.12.2022 Bulletin 2022/52**

(21) Application number: **21758003.4**

(22) Date of filing: **19.02.2021**

(51) International Patent Classification (IPC):
***C12N 5/0786*** *(2010.01)* ***A61K 9/10*** *(2006.01)*
***A61K 35/15*** *(2015.01)* ***A61K 35/19*** *(2015.01)*
***A61K 47/10*** *(2017.01)* ***A61K 47/20*** *(2006.01)*
***A61K 47/26*** *(2006.01)* ***A61K 47/32*** *(2006.01)*
***A61K 47/36*** *(2006.01)* ***A61P 7/00*** *(2006.01)*
***A61P 35/02*** *(2006.01)* ***C12M 1/00*** *(2006.01)*
***C12M 1/34*** *(2006.01)* ***C12M 3/00*** *(2006.01)*
***C12N 5/0784*** *(2010.01)* ***C12Q 1/04*** *(2006.01)*

(52) Cooperative Patent Classification (CPC):
**A61K 9/10; A61K 35/15; A61K 35/19; A61K 47/10; A61K 47/20; A61K 47/26; A61K 47/32; A61K 47/36; A61P 7/00; A61P 35/02; C12M 1/00; C12M 1/34; C12M 3/00; C12N 5/06; C12Q 1/04**

(86) International application number:
**PCT/JP2021/006464**

(87) International publication number:
**WO 2021/167094 (26.08.2021 Gazette 2021/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.02.2020 JP 2020027595**
**01.05.2020 JP 2020081523**

(71) Applicant: **Suehiro, Youko**
**Fukuoka-shi, Fukuoka 811-1395 (JP)**

(72) Inventor: **Suehiro, Youko**
**Fukuoka-shi, Fukuoka 811-1395 (JP)**

(74) Representative: **Engelhard, Markus**
**Boehmert & Boehmert Anwaltspartnerschaft mbB**
**Pettenkoferstrasse 22**
**80336 München (DE)**

# (54) MONOCYTE PURIFICATION METHOD IN PERIPHERAL BLOOD RAW MATERIAL COLLECTION/FREEZING-THAWING STEP

(57) The present disclosure provides the manufacturing of a monocyte that serves as a raw material for a dendritic cell for use in the manufacture of a therapeutic drug for adult T-cell leukemia and the like. The present disclosure provides: a method of producing a monocyte preparation from a blood preparation, the method comprising the step of obtaining a blood preparation from a subject, and the step of enriching the monocyte based on the specific gravity and cell size in the blood preparation; a monocyte preparation manufactured by the method; a dendritic cell induced from the monocyte preparation; and a product for regenerative medicine or the like. The present disclosure also provides treatment of an adult T-cell leukemia (ATL) patient.

EP 4 108 765 A1

## Description

[Technical Field]

The present disclosure relates to a novel mononucleosis preparation technique. More specifically, the present disclosure relates to a monocyte purification method in a peripheral blood raw material collection/freezing-thawing step. Specifically, the present disclosure provides a technique optimal in the manufacture of a dendritic cell formulation (especially a product for regenerative medicine or the like wherein a Tax antigen peptide is pulsed) suited for treatment of adult T-cell leukemia (ATL).

[Background Art]

In the general medical practice, the composition of an apheresis raw material used in hematopoietic stem cell transplantation is mainly corpuscle components which are lymphocyte, monocyte and neutrophil, red blood cell, platelet and plasma which are mixed in upon collection.

Freezing-thawing of an apheresis raw material affects each component differently, wherein especially neutrophil, lymphocytes and red blood cells face the problem of fragility due to the stress of freezing and platelets face aggregate formation by coagulation induction involved in activation, leading to loss monocytes. Therefore, upon raw material collection by apheresis, the point of collecting a raw material with high monocyte purity while avoiding these components to be mixed in as much as possible would be greatly related to the product yield and quality thereafter.

[Summary of Invention]

[Solution to Problem]

The present disclosure is related to manufacturing of a dendritic cell product (e.g., ATL-DC-101) which is an ATL treatment product, comprising a monocyte purification method in the peripheral blood mononuclear cell collection step and the specific gravity centrifugation step after thawing of a frozen raw material. The dendritic cell product of the present disclosure includes a product for regenerative medicine or the like, in which a dendritic cell has been induced from a monocyte comprised in a peripheral blood mononucleosis component (apheresis raw material) and a targeted Tax antigen peptide had been pulsed. Since the product of the present disclosure is an autologous dendritic cell product, the step of conveying a patient apheresis raw material to a manufacturing facility is caused, but in the case of a fresh rawmaterial, change of quality of the raw material due to conveyance, degradation of quality associated with conveyance time prolongation due to risks during the conveyance (traffic condition, personal accident, natural disaster and the like) and spread of HTLV-1 virus infection within the raw material would be problematic (an HTLV-1 infected cell is comprised in an ATL patient apheresis specimen) . In this regard, for popularization of treatment, it is necessary to establish a manufacturing method of freezing a patient apheresis raw material, conveying the patient apheresis raw material to a manufacturing facility and preparing a dendritic cell from the frozen raw material. If it becomes possible to attempt purification of a monocyte from a frozen raw material with the present disclosure, popularization of the treatment also becomes possible for a patient at a location remote from a manufacturing facility.

The present disclosure, provides, for example, the following items.

(Item 1)

A method of producing a monocyte preparation from a blood preparation, comprising:

1) the step of obtaining a blood preparation from a subject; and
2) the step of enriching a monocyte based on specific gravity and cell size in the blood preparation.

(Item 2)

The method of the item above, wherein the step 2) enriches a monocyte based on specific gravity and then enriches a monocyte based on cell size in the blood preparation.

(Item 3)

A method of producing a monocyte preparation from a blood preparation, comprising:

1) the step of obtaining a blood preparation with a blood cell concentration lower than a predetermined value from a subject; and
2) the step of enriching a monocyte based on specific gravity and/or cell size in the blood preparation.

(Item 4)

The method of any one of the items above, wherein the predetermined value is a value for taking a buffy coat.

(Item 5)

The method of any one of the items above, wherein the blood cell concentration is adjusted while referring to a collection preference.

(Item 6)

The method of any one of the items above, wherein the collection preference is set at a value higher than 60 - {(0.2 × white blood cell count (1000/μl)) + (0.08 × platelet count (1000/μl)}.

(Item 7)

The method of any one of the items above, wherein the collection preference is set at 60 - {(0.2 × white blood cell count (1000/μl)) + (0.08 × platelet count (1000/μl)} + 10 to 20.

(Item 8)

The method of any one of the items above, wherein a predetermined value of the collection preference is 30 to 50 (e.g., 30, 40, 50).

(Item 9)

The method of any one of the items above, wherein the collection preference is set to be higher than a predetermined value by 10 to 20.

(Item 10)

The method of any one of the items above, wherein the collection preference is set at 40 to 70 (e.g., 40, 50, 60, 70) .

(Item 11)

The method of any one of the items above, wherein the enriching step comprises causing a spillover.

(Item 12)

A method of producing a monocyte preparation from a blood preparation, comprising:

1) the step of obtaining a blood preparation from a subject; and
2) the step of enriching a monocyte based on specific gravity and/or cell size from the blood preparation, wherein the enriching step comprises causing a spillover of a lymphocyte fraction so as to remove a lymphocyte fraction.

(Item 13)

The method of any one of the items above, wherein, when a value of a treatment blood volume (reference treatment blood volume) at a time point of when a red blood cell is continuously overflowing from a chamber is 1 as a spillover, the spillover is performed with a treatment blood volume set at a value greater than a reference treatment blood volume.

(Item 14)

The method of any one of the items above, wherein the treatment blood volume is at least 1.2-fold of the reference treatment blood volume.

(Item 15)

The method of any one of the items above, characterized in that the monocyte preparation is directly obtained from a blood cell preparation obtained from the subject.

(Item 16)

The method of any one of the items above, wherein the enrichment is carried out in 5 hours or less after the blood preparation is obtained from the subject.

(Item 17)

The method of any one of the items above, wherein the separation by cell size is carried out in 3 hours or less after the separation by specific gravity.

(Item 18)

The method of any one of the items above, characterized in that the monocyte preparation comprises a monocyte with a concentration that is about 30% or greater with respect to all nucleated cells.

(Item 19)

A method of producing a monocyte preparation from a blood preparation, comprising:

1) the step of obtaining a blood preparation from a subject;
2) the step of adding a cryoprotectant agent to the blood preparation; and
3) the step of separating the blood preparation added with the cryoprotectant agent by specific gravity to obtain a fraction that is heavier than a lymphocyte.

(Item 20)

The method of any one of the items above, wherein the blood preparation had went through apheresis separation.

(Item 21)

The method of any one of the items above, wherein the cryoprotectant agent comprises a component selected from the group consisting of hydroxyethyl starch, sucrose, lactose, glucose, mannitol, trehalose, dimethyl sulfoxide (DMSO), glycerol, polyethylene glycol, propylene glycol, polyvinyl pyrrolidone, sorbitol and dextran.

(Item 22)

The method of any one of the items above, characterized in that the enrichment is carried out after cryopreserving.

(Item 23)

The method of any one of the items above, characterized in that the separation by specific gravity is centrifugal separation with a specific gravity of 1.074 to 1.076, preferably 1.075.

(Item 24)

The method of any one of the items 19 to 23, wherein the separation by specific gravity is carried out under following centrifugal separation conditions:

First centrifugal separation
225 mL centrifuge tube, 300 × g to 400 × g (e.g., 350 × g), 5 minutes, room temperature
Second to third centrifugal separations
225 mL centrifuge tube, 250 × g to 350 × g (e.g., 300 × g), 5 minutes, room temperature

(Item 25)

The method of any one of the items above, where the enrichment is carried out after carrying out the apheresis treatment of any one of the items above.

(Item 26)

A method of producing a monocyte preparation from a blood preparation, comprising:

1) the step of obtaining a monocyte preparation comprising a cryoprotectant agent from an obtained blood preparation; and
2) the step of washing the monocyte preparation with a washing liquid comprising 10% or less of a blood preservation liquid A liquid (ACD-A liquid) containing sodium citrate hydrate.

(Item 27)

The method of any one of the items above, wherein the ACD-A liquid is present at 6% or less.

(Item 28)

The method of any one of the items above, wherein the ACD-A liquid is present at 5.5%.

(Item 29)

The method of any one of the items above, comprising washing of the blood preparation with a washing liquid having a composition of 5.5% ACD-A liquid/1% human serum albumin (HSA)/RPMI-1640.

(Item 30)

The method of any one of the items above, further comprising practice of a dead cell removal treatment.

(Item 31)

The method of any one of the items above, wherein the dead cell removal treatment comprises removal of also a lymphocyte and a platelet.

(Item 32)

The method of any one of the items above, wherein the dead cell removal treatment comprises specific gravity centrifugal separation.

(Item 33)

The method of any one of the items above, characterized in that the specific gravity centrifugal separation is carried out using a density medium Iodixanol aqueous solution.

(Item 34)

The method of any one of the items above, wherein the Iodixanol aqueous solution is provided at 60 w/v%.

(Item 35)

The method of any one of the items above, wherein the enrichment is carried out with or without addition of

DNase.

(Item 36)

The method of any one of the items above, wherein the DNase comprises Pulmozyme.

(Item 37)

A method of producing a Tax-specific dendritic cell, comprising:

the step of inducing a dendritic cell from a monocyte preparation obtained with the method of any one of the items above; and
the step of pulsing a Tax antigen peptide with respect to the induced dendritic cell.

(Item 38)

A method of producing a product for regenerative medicine or the like, comprising the step of filling the Tax-specific dendritic cell produced with the method of any one of the items above that had been suspended in a cryoprotectant liquid into a container (cryo tube, cryo bag, or the like) to render a product for regenerative medicine or the like.

(Item 39)

A product for regenerative medicine or the like, which is produced with the method of any one of the items above.

(Item 40)

A system of producing a monocyte-containing substance, comprising at least one of:

an apheresis apparatus having a function of adjusting a blood cell concentration;
a means for realizing a spillover;
a size separation part that size-separates a cell;
a cryoprotectant agent feeding part that feeds a cryoprotectant agent;
a monocyte separation part that carries out separation of a monocyte by specific gravity; and
a dead cell removal means.

(Item 41)

The system of item 40, further comprising at least of characteristic of any one or more of the items above.

(Item 42)

The system of item 40 or 41, further comprising at least one of a separation part by specific gravity, a cryopreserving part that cryopreserves a cell, a thawing-washing part that carries out thawing-washing, and a specific gravity centrifugal separation part that carries out specific gravity centrifugal separation.
Furthermore, the present disclosure also provides the following items.

(Item A1)

A method of producing a monocyte preparation from a blood preparation, comprising:

1) the step of obtaining a blood preparation from a subject; and
2) the step of enriching a monocyte based on specific gravity and cell size in the blood preparation.

(Item A2)

The method of any one of the items above, wherein the step 2) enriches a monocyte based on specific gravity and then enriches a monocyte based on cell size in the blood preparation.

(Item A3)

A method of producing a monocyte preparation from a blood preparation, comprising:

1) the step of obtaining a blood preparation with a blood cell concentration lower than a predetermined value from a subject; and
2) the step of enriching a monocyte based on specific gravity and/or cell size in the blood preparation.

(Item A4)

The method of any one of the items above, wherein the predetermined value is a value for taking a buffy coat.

(Item A5)

The method of any one of the items above, wherein the blood cell concentration is adjusted while referring to a collection preference.

(Item A6)

The method of any one of the items above, wherein the collection preference is set at a value higher than 60 - {(0.2 × white blood cell count (1000/μl)) + (0.08 × platelet count (1000/μl)}.

(Item A7)

The method of any one of the items above, wherein the collection preference is set at 60 - {(0.2 × white blood cell count (1000/μl)) + (0.08 × platelet count (1000/μl)} + 10 to 20.

(Item A8)

The method of any one of the items above, wherein a predetermined value of the collection preference is 30 to 50.

(Item A9)

The method of any one of the items above, wherein the collection preference is set to be higher than a predetermined value by 10 to 30.

(Item A10)

The method of any one of the items above, wherein the collection preference is set to be higher than a predetermined value by 20 to 30.

(Item A11)

The method of any one of the items above, wherein the collection preference is set at 40 to 80 (40, 50, 60, 70, 80) .

(Item A12)

The method of any one of the items above, wherein the enriching step comprises causing a spillover.

(Item A13)

A method of producing a monocyte preparation from a blood preparation, comprising:

1) the step of obtaining a blood preparation from a subject; and
2) the step of enriching a monocyte based on specific gravity and/or cell size from the blood preparation, wherein the enriching step comprises causing a spillover of a lymphocyte fraction so as to remove a lymphocyte fraction.

(Item A14)

The method of any one of the items above, wherein, when a value of a treatment blood volume (reference treatment blood volume) at a time point of when a red blood cell is continuously overflowing from a chamber is 1 as a spillover, the spillover is performed with a treatment blood volume set at a value greater than a reference treatment blood volume.

(Item A15)

The method of any one of the items above, wherein the treatment blood volume is at least 1.2-fold of the reference treatment blood volume.

(Item A16)

The method of any one of the items above, characterized in that the monocyte preparation is directly obtained from a blood cell preparation obtained from the subject.

(Item A17)

The method of any one of the items above, wherein the enrichment is carried out in 5 hours or less after the blood preparation is obtained from the subject.

(Item A18)

The method of any one of the items above, wherein the separation by cell size is carried out in 3 hours or less after the separation by specific gravity.

(Item A19)

The method of any one of the items above, characterized in that the monocyte preparation comprises a monocyte with a concentration that is about 30% or greater with respect to all nucleated cells.

(Item A20)

A method of producing a monocyte preparation from a blood preparation, comprising:

1) the step of obtaining a blood preparation from a subject;
2) the step of adding a cryoprotectant agent to the blood preparation; and
3) the step of separating the blood preparation added with the cryoprotectant agent by specific gravity to obtain a fraction that is heavier than a lymphocyte.

(Item A21)

The method of any one of the items above, wherein the blood preparation had went through apheresis separation.

(Item A22)

The method of any one of the items above, wherein the cryoprotectant agent comprises a component selected from the group consisting of hydroxyethyl starch, sucrose, lactose, glucose, mannitol, trehalose, dimethyl sulfoxide (DMSO), glycerol, polyethylene glycol, propylene glycol, polyvinyl pyrrolidone, sorbitol and dextran.

(Item A23)

The method of any one of the items above, characterized in that the enrichment is carried out after cryopreserving.

(Item A24)

The method of any one of the items above, characterized in that the separation by specific gravity is centrifugal separation with a specific gravity of 1.074 to 1.076.

(Item A25)

The method of any one of the items above, wherein the separation by specific gravity is carried out under following centrifugal separation conditions:

First centrifugal separation
225 mL centrifuge tube, 300 $\times$ g to 400 $\times$ g, 5 minutes, room temperature
Second to third centrifugal separations
225 mL centrifuge tube, 250 $\times$ g to 350 $\times$ g, 5 minutes, room temperature

(Item A26)

The method of any one of the items above, where the enrichment is carried out after carrying out the apheresis treatment of any one of the items above.

(Item A27)

A method of producing a monocyte preparation from a blood preparation, comprising:

1) the step of obtaining a monocyte preparation comprising a cryoprotectant agent from an obtained blood preparation; and
2) the step of washing the monocyte preparation with a washing liquid comprising 10% or less of a blood preservation liquid A liquid (ACD-A liquid) containing sodium citrate hydrate.

(Item A28)

The method of any one of the items above, wherein the ACD-A liquid is present at 6% or less.

(Item A29)

The method of any one of the items above, wherein the ACD-A liquid is present at 5.5%.

(Item A30)

The method of any one of the items above, comprising washing of the blood preparation with a washing liquid having a composition of 5.5% ACD-A liquid/1% human serum albumin (HSA) /RPMI-1640.

(Item A31)

The method of any one of the items above, further comprising practice of a dead cell removal treatment.

(Item A32)

The method of any one of the items above, wherein the dead cell removal treatment comprises removal of also a lymphocyte and a platelet.

(Item A33)

The method of any one of the items above, wherein the dead cell removal treatment comprises specific gravity centrifugal separation.

(Item A34)

The method of any one of the items above, characterized in that the specific gravity centrifugal separation is carried out using a density medium Iodixanol aqueous solution.

(Item A35)

The method of any one of the items above, wherein the Iodixanol aqueous solution is provided at 60 w/v%.

(Item A36)

The method of any one of the items above, wherein the enrichment is carried out with or without addition of DNase.

(Item A37)

The method of any one of the items above, wherein the DNase comprises Pulmozyme.

(Item A38)

A method of producing a Tax-specific dendritic cell, comprising:

the step of inducing a dendritic cell from a monocyte preparation obtained with the method of any one of the items above; and
the step of pulsing a Tax antigen peptide with respect to the induced dendritic cell.

(Item A39)

A method of producing a product for regenerative medicine or the like, comprising the step of filling the Tax-specific dendritic cell produced with the method of item A38 that had been suspended in a cryoprotectant liquid into a cryo tube to render a product for regenerative medicine or the like.

(Item A40)

A product for regenerative medicine or the like, which is produced with the method of item A39.

(Item A41)

A system of producing a monocyte-containing substance, comprising at least one of:

an apheresis apparatus having a function of adjusting a blood cell concentration;
a means for realizing a spillover;
a size separation part that size-separates a cell;
a cryoprotectant agent feeding part that feeds a cryoprotectant agent;
a monocyte separation part that carries out separation of a monocyte by specific gravity; and
a dead cell removal means.

(Item A42)

The system of any one of the items above, further comprising at least one of a separation part by specific gravity, a cryopreserving part that cryopreserves a cell, a thawing-washing part that carries out thawing-washing, and a specific gravity centrifugal separation part that carries out specific gravity centrifugal separation.

(Item A43)

A method of treating an adult T-cell leukemia (ATL) patient, comprising:

1) the step of obtaining a blood preparation form the patient;

2) the step of measuring a blood cell concentration in the blood preparation; and
3) the step of obtaining a blood preparation with a blood cell concentration equivalent to a predetermined value from the patient when the blood cell concentration is lower than a predetermined value,

wherein the patient is a patient receiving apheresis in order to obtain a dendritic cell formulation for treating the ATL.

(Item A44)

The method of any one of the items above, wherein the predetermined value is a value for taking a buffy coat.

(Item A45)

The method of any one of the items above, wherein the blood cell concentration is adjusted while referring to a collection preference.

(Item A46)

The method of any one of the items above, wherein the collection preference is set at a value higher than 60 - {(0.2 × white blood cell count (1000/μl)) + (0.08 × platelet count (1000/μl)}.

(Item A47)

The method of any one of the items above, wherein the collection preference is set at 60 - {(0.2 × white blood cell count (1000/μl)) + (0.08 × platelet count (1000/μl)} + 10 to 30.

(Item A48)

The method of any one of the items above, wherein a predetermined value of the collection preference is 30 to 50.

(Item A49)

The method of any one of the items above, wherein the collection preference is set to be higher than a predetermined value by 10 to 30.

(Item A50)

The method of any one of the items above, wherein the collection preference is set to be higher than a predetermined value by 20 to 30.

(Item A51)

The method of any one of the items above, wherein the collection preference is at 40 to 80 (40, 50, 60, 70, 80).

(Item A52)

The method of any one of the items above, wherein the step of obtaining a blood preparation comprises the step of taking out a monocyte and the step of taking out a lymphocyte.

(Item A53)

The method of any one of the items above, wherein the step of taking out a monocyte comprises at least one characteristic of any one or more of items A1 to 36.

(Item A54)

The method of any one of the items above, wherein the step of taking out a lymphocyte comprises at least one characteristic of any one or more of items A43 to 51 and is performed for at least 20 minutes or longer when performed following the step of taking out a monocyte.

(Item A55)

The method of any one of the items above, wherein the step of taking out a lymphocyte is performed for 30 minutes or longer.

(Item A56)

The method of any one of the items above, wherein the step of taking out a lymphocyte is performed with the collection preference set at 30 to 50.

(Item A57)

A program that has a computer execute a method of treating an adult T-cell leukemia (ATL) patient, the method comprising:

1) the step of obtaining a blood preparation from the patient;
2) the step of measuring a blood cell concentration in the blood preparation; and
3) the step of obtaining a blood preparation with a blood cell concentration equivalent to a predetermined value from the patient when the blood cell concentration is lower than a predetermined value,

wherein the patient is a patient receiving apheresis in order to obtain a dendritic cell formulation for treating the ATL.

(Item A58)

The program of any one of the items above, wherein the predetermined value is a value for taking a buffy coat.

(Item A59)

The program of any one of the items above, wherein the blood cell concentration is adjusted while referring to a collection preference.

(Item A60)

The program of any one of the items above, wherein the collection preference is set at a value higher than 60 - {(0.2 × white blood cell count (1000/μl)) + (0.08 × platelet count (1000/μl)}.

(Item A61)

The program of any one of the items above, wherein the collection preference is set at 60 - {(0.2 × white blood cell count (1000/μl)) + (0.08 × platelet count (1000/μl)} + 10 to 30.

(Item A62)

The program of any one of the items above, wherein a predetermined value of the collection preference is 30 to 50.

(Item A63)

The program of any one of the items above, wherein the collection preference is set to be higher than a predetermined value by 10 to 30.

(Item A64)

The program of any one of the items above, the collection preference is set to be higher than a predetermined value by 20 to 30.

(Item A65)

The program of any one of the items above, wherein the collection preference is set at 40 to 80 (40, 50, 60, 70, 80).

(Item A66)

The program of any one of the items above, wherein the step of obtaining a blood preparation comprises the step of taking out a monocyte and the step of taking out a lymphocyte.

(Item A67)

The program of any one of the items above, wherein the step of taking out a monocyte comprises at least one characteristic of any one or more of items A1 to 36.

(Item A68)

The program of any one of the items above, wherein the step of taking out a lymphocyte comprises at least one characteristic of any one of items A43 to 51 and is performed for at least 20 minutes or longer when performed following the step of taking out a monocyte.

(Item A69)

The program of any one of the items above, wherein the step of taking out a lymphocyte is performed for 30 minutes or longer.

(Item A70)

The program of any one of the items above, wherein the step of taking out a lymphocyte is performed with the collection preference set at 30 to 50.

(Item A71)

A record medium storing a program that has a computer execute a method of treating an adult T-cell leukemia (ATL) patient, the method comprising:

1) the step of obtaining a blood preparation from the patient;
2) the step of measuring a blood cell concentration in the blood preparation; and
3) the step of obtaining a blood preparation with a blood cell concentration equivalent to a predetermine value from the patient when the blood cell concentration is lower than a predetermined value,

wherein the patient is a patient receiving apheresis in order to obtain a dendritic cell formulation for treating the ATL.

(Item A72)

The record medium of any one of the items above, wherein the predetermined value is a value for taking a buffy coat.

(Item A73)

The record medium of any one of the items above, wherein the blood cell concentration is adjusted while referring to a collection preference.

(Item A74)

The record medium of any one of the items above, wherein the collection preference is set at a value higher than 60 - {(0.2 × white blood cell count (1000/μl)) + (0.08 × platelet count (1000/μl)}.

(Item A75)

The record medium of any one of the items above, wherein the collection preference is set at 60 - {(0.2 × white blood cell count (1000/μl)) + (0.08 × platelet count (1000/μl)} + 10 to 30.

(Item A76)

The record medium of any one of the items above, wherein a predetermined value of the collection preference is 30 to 50.

(Item A77)

The record medium of any one of the items above, wherein the collection preference is set to be higher than a predetermined value by 10 to 30.

(Item A78)

The record medium of any one of the items above, wherein the collection preference is set to be higher than a predetermined value by 20 to 30.

(Item A79)

The record medium of any one of the items above, wherein the collection preference is set at 40 to 80 (40, 50, 60, 70, 80) .

(Item A80)

The record medium of any one of the items above, wherein the step of obtaining a blood preparation comprises the step of taking out a monocyte and the step of taking out a lymphocyte.

(Item A81)

The record medium of any one of the items above, wherein the step of taking out a monocyte comprises at least one characteristic of any one or more of items A1 to 36.

(Item A82)

The record medium of any one of the items above, wherein the step of taking out a lymphocyte comprises at least one characteristic of any one or more of items A43 to 51 and is performed for at least 20 minutes or longer when performed following the step of taking out a monocyte.

(Item A83)

The record medium of any one of the items above, wherein the step of taking out a lymphocyte is performed for 30 minutes or longer.

(Item A84)

The record medium of any one of the items above, wherein the step of taking out a lymphocyte is performed with the collection preference set at 30 to 50.

(Item A85)

A system for treating an adult T-cell leukemia (ATL) patient, comprising:

1) a preparation obtainment part that obtains a blood preparation from the patient,
2) a measurement part that measures a blood cell concentration in the blood preparation; and
3) an equivalent preparation obtainment part that obtains a blood preparation with a blood cell concentration that is

equivalent to a predetermine value from the patient when the blood cell concentration is lower than a predetermined value,

wherein the patient is a patient receiving apheresis in order to obtain a dendritic cell formulation for treating the ATL.

(Item A86)

The system of any one of the items above, wherein the predetermined value is a value for taking a buffy coat.

(Item A87)

The system of any one of the items above, wherein the blood cell concentration is adjusted while referring to a collection preference.

(Item A88)

The system of any one of the items above, wherein the collection preference is set at a value higher than 60 - {(0.2 × white blood cell count (1000/μl)) + (0.08 × platelet count (1000/μl)}.

(Item A89)

The system of any one of the items above, wherein the collection preference is set at 60 - {(0.2 × white blood cell count (1000/μl)) + (0.08 × platelet count (1000/μl)} + 10 to 30.

(Item A90)

The system of any one of the items above, wherein a predetermined value of the collection preference is 30 to 50.

(Item A91)

The system of any one of the items above, wherein the collection preference is set to be higher than a predetermined value by 10 to 30.

(Item A92)

The system of any one of the items above, wherein the collection preference is set to be higher than a predetermined value by 20 to 30.

(Item A93)

The system of any one of the items above, wherein the collection preference is set at 40 to 80 (40, 50, 60, 70, 80.

(Item A94)

The system of any one of the items above, wherein the step of obtaining a blood preparation comprises the step of taking out a monocyte and the step of taking out a lymphocyte.

(Item A95)

The system of any one of the items above, wherein the step of taking out a monocyte comprises at least one characteristic of any one or more of items A1 to 36.

(Item A96)

The system of any one of the items above, wherein the step of taking out a lymphocyte comprises at least one characteristic of any one or more of items A43 to 51 and is performed for at least 20 minutes or longer when performed following the step of taking out a monocyte.

(Item A97)

The system of any one of the items above, wherein the step of taking out a lymphocyte is performed for 30 minutes or longer.

(Item A98)

The system of any one of the items above, wherein the step of taking out a lymphocyte is performed with the collection preference set at 30 to 50.

(Item A99)

An operation method of a system for treating an adult T-cell leukemia (ATL) patient, the system comprising:

1) a preparation obtainment part that obtains a blood preparation from the patient;
2) a measurement part that measures a blood cell concentration in the blood preparation; and
3) an equivalent preparation obtainment part that obtains a blood preparation with a blood cell concentration equivalent to a predetermined value from the patient when the blood cell concentration is lower than a predetermined value,

wherein the method comprises:

1) the step of obtaining a blood preparation from the patient;
2) the step of measuring a blood cell concentration in the blood preparation; and
3) the step of obtaining a blood preparation with a blood cell concentration equivalent to a predetermined value from the patient when the blood cell concentration is lower than a predetermined value,

wherein the patient is a patient receiving apheresis in order to obtain a dendritic cell formulation for treating the ATL.

(Item A100)

The method of any one of the items above, wherein the predetermined value is a value for taking a buffy coat.

(Item A101)

The method of any one of the items above, wherein the blood cell concentration is adjusted while referring to a collection preference.

(Item A102)

The method of any one of the items above, wherein the collection preference is set at a value higher than 60 - { (0.2 × white blood cell count (1000/μl)) + (0.08 × platelet count (1000/μl)}.}

(Item A103)

The method of any one of the items above, wherein the collection preference is set at 60 - {(0.2 × white blood cell count (1000/μl)) + (0.08 × platelet count (1000/μl)}} + 10 to 30.

(Item A104)

The method of any one of the items above, wherein a predetermined value of the collection preference is 30 to 50.

(Item A105)

The method of any one of the items above, wherein the collection preference is set to be higher than a predetermined value by 10 to 30.

(Item A106)

The method of any one of the items above, wherein the collection preference is set to be higher than a predetermined value by 20 to 30.

(Item A107)

The method of any one of the items above, wherein the collection preference is set at 40 to 80 (40, 50, 60, 70, 80) .

(Item A108)

The method of any one of the items above, wherein the step of obtaining a blood preparation comprises the step of taking out a monocyte and the step of taking out a lymphocyte.

(Item A109)

The method of any one of the items above, wherein the step of taking out a monocyte comprises at least one characteristic recited in any one or more of items A1 to 36.

(Item A110)

The method of any one of the items above, wherein the step of taking out a lymphocyte comprises at least one characteristic recited in any one or more of items A43 to 51 and is performed for at least 20 minutes when performed following the step of taking out a monocyte.

(Item A111)

The method of any one of the items above, wherein the step of taking out a lymphocyte is performed for 30 minutes or longer.

(Item A112)

The method of any one of the items above, wherein the step of taking out a lymphocyte is performed with the collection preference set at 30 to 50.

In the present disclosure, the above-described one or more characteristics intend that, in addition to the explicitly shown combination, further combinations may be provided. Further embodiment and advantage of the present disclosure can be recognized by those skilled in the art by reading and understanding the following detailed explanation as needed.

[Advantageous Effects of Invention]

The present disclosure can provide an adult T-cell leukemia (ATL) treatment where significant therapeutic effect is expected as a product for regenerative medicine or the like.

[Brief Description of Drawings]

[Figure 1] Figure **1** shows a flow cytometry result of a test specimen after thawing-washing with the Pulmozyme treatment present in Example 5.

[Figure 2] Figure **2** is a result showing the quality and purity of a monocyte after the OptiPrep treatment with the Pulmozyme treatment present in Example 5.

[Figure 3] Figure **3** shows a flow cytometry result of a test specimen after thawing-washing with the Pulmozyme treatment absent in Example 5.

[Figure 4] Figure **4** is a result showing the quality and purity of a monocyte after the OptiPrep treatment with the Pulmozyme treatment absent in Example 5.

[Figure 5] Figure **5** confirms the feature of the dendritic cell (Pulmozyme present) obtained in Example 6. Figure **5** shows FCM to each marker using an antibody. Figure **5** shows FSC-H, FSC-A, SSC-A (live cell and doublet free), CD11c, CD14 and HLA-DR at a DC gate and CD11c, CD14 and HLA-DR at a lymphoid gate as shown.

[Figure 6] Figure **6** confirms the feature of the dendritic cell (Pulmozyme present) obtained in Example 6. Figure **6** shows FCM to each marker using an antibody. Figure **6** shows FSC-H, FSC-A, SSC-A (live cell and doublet free), CD11c, CD40 and CD83 at a DC gate and CD11c, CD40 and CD83 at a lymphoid gate as shown.

[Figure 7] Figure **7** confirms the feature of a dendritic cell (Pulmozyme present) obtained in Example 6. Figure **7** shows FCM to each marker using an antibody. Figure **7** shows FSC-H, FSC-A, SSC-A (live cell and doublet free), CD11c, CD80 and CD86 at a DC gate and CD11c, CD80 and CD86 at a lymphoid gate as shown.

[Figure 8] Figure **8** confirms the feature of a dendritic cell (Pulmozyme absent) obtained in Example 6. Figure **8** shows FCM to each marker using an antibody. Figure **8** shows FSC-H, FSC-A, SSC-A (live cell and doublet free), CD11c, CD14 and HLA-DR at a DC gate and CD11c, CD14 and HLA-DR at a lymphoid gate as shown.

[Figure 9] Figure **9** confirms the feature of a dendritic cell (Pulmozyme absent) obtained in Example 6. Figure **9** shows FCM to each marker using an antibody. Figure **9** shows FSC-H, FSC-A, SSC-A (live cell and doublet free), CD11c, CD40 and CD83 at a DC gate and CD11c, CD40 and CD83 at a lymphoid gate as shown.

[Figure 10] Figure **10** confirms the feature of a dendritic cell (Pulmozyme absent) obtained in Example 6. Figure **10** shows FCM to each marker using an antibody. Figure **10** shows FSC-H, FSC-A, SSC-A (live cell and doublet free), CD11c, CD80 and CD86 at a DC gate and CD11c, CD80 and CD86 at a lymphoid gate as shown.

[Figure 11] Figure **11** shows the result of MLR of Example 8. Figure **11** is a flow cytometry result (especially CD4) of preparations labeled with an anti-CD3 antibody and an anti-CD4 antibody after mixing CFSE-labeled CD4 cells (CFSE-CD4) and dendritic cells (DCs) in the proportion of CFSE-CD4:DC = 1:0, 1:0.1, 1:0.01 and 1:0.001 and carrying out single cultivation and co-cultivation. P2Exp14, P2Exp21-S and P2Exp21-P used the dendritic cell (DC) obtained in the step using a conventional method, the DC obtained in the step with the DNase treatment absent using the method of the present disclosure and the DC obtained in the step with the DNase treatment present using the method of the present disclosure, respectively.

[Figure 12] Figure **12** shows the protocol schematic diagram of Example 4.

[Figure 13] Figure **13** shows the antibody table of the antibody staining of Example 5.

[Figure 14] Figure **14** shows a schematic diagram of the manufacturing method of the present disclosure.

[Description of Embodiments]

The present disclosure is explained in further details below.

Throughout the entire specification, a singular expression should be understood as including the concept thereof in the plural form, unless specifically noted otherwise. Thus, singular articles (e.g., "a", "an", "the", and the like in the case of English) should also be understood as including the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present disclosure pertains. In case of a contradiction, the present specification (including the definitions) takes precedence.

(Definition)

The terms and general technique used in the present disclosure are explained first.

(Regarding human T-cell leukemia virus (HTLV))

A human T-cell leukemia virus (HTLV) is a retrovirus that is known as a cause of adult T-cell leukemia/lymphoma (ATL) of a human. While a typical disease caused by a type I human T-cell leukemia virus (HTLV-I) is ATL, HTLV-I may also cause HTLV-1-associated myelopathy and HTVL-1 uveitis . HTLV includes a plurality of types such as HTLV-1, HTLV-2 and HTLV-3. Among the above, HTLV-1 has a provirus gene that is approximately 9 kb in length. The provirus gene of HTLV-1 encodes various proteins such as Tax, Rex, gag, pol and env. Among the above, the Tax protein has approximately 353 amino acid residues in full length and has many functions such as activation of a transcription factor of a host such as NF-κB, SRF and CREB in a host cell and suppression of function of a protein such as p53.

Specific cells in an organism subjected infection with HTLV-1 include a CD4+ T cell, a CD8+ T cell, a monocyte and a dendritic cell. When infected with HTLV-1, these cells present a partial peptide (e.g., Tax partial peptide) of a protein such as Tax encoded by HTLV-1 via a major histocompatibility complex (MHC) . In this regard, an immunotherapy using a CD4+ T cell and a CD8+ T cell (CD8+ cytotoxic T cell; also referred to as CD8+CTL), which are cells that target a cell presenting this HTLV-1-specific partial peptide (i.e., HTLV-1 infected cell) and can provoke an immune response in an organism, has been reported as an immunotherapy that is useful for an HTLV-1-associated disease such as ATL (Japanese Laid-Open Publication No. 2008-22702, Japanese Laid-Open Publication No. 2014-133712, Miyazaki et al.,

Blood 2013 121:4894-4901 and the like).

The monocyte of the present disclosure is used to induce a dendritic cell, wherein such a dendritic cell can undergo pulse stimulation with an HTLV-I-specific cytotoxic T lymphocyte (CTL)-inducing active peptide or the like. Such a peptide is not particularly limited as long as the peptide has the activity of inducing a CTL specific to HTLV-I, wherein a Tax-specific CTL-inducing active peptide can be suitably exemplified, wherein especially Tax 301-309 which is an HLA-A24:02-restricted CTL epitope (see International Publication No. WO 2004/092373 Pamphlet) and the like can be suitably exemplified. In this regard, for example, a Tax peptide for HLA-A11:01 is one of the peptides targeted for this dendritic cell therapy, wherein the dendritic cell itself can be used for various cancers and immune control purpose by changing the target. A TCR modification type T cell targets only a peptide for HLA-A24, wherein the present disclosure includes the concept of targeting Tax 301-309. For example, the present disclosure can subject HLA-A24:01, HLA-A02:01, HLA-A02:07 and HLA-A11:01 and can utilize subject in which a dendritic cell had stimulated and activated a T cell that can attack a target in a body in an HLA-restricted manner.

It has recently been known that immunity against a cancer cell of a test subject is effectively increased by administering to the test subject a fusion peptide binding a cancer antigen epitope for a CD4+ T cell which is a helper T cell and a cancer antigen epitope for a CD8+ T cell which is a CTL (Helper/Killer-Hybrid Epitope Long peptide) (e.g., Cancer Science (2012) 103, 150-153 and the like). The present disclosure can also use a peptide of an appropriate amino acid sequence to induce HTLV-I-specific CTL together with an HTLV-I-specific CD4+ T cell and effectively increase immunity against HTLV-I. In addition, it is considered possible to manufacture a Tax pulse dendritic cell using a peptide of an appropriate amino acid sequence corresponding to Tax as a pulse.

(Dendritic cell and monocyte)

A dendritic cell (DC) is known to be a powerful antigen-presenting cell to initiate and control cellular immune response in a human. Since a dendritic cell may be either immunity-activating or immunosuppressive depending on which potential feature's set will be expressed upon interaction with a responsiveness-specific clone of a T cell, it is considered that a dendritic cell plays a very important and central role in T cell-mediated immune reaction. A broadly interpreted but widely supported general theory considers that immature dendritic cells are "tolerogenic" compared to a more mature corresponding product thereof, whereas mature dendritic cells are "immunogenic" compared to an immature precursor thereof. A dendritic cell is generated from a monocyte ex vivo and has a specific antigen, wherein the dendritic cell's ability that effectively functions in any immunological direction depends on the survival rate and vitality thereof after being put back to a patient. The balance between counteractive immunity-activating and immunosuppressive dendritic cells is considered a major determinant of both the direction and intensity of a dendritic cell-dependent therapeutic immune response.

Normal human blood generally comprises: red blood cells ("RBCs"), the most numerous and most dense cell; platelets ("PLTs"), the least numerous and least dense cell; white blood cells ("WBCs"), the largest cell with a density between the RBCs and the PLTs; and plasma. These three cell fractions separate into distinct populations during centrifugation. On average, RBCs configure approximately 99.9% of an individual's total blood cells and occupy approximately 45% of the total volume of blood within an individual, but this is well known to vary among individuals and vary within the same individual over time . An RBC serves an important function as the principal means of delivering oxygen to body tissues, but is not useful for regenerating tissue or enhancing an immune system to counteract a specific disease such as cancer. Almost all of the remainder of an individual's blood volume consists of plasma which is a non-cellular liquid occupying approximately 55% of the total blood volume, wherein all blood cells are suspended. Therefore, 99% or more of the normal blood volume consists of plasma and red blood cells.

The remaining approximately <0.6% of the normal blood volume consists of WBCs and Platelets (PLTs). PLTs are small and irregularly shaped nuclear cells that outnumber the WBCs by a factor of approximately 30 times. A PLT plays a fundamental role in hemostasis and healing by stopping bleeding and releasing many growth factors that repair and regenerate damaged tissues. However, their adhesiveness interferes with the efficient enhancement and purification of the rare and clinically important target cells. The least prevalent blood cells are WBCs, occupying only approximately one tenth of 1% of the total cells in a typical blood sample. A WBC is critical to an immune system of a body and related to the defense of the body against an infection, a foreign material and a hematologic and solid tumor cancer. Almost all of the cells that are clinically utilized in immunotherapy or regenerative medicine exist within a WBC fraction. WBCs may be further divided into subgroups. The largest and most dense subgroup is the granulocytes (GRNs), which occupies approximately 60% of all WBCs. The smaller and less dense subgroup is the mononuclear cells (MNCs), which configure the remaining approximately 40%. MNCs may further be separated into lymphocytes and monocytes, but they are collectively referred to as MNCs due to the presence of a single and round nucleus in each cell. MNCs are important constituent elements of the immune system, comprising T cells, B cells and NK cells that migrate to an infected site in body tissue and then divide and differentiate into macrophages and dendritic cells to elicit an immune response. Many cell therapies that are currently examined by clinical tests utilize a cell present in an MNC fraction. In the present

disclosure, it is preferable to further separate the monocyte therein for use as a raw material for dendritic cell induction.

As used herein, "blood preparation" can subject any blood obtained from a subject. In the case of apheresis, it is normal to fractionate the total blood. Thus, a blood preparation includes, but not limited to, total blood. A fraction prepared as a fraction that already comprises a monocyte (e.g., a monocyte-containing preparation after blood component donation) can also be used as a blood preparation in the present disclosure.

As used herein, "blood cell concentration" refers to the concentration occupied by blood cells in a certain preparation. The blood cell concentration can be expressed by the collection preference used herein, wherein it can be expressed that the blood cell concentration is low when the collection preference is high and the blood cell concentration is high when the collection preference is low.

As used herein, "collection preference" refers to a reference value used to adjust the plasma pump flow volume which affects the blood cell concentration flowing inside a collection port in an apheresis apparatus, which is an indicator for focusing on and labeling particularly the concentration occupied by red blood cells of a collected blood preparation. Correlation with a patient's white blood cell count and platelet count is required in order for blood cells to be efficiently delivered from a channel with a pump. It can be expressed that the blood cell concentration is low when the collection preference is high and the blood cell concentration is high when the collection preference is low. Normally, when a patient's blood cell count is high, the collection preference needs to be rendered smaller (the color becomes deeper) . When a patient' s blood cell count is low, the collection preference needs to be rendered larger (the color becomes lighter). Typically, the setting can be done as: collection preference = 60 - {(0.2 $\times$ white blood cell count (1000/$\mu$l)) + (0.08 $\times$ platelet count (1000/$\mu$l)}.}

As used herein, "monocyte" is a type of white blood cell which is the largest type of white blood cell, referring to a macrophage or a cell that can differentiate into dendritic cells. A monocyte has an effect as a part of the natural immunity of a vertebrate animal and also affects the process of adaptive immunity. It is considered that there are at least three types of monocytes in human blood.

As used herein, "buffy coat" refers to a portion of a blood sample that had underwent anti-coagulation treatment comprising most of the white blood cells and platelets after density gradient centrifugal separation of blood.

Herein, a preparation comprising a monocyte may be called a "monocyte preparation" . The ratio of monocytes in a nucleated cell can be utilized as an indicator of the quality of such a monocyte preparation. In a conventionally used apheresis, it is considered that the ratio of a monocyte preparation is often about 10%.

As used herein, "enrich, enrichment" refers to increase in the ratio of a specific cell (e.g., monocyte) in a certain population (e.g., nucleated cell), which is also referred to as "purify, purification". For example, in the case in which the ratio of a specific cell occupying a population which was 10% before operation is increased to 30% after the operation, such a case falls under the concept of enrichment or purification.

(Tax antigen peptide pulse dendritic cell)

One characteristic of the present disclosure is the point of providing a raw material inducing a dendritic cell from a monocyte comprised in an apheresis raw material and manufacturing a product for regenerative medicine or the like in which a target Tax antigen peptide had been pulsed. While development of a dendritic cell vaccine targeting HTLV-1 Tax for ATL is currently being carried out and is highly regarded, a tax antigen peptide pulse dendritic cell with better quality can be provided by using the monocyte provided by the present disclosure.

(Preferable embodiments)

Preferable embodiments of the present disclosure are explained below. The embodiments presented below are provided for better understanding of the present disclosure, and it is understood that the scope of the present disclosure should not be limited to the description below. Therefore, it is clear that those skilled in the art can refer to the description herein and appropriately make modifications within the scope of the present disclosure. In addition, it is understood that the following embodiments of the present disclosure can be used individually or used in combination.

(Monocyte purification and dendritic cell manufacturing)

The present disclosure provides improvement in the apheresis method (reduction of lymphocytes, neutrophils, red blood cells and platelets being mixed in) and a dendritic cell manufacturing method using a monocyte purification step in a specific gravity centrifugation method after freezing-thawing, upon performing dendritic cell manufacturing from a frozen raw material. This manufacturing method comprises the following steps.

Autologous peripheral blood mononucleosis collection method (apheresis improvement method)
Cryopreserving/conveyance of an apheresis raw material

Raw material thawing/washing/centrifugal separation method
Specific gravity centrifugal separation method (monocyte purification))
Adhesion method
Method of removing supernatant and floating cell after adhesion treatment
Dendritic cell induction
Dendritic cell maturation/activation

First, innovations in apheresis can dramatically enhance the purity of a monocyte. In addition, the blood cell size, specific gravity and survival rate right after thawing a frozen apheresis raw material would change from the raw material before being frozen. Specifically, the specific gravity of a monocyte with a wide cytoplasm would be heavier due to the effect of a cryoprotectant agent with heavy specific gravity which is stored in a cell. When a cryoprotectant agent is added, the specific gravity of a monocyte and a lymphocyte would fluctuate, which thus enables separation by separation using the specific gravity of centrifugal separation or the like as an indicator. Purification of a monocyte by centrifugal separation can be attempted by utilizing this property.

Furthermore, in association with simplification of steps and shortened working time, preparation mistakes caused by a worker can be reduced and uniformity of products can be retained. It is considered that work personnel reduction and shortened work time would lead to reduction of personnel cost for product manufacturing after being released in the market, achieving a sufficient economical effect.

The raw material collection and the dendritic cell manufacturing method using the monocyte purification method by freezing-thawing of the present disclosure can be of help in a national-wide popularization of manufacturing/sales and treatment. In order to do so, it is necessary to prepare a system of freezing the raw material of a patient apheresis specimen and conveying the raw material to a manufacturing facility and establish a method of manufacturing from a frozen raw material based on the following viewpoints. Improvement can be found in any of the following points in the present disclosure.

1) Prevention of spread of virus infection from HTLV-1 infected cell in a raw material to other non-infected cells.
2) Prevention of quality degradation of a raw material by conveyance.
3) Reduction of risk due to prolonged conveyance time: traffic condition, personal accident, natural disaster.
4) Planned adjustment of patient apheresis, conveyance and manufacturing initiation date.
5) Reduction of personnel cost/manufacturing cost.

(Apheresis)

The present disclosure provides apheresis improvement method regarding a monocyte, wherein the present disclosure typically provides an autologous peripheral blood mononucleosis collection method. In this regard, increase in the purity of a monocyte was discovered by combining separation by specific gravity and separation by size in the improvement of apheresis.

In this regard, it is possible to utilize an apparatus that separates blood cells by the combination of specific gravity and size such as the Spectra Optia provided by Terumo, which is an exemplary apparatus, in order to improve apheresis. Apheresis using the Spectra Optia is used as a blood component collecting apparatus of peripheral blood stem cell (CD34-positive cell) collection, wherein, in most cases, collection is carried out in CMNC mode that does not use secondary chambers. These secondary chambers may be used for the purpose of further reducing the red blood cells and platelets being mixed in.

One embodiment exemplifies peripheral blood mononucleosis collection or monocyte collection using a centrifugation type blood component separation apparatus (Spectra Optia: Terumo BCT).

One typical example employs the separation step with two phases, blood cell separation in accordance with the specific gravity and separation in accordance with the cell size, by a secondary chamber separation system (MNC) program of a centrifugation type blood component separation apparatus (e.g., Spectra Optia (Terumo)) in order to reduce the platelets and red blood cells being mixed in and collect a monocyte fraction with high purity. While a collection preference is set by automatic calculation by an apparatus based on donor peripheral blood information, it is possible to reduce neutrophils and red blood cells being mixed in by setting the collection preference to be higher than the automatically set value by +10 to 20. In addition, platelets being mixed in are reduced by adjusting the treatment blood volume and collection pump speed upon the chamber separation step. Furthermore, in this step, a lymphocyte is spilled over (spillover) to enable control of the lymphocyte collection rate and collection of a monocyte with higher purity. More preferably, the collection preference may be set to be higher than the automatically set value by +20 to 30. Although not wishing to be bound by any theory, there is a case in which this setting requires further lymphocyte removal, wherein, in such a case, apheresis with the automatically set preference value or apheresis with the preference value set to 30 to 50 may be added as a lymphocyte removal mode after monocyte collection. As an example, in a device to be used,

collection was carried out with the preference changed back to the automatically set value after monocyte collection. Addition of this step enables removal of lymphocytes equivalent to the number of lymphocytes that were able to be removed upon conventional collection.

As used herein, "spillover" is a state in which red blood cells are continuously overflowing from a chamber.

In one embodiment, when a value of a treatment blood volume (reference treatment blood volume) at a time point of when a red blood cell is continuously overflowing from a chamber is 1 as a spillover, the spillover may be performed with a treatment blood volume set at a value greater than a reference treatment blood volume.

In one embodiment, the treatment blood volume can be set to be, but not limited to, at least 1.1-fold, at least 1.2-fold, at least 1.3-fold, at least 1.4-fold, at least 1.5-fold, at least 1.6-fold, at least 1.7-fold, at least 1.8-fold, at least 1.9-fold, at least 2.0-fold, at least 3-fold, at least 4-fold, or the like of the reference treatment blood volume.

While an appropriate condition for a spillover can be set, it is preferable to carry out the setting so as to decrease the platelets being mixed in and suppress the neutrophils being mixed in for treatment blood volume setting. Although not wishing to be bound by any theory, since a layer higher than a normal treatment would be collected, the number of collections is considered to be 1 to 2 times. In the case of dividing into two times, it is exemplified, but not limited to, that the entire treatment blood volume is calculated as 7200 ml with the blood collection flow volume of 40 ml/min × 180 minutes, and when it is calculated to be carried out with two collections, the first time would be about 60% which is 4300 ml and the second time would be the remaining 40% which is about 2900 ml.

The characteristic is the point of combining apheresis that carries out separation with specific gravity and separation by specific gravity and size. This enables isolation of a monocyte with high purity.

Specific gravity: platelet < monocyte < lymphocyte < neutrophil < red blood cell
Size: platelet < red blood cell < mononucleose (lymphocyte < monocyte)

The method of the present disclosure typically comprises the following.

- Carry out size separation using a chamber of Spectra Optia.
- Spectra Optia is mostly used in cell collection for hematopoietic stem cell transplantation and separation is not carried out using a chamber.
- In the case of the apheresis conventionally carried out by the present inventors (separation of specific gravity alone), the ratio of monocytes in a preparation is about 10 to 15%, whereas when size separation is also carried out in addition to specific gravity separation using the chamber of Spectra Optia, the ratio elevates to about 30% (concentration in a collection bag; Mo (%) = monocyte count/nucleated cell count × 100).

In addition, the ratio of platelets and red blood cells would be less, which is advantageous upon formulation.

- It is possible to achieve a layer with many monocytes and less neutrophils and red blood cells being mixed in by setting the collection preference at a value that is higher than the automatically set value (set value for collecting a buffy coat) by 10 to 20. A specific calculation method of an automatically set value is determined by 60 - { (0.2 × white blood cell count) + (0.08 × platelet count)}} and determined by the permeability of light. In this regard, the white blood cell count refers to the white blood cell count (× 1000/μL) in a subject and the platelet count refers to the platelet count (× 1000/μL) in the subject. When the collection preference is low, a layer with high specific gravity would be collected, and when the collection preference is high, a layer with low specific gravity would be collected.
- A chamber may be spilled over so as not to collect a small size cell. In a typical example, it may be desirable to achieve a spillover by treating blood that is 1.2-fold of the blood treatment volume at the time point of when red blood cells are continuously overflowing from the chamber as a spillover.

Accordingly, the present disclosure provides a method of producing a monocyte preparation from a blood preparation, comprising:

1) the step of obtaining a blood preparation from a subject; and
2) the step of enriching a monocyte based on the specific gravity and cell size in the blood preparation. In this regard, the characteristic is preferably the point that the blood preparation of a portion where the blood cell concentration is lower than normal is exposed to the enriching step by setting the collection preference to be higher than normal.

In a preferable embodiment, step **2**) enriches a monocyte based on specific gravity and then enriches a monocyte based on cell size regarding the blood preparation. An apparatus realizing this includes Spectra Optia (registered).

In another aspect, the present disclosure provides a method of producing a monocyte preparation from a blood preparation, comprising 1) the step of obtaining a blood preparation from a subject and 2) the step of enriching a monocyte

based on specific gravity and/or cell size from a portion where a blood cell concentration is lower than a predetermined value in the blood preparation.

Preferably, the blood cell concentration is adjusted while referring to a collection preference. The collection preference is set at 60 - {(0.2 $\times$ white blood cell count) + (0.08 $\times$ platelet count)}} (the white blood cell count and platelet count are $\times 1000/\mu l$). Preferably, the actual set collection preference is higher than this value.

The collection preference is set to be higher than a predetermined value by 10 to 20. The predetermined value is a value for taking a buffy coat (e.g., default 30 to 50) and the collection preference is set at 60 - {(0.2 $\times$ white blood cell count) + (0.08 $\times$ platelet count)}} + 10 to 20. The collection preference may be set at 40 to 70.

In one embodiment, spilling over is comprised in the enrichment step. Extra platelets and lymphocytes may be removed by a spillover. In an exemplary embodiment, for example, when a value of a treatment blood volume (reference treatment blood volume) at a time point of when a red blood cell is continuously overflowing from a chamber is 1 as a spillover, the spillover is performed with a treatment blood volume set at a value greater than a reference treatment blood volume. In some embodiments, the treatment blood volume is at least 1.2-fold of the reference treatment blood volume.

In one embodiment, a spillover is carried out under the condition in which a small size cell would preferentially overflow. In a specific embodiment, a spillover is carried out with a means for realizing a spillover. In some embodiments, the means for realizing a spillover has a red blood cell detector.

In one aspect, the present disclosure provides a method of producing a monocyte preparation from a blood preparation, comprising: 1) the step of obtaining a blood preparation with a blood cell concentration that is lower than a predetermined value from a subject; and 2) the step of enriching a monocyte based on specific gravity and/or cell size in the blood preparation.

In one embodiment, the monocyte preparation is characterized by being directly obtained from a blood cell preparation obtained from the subject, wherein the enrichment is carried out in 7 hours or less, in 6 hours or less, in 5 hours or less, in 4 hours or less, preferably in 3 hours or less after obtaining the blood preparation from the subject.

As another characteristic, it is preferable that separation by cell size is carried out in 5 hours or less, in 4 hours or less, or in 3 hours or less, in 2 hours or less, in 1 hour or less after the separation by specific gravity.

The monocyte preparation may comprise a monocyte with the purity that is about 10% or greater, about 20% or greater, or about 30% or greater (proportion relative to a nucleated cell) . The point that a monocyte with the purity of about 40% or greater, about 50% or greater, about 60% or greater, about 70% or greater may be comprised is also one of the characteristics of the preset disclosure.

(Cryoprotectant agent)

In one aspect, the present disclosure provides a method of producing a monocyte preparation from a blood preparation, comprising 1) the step of obtaining a blood preparation from a subject, 2) the step of adding a cryoprotectant agent to the blood preparation, and 3) the step of separating the blood preparation added with a cryoprotectant agent by specific gravity to obtain a fraction that is heavier than a lymphocyte. In one embodiment, the blood preparation had went through apheresis separation.

Typically, a cryoprotectant agent can include an agent comprising components such as hydroxyethyl starch, sucrose, lactose, glucose, mannitol, trehalose, dimethyl sulfoxide (DMSO), glycerol, polyethylene glycol, propylene glycol, polyvinyl pyrrolidone, sorbitol and dextran, for example, ReproCryo DMSO Free RM (ReproCELL), STEM-CELLBANKER(registered trademark) DMSO Free GMP Grade (Clontech) and the like. Specifically, other than an agent comprising DMSO, an agent not comprising DMSO is also preferably used.

In one aspect, the present disclosure provides a method of producing a monocyte preparation from a blood preparation, comprising 1) the step of obtaining a monocyte preparation comprising a cryoprotectant agent from an obtained blood preparation and 2) the step of washing the monocyte preparation with a washing liquid comprising a blood preservation agent A (ACD-A liquid) containing sodium citrate hydrate or the like at a concentration that is lower than normal (about 15%), which is, for example, 10% or less. This is because it was unexpectedly discovered that use with a normal concentration of ACD-A would degrade the monocyte preparation, which was able to be solved by comprising ACD-A at a concentration that is lower than normal (about 15%), which is, for example 10% or less. Preferably, it is desirable to comprise at about 9% or less, about 8% or less, about 7% or less, or about 6% or less. In one preferable embodiment, it may be desirable to comprise ACD-A at about 5.5%.

In another aspect, the present disclosure provides a method of producing a monocyte preparation from a blood preparation, comprising 1) the step of obtaining a monocyte preparation from an obtained blood preparation and 2) the step of adding a cryoprotectant agent to the monocyte preparation and then washing with a washing liquid comprising ACD-A at a concentration lower than normal (about 15%), which is, for example, 10% or less.

In other aspects, the present disclosure provides a method of producing a monocyte preparation from a blood preparation, comprising 1) the step of obtaining a monocyte preparation from an obtained blood preparation and 2) the

step of cryopreserving the monocyte preparation and then thawing-washing with a washing liquid comprising ACD-A at a concentration that is lower than normal (about 15%), which is, for example, 10% or less.

In a preferable embodiment, the enrichment is carried out after the cryopreserving. This is because the survival rate would be significantly improved by carrying out the cryopreserving. Specifically, as one example, a blood preparation is added with the cryoprotectant agent and then washed with a washing liquid having the composition of 5.5% ACD-A/1% HSA/RPMI-1640. As another example, a blood preparation undergoes the cryopreserving and then undergoes thawing-washing with a liquid having the composition of 5.5% ACD-A/1% HSA/RPMI-1640. It is preferable that ACD-A is present at 6% or less, and it is further preferable that ACD-A is present at 5.5%.

Separation of a monocyte by specific gravity may be any kind as long as there is the function of being able to separate a monocyte by the difference in specific gravity. For example, the case of washing by centrifugal separation for the purpose of washing a cell, the case of carrying out specific gravity separation using a specific gravity liquid and the like can be thought of, and these are included. In one specific embodiment, the enrichment step by specific gravity of the present disclosure may comprise a step by a specific gravity centrifugal separation method. In the specific gravity centrifugal separation method, it is preferable to carry out the centrifugal separation with a specific gravity liquid of 1.074 to 1.076, preferably 1.075. The specific gravity is not the normal 1.070 to 1.073 but slightly higher due to the unexpected effect by a cryoprotectant agent. In addition, 1.077 or greater would be not preferable since it is found that a lymphocyte would be mixed in.

In another embodiment, separation by specific gravity is carried out under the following centrifugal separation condition.

First centrifugal separation
225 mL centrifuge tube, 300 × g to 400 × g, for example, 350 × g, 5 min, RT
Second to third centrifugal separations
225 mL centrifuge tube, 250 × g to 350 × g, for example, 300 × g, 5 min, RT

In one preferable embodiment, the enrichment is carried out after carrying out the apheresis treatment of the present disclosure.

1) Washing liquid composition and 2) centrifugation condition of the thawing-washing step of a frozen raw material are one of the important conditions.

1) Washing liquid composition
5.5% ACD-A/1% HSA/RPMI-1640
2) Centrifugation condition:

First centrifugation
225 mL centrifuge tube, 350g, 5 min, RT
Second to third centrifugations
225 mL centrifuge tube, 300g, 5 min, RT.

These conditions of the first and second times may be conditions that are unexpectedly advantageous for removing a lymphocyte and efficiently removing other blood components. Centrifugal separation is normally carried out at a gravity acceleration higher than this (e.g., 500 × g, 700 × g), but such a high gravity acceleration cannot achieve sufficient removal.

(Removal of dead cell and the like)

The present disclosure may comprise the step of further removal such as a lymphocyte, platelet and dead cell removal. Dead cell removal treatment alone, or removal of a dead cell, lymphocyte and platelet may be carried out. Such cell removal may be carried out by specific gravity centrifugal separation, wherein the specific gravity centrifugal separation is preferably carried out using a density medium Iodixanol aqueous solution, wherein more preferably the Iodixanol aqueous solution is provided at 60 w/v%, wherein OptiPrep™ can be used. In one specific embodiment, the removal of dead cell and the like of the present disclosure may comprise a step by the specific gravity centrifugal separation method. In the specific gravity centrifugal separation method, it is preferable to carry out centrifugal separation with a specific gravity liquid of 1.074 to 1.076, preferably 1.075. The specific gravity is not the normal 1.070 to 1.073 but slightly higher due to the unexpected effect by a cryoprotectant agent. In addition, 1.077 or greater would be not preferable since it is found that a lymphocyte would be mixed in.

(Presence/absence of DNase)

The enrichment is carried out with or without adding DNase. The DNase comprises Pulmozyme. Since the quality and purity of a monocyte was able to be secured without a DNase treatment, it has been proven that application is possible even in the case of absence in an actual clinical scene. Of course, since DNase has preferable aspects in various sides, it is understood that the present disclosure does not exclude either of these cases.

(System)

In one aspect, the present disclosure provides a system of producing a monocyte-containing substance comprising at least one of: an apheresis apparatus having a function of adjusting a blood cell concentration; a size separation part that size-separates a cell; a means for realizing a spillover; a cryoprotectant agent feeding part that feeds a cryoprotectant agent; a monocyte separation part that carries out separation of a monocyte by specific gravity; and a means for dead cell removal. The system of the present disclosure comprises two, three, four, or all of these.

The apheresis apparatus of the system of the present disclosure has, preferably, but not limited to, the function of cell size separation other than specific gravity separation. It is understood that the function of size separation may be provided separately. Such an example includes, but not limited to, Spectra Optia (Terumo) . In addition, an apheresis apparatus may have the function of adjusting blood cell concentration by itself, and may comprise a portion that exerts the function of having the function of adjusting blood cell concentration additionally.

The means for realizing a spillover may be any kind as long as the liquid volume can be controlled.

The size separation part that size-separates a cell may be any kind as long as it is possible to separate a cell by size.

The cryoprotectant agent feeding part that feeds a cryoprotectant agent may be any kind as long as there is the function of being able to store a cryoprotectant agent and appropriately feed the cryoprotectant agent to a preparation.

The monocyte separation part that separates a monocyte by specific gravity may be any kind as long as there is the function of being able to separate a monocyte by the difference in specific gravity. For example, the case of washing by centrifugal separation for the purpose of washing a cell, the case of carrying out specific gravity separation using a specific gravity liquid and the like can be thought of, and these are comprised. When specific gravity is changed by a cryoprotectant agent, in the separation of a monocyte, the purpose is also achieved by washing by centrifugal separation for the purpose of normal washing. Thus, a centrifugal separation part apparatus may be employed in this case. When specific gravity separation is carried out using a specific gravity liquid, the specific gravity liquid may also be comprised in this monocyte separation unit in addition to a centrifugal separation device.

It is appropriate that the means for dead cell removal is any means that can remove a dead cell, which normally uses specific gravity centrifugal separation, wherein, for example, a means using a density medium Iodixanol aqueous solution is used. It is preferable that the Iodixanol aqueous solution is provided at 60 w/v%.

This system may further comprise one or more or all of a separation part by specific gravity, a cryopreserving part that cryopreserves a cell, a thawing-washing part that carries out thawing-washing or cell thawing part that causes thawing of a frozen cell, a specific gravity centrifugal separation part that carries out the specific gravity centrifugal separation discussed above, a means for inducing a dendritic cell, a means for pulse stimulation, a means for preparing a product for regenerative medicine or the like, and the like.

These systems may be automatically controlled by a computer or be manually controlled.

The system may comprise a program for having a computer execute the method described herein, and, for example, a record medium storing such a program may be comprised. In addition, a calculation apparatus (e.g., computer) for executing an order instructed by a program may be comprised. A calculation apparatus may consist of a plurality of constituent elements that are physically unified, or physically separated. A function corresponding to a learning part, calculation part, obtainment part and the like may be optionally comprised inside these calculation apparatuses.

The system of the present disclosure can be realized as an ultra-multifunctional LSI that was manufactured by integrating a plurality of configuration parts on one chip, which specifically may be a computer system configured by comprising a microprocessor, ROM (Read Only Memory), RAM (Random Access Memory) and the like. A computer program is stored in a ROM. System LSI achieves the function thereof by the microprocessor operating in accordance with the computer program.

While system LSI is employed herein, it may be called IC, LSI, super-LSI, or ultra-LSI based on the difference in the integration degree. In addition, a method of circuit integration is not limited to LSI and may be realized by a dedicated circuit or general purpose processor. It is possible to utilize a reconfigurable processor that can reconfigure the connection or setting of a circuit cell inside LSI or FPGA (Field Programmable Gate Array) that can be programmed after LSI manufacturing. Furthermore, if the technology of circuit integration, which would replace LSI by an improved semiconductor technology or another derived technology, appears, the technology may naturally be used to carry out integration of function blocks. Application of biotechnology or the like may be a possibility.

In addition, in each of the above-mentioned embodiments, each constituent element may be realized by configuring each constituent element with a dedicated hardware, or executing a software program suitable for each constituent element. Each constituent element may be realized by a program execution part such as a CPU or a processor reading and executing a software program recorded in a record medium such as a hard disk or a semiconductor memory.

As used herein, "program" is used as the normal meaning used in the subject field, wherein treatments to be carried out by a computer are described in order, which is legally handled as a "product". All computers operate in accordance with a program. In computers today, a program is expressed as data and stored in a record medium or storage apparatus.

As used herein, "record medium" is a record medium storing a program that is caused to execute the present disclosure, wherein the record medium may be any kind as long as a program can be recorded. For example, the record medium may be, but not limited to, a ROM or HDD that may be stored inside, a magnetic disk, or an external storage apparatus such as a flash memory such as a USB memory.

As used herein, "system" refers to a configuration that executes the method or program of the present invention, which originally refers to a scheme or organization for achieving a purpose, wherein a plurality of elements are systematically configured and affect one another, which, in the field of computers, refers to the entire configuration of hardware, software, OS, network and the like.

(Manufacturing of Tax antigen peptide pulse dendritic cell)

The present disclosure provides a method of producing a Tax-specific dendritic cell, comprising the step of inducing a dendritic cell from a monocyte preparation that has been prepared by the step of the present disclosure and the step of pulsing a Tax antigen peptide relative to the induced dendritic cell.

The present disclosure also provides a method of producing a product for regenerative medicine or the like, comprising the step of filling a Tax-specific dendritic cell that had been suspended in a cryoprotectant liquid into a container such as a cryo tube or a cryo bag to render a product for regenerative medicine or the like.

In addition, the present disclosure provides a product for regenerative medicine or the like manufactured by the method of the present disclosure.

(Treatment)

The product for regenerative medicine or the like of the present disclosure can be administered in a treatment at an appropriate position based on effective component volume conversion, wherein the same conversion is also possible in the case of non-oral, local or transmucosal, for example, nasal, transrectal or transdermal, or local administration. The volume of the pharmaceutical composition administered with the method of the present disclosure is determined in accordance with the subject to be treated, severity of a disorder or a symptom of a disorder, administration method, administration frequency and judgement by a physician. The administration frequency is within the range from administration of almost every hour to administration of every month. In a specific embodiment, the administration may be carried out with, for example, $1 \times 10^6$ to $1 \times 10^7$, preferably $5 \times 10^6$ cells, the number of administration may be typically 1 to 5 times (preferably 3 times), and regarding the administration interval, administration may be carried out, for example, every 1 to 4 weeks, preferably every 2 weeks. Typically, administration may be carried out 3 times in every 2 weeks with $5\times10^6$ cells. This treatment may also be used for prevention.

In a preferable embodiment, the present disclosure may comprise removal of a lymphocyte by apheresis after collecting a monocyte from a subject. The lymphocyte removal of the present disclosure removes a lymphocyte in a subject by carrying out apheresis at an automatically set value and induces activation of antitumor immunity after monocyte collection by apheresis or the like. A similar technique includes therapeutic cytapheresis such as a lymphocyte removal therapy using a centrifugal separation method, white blood cell removal therapy using an adsorption method, granulocyte removal therapy and the like, wherein any of these can be employed. This technique also mainly has the side of cytotoxic activation neutrophil component removal for the purpose of inflammation suppression. As a result of the removal of an activation neutrophil, it is possible to obtain reduction of inflammatory cytokine. In addition, the apheresis upon the raw material collection of the present disclosure and the apheresis for lymphocyte removal thereafter can simultaneously remove a lymphocyte at a $10^9$ cell level upon collecting a monocyte that is a raw material of a dendritic cell with the centrifugal separation method. As a result, it is possible to remove a lymphocyte such as Treg that configures the immunotolerant state of a cancer patient and induce activation of antitumor immunity. Such therapy that intends a certain sense of combined use is also within the scope of the present disclosure.

In one aspect, the present disclosure provides a method of treating an adult T-cell leukemia (ATL) patient, comprising:

1) the step of obtaining a blood preparation from a subj ect

2) the step of measuring a blood cell concentration in the blood preparation; and
3) the step of obtaining a blood preparation with a blood cell concentration equivalent to a predetermined value from a subject when the blood cell concentration is lower than a predetermined value,

wherein the patient is a patient receiving apheresis in order to obtain a dendritic cell formulation for treating the ATL. This treatment may also be used for prevention.

In some embodiments, the predetermined value may be a value for taking a buffy coat. In some embodiments, the blood cell concentration may be adjusted while referring to a collection preference. In some embodiments, the collection preference is set at a value higher than 60 - {(0.2 × white blood cell count (1000/μl)) + (0.08 × platelet count (1000/μl). In some embodiments, the collection preference is set at 60 - {(0.2 × white blood cell count (1000/μl)) + (0.08 × platelet count (1000/μl)} + 10 to 20. In some embodiments, a predetermined value of the collection preference is 30 to 50. In some embodiments, the collection preference is set to be higher than a predetermined value by 10 to 30. In a preferable embodiment, the collection preference is set to be higher than a predetermined value by 20 to 30. In some embodiments, the collection preference may be set at 40 to 80 (or the lower limit may be a value in the middle such as 50, 60, or the like, and the upper limit may be a value in the middle such as 60, 70, or the like).

In another embodiment, in the method of the present disclosure, the step of obtaining a blood preparation comprises the step of taking out a monocyte and the step of taking out a lymphocyte. While the step of taking out a lymphocyte may be continued for any amount of time, it is advantageous to carry out the step preferably for 20 minutes or longer, more preferably for 30 minutes or longer. In some embodiments, the step of taking out a lymphocyte may be performed with the collection preference set at 30 to 50.

Alternatively, in another embodiment, in the method of the present disclosure, the step of taking out a monocyte may have any of the characteristics regarding the treatment of a monocyte described herein or a combination thereof.

In another aspect, the present disclosure provides a program that has a computer execute a method of treating an adult T-cell leukemia (ATL) patient, the method comprising 1) the step of obtaining a blood preparation from the patient, 2) the step of measuring a blood cell concentration in the blood preparation and 3) the step of obtaining a blood preparation with a blood cell concentration equivalent to a predetermined value from the patient when the blood cell concentration is lower than a predetermined value, wherein the patient is a patient receiving apheresis in order to obtain a dendritic cell formulation for treating the ATL. The method used herein may comprise any of the further characteristics described in other parts herein or a combination thereof.

In yet another aspect, the present disclosure provides a record medium that records a program that has a computer execute a method of treating an adult T-cell leukemia (ATL) patient, the method comprising 1) the step of obtaining a blood preparation from the patient, 2) the step of measuring a blood cell concentration in the blood preparation and 3) the step of obtaining a blood preparation with a blood cell concentration equivalent to a predetermined value from the patient when the blood cell concentration is lower than a predetermined value, wherein the patient is a patient receiving apheresis in order to obtain a dendritic cell formulation for treating the ATL. The method used herein may comprise any of the further characteristics described in other parts herein or a combination thereof.

In yet another aspect, the present disclosure provides a system for treating an adult T-cell leukemia (ATL) patient, comprising 1) a preparation obtainment part that obtains a blood preparation from the patient, 2) a measurement part that measures a blood cell concentration in the blood preparation and 3) an equivalent preparation obtainment part that obtains a blood preparation with a blood cell concentration equivalent to a predetermined value from the patient when the blood cell concentration is lower than a predetermined value, wherein the patient is a patient receiving apheresis in order to obtain a dendritic cell formulation for treating the ATL. Various portions or constituent elements employed herein may comprise any of the further characteristics described in other parts herein or a combination thereof.

In yet another aspect, the present disclosure provides an operation method of a system for treating an adult T-cell leukemia (ATL) patient, the system comprising 1) a preparation obtainment part that obtains a blood preparation from the patient, 2) a measurement part that measures a blood cell concentration in the blood preparation and 3) an equivalent preparation obtainment part that obtains a blood preparation with a blood cell concentration equivalent to a predetermined value from the patient when the blood cell concentration is lower than a predetermined value, and the method comprising 1) the step of obtaining a blood preparation from the patient, 2) the step of measuring a blood cell concentration in the blood preparation and 3) the step of obtaining a blood preparation with a blood cell concentration equivalent to a predetermined value from the patient when the blood cell concentration is lower than a predetermined value, wherein the patient is a patient receiving apheresis in order to obtain a dendritic cell formulation for treating the ATL. Various portions, constituent elements or methods employed herein may comprise any of the further characteristics described in other parts herein or a combination thereof.

(Other embodiments)

The determination method and analysis method regarding one or more forms of the present disclosure have

been explained based on an embodiment, but the present disclosure is not limited to this embodiment. Various modifications applied to the present embodiments and embodiments constructed by combining constituent elements in different embodiments that are conceivable to those skilled in the art are also included within the scope of one or more embodiments of the present disclosure as long as such embodiments do not deviate from the intent of the present disclosure.

As used herein, "or" is used when "at least one or more" of the listed matters in the sentence can be employed. When explicitly described herein as "within the range of two values", the range also includes the two values themselves.

As described above, the present disclosure has been described while showing preferred embodiments to facilitate understanding. The present disclosure is described hereinafter based on Examples. The above descriptions and the following Examples are not provided to limit the present disclosure, but for the sole purpose of exemplification. Thus, the scope of the present disclosure is not limited to the embodiments or the Examples specifically described herein and is limited only by the scope of claims.

[Examples]

The present Examples explain the method of purification of a monocyte of the present disclosure and a series of experimentation of inducing dendritic cell, carrying out Tax peptide pulse and formulating thereafter, wherein detailed discussion is provided regarding characteristic purification of a monocyte. The following Examples were carried out based on the approval of the ethics committee where the inventors belong while observing the medical ethics codes such as the Declaration of Helsinki, regulations such as GCP and regulations set forth by other national hospital organizations. The following experimentations were carried out based on the obtainment of necessary informed consent.

Figure **14** shows a schematic diagram of a typical method of monocyte purification examined in the present Examples, wherein it is understood that some (e.g., freezing-thawing and specific gravity centrifugal separation) are optional steps that may be omitted.

(Example 1: Performing apheresis)

In this Example, improvement of apheresis was attempted. The procedure is shown below.

(Protocol)

- Upon performing the apheresis, the health condition of a donor was considered first and the same medical measures as the general clinical treatment were carried out.
- Blood was collected from the donor beforehand to measure blood count and machine classification.
- Spectra Optia (Terumo BCT) was used as a device.
- MNC program was employed as a program.
- 2 routes of peripheral intravenous paths were secured for blood removal and blood return.
- The following conditions were set as collection conditions.

Blood collection flow volume
40 ml/h or less
Plasma volume
Adjust so that the final specimen would be about 200 ml
Collected blood volume : ACD-A liquid volume ratio 12:1
Collection preference

Automatically set value + 10 to 20
*Automatically set value = 60-(0.2 $\times$ white blood cell count {10^3/$\mu$l}) - (0.08 $\times$ platelet count {10^3/$\mu$l}) $\approx$ 30

Total blood treatment volume
treatment volume of red blood cell detection about 7200 ml
•After termination of the collection, about 1 ml of sample is collected from a collected specimen to measure blood count and machine classification.

(Freezing treatment)

<Prepared articles>

Specimen (PBMC collection bag)
Double separation bag (Kawasumi blood separation bag KBP-66DC)

1 Kocher, tube sealer, roller bench
Aseptic joining device (TSCD)
ACD-A liquid
500 mL (the used volume is little volume)
Blood donation albumin 20% IV 10 g/50 mL(JB)
1 CP-1 40 ml
1 (Seiko)
Freeze bag (Nipro frozen bag F-100)
1
Ethanol spray
Yellow gown, hat
PBMC freezing step record document.

(1. Transfer)

(1) Transfer to a double bag

1. Before opening a plastic bag, a needle portion of a double separation bag was clamped with a kocher over the bag, the plastic bag was opened and cut off at a portion close to the needle with a tube sealer.
2. A klemme was used for clamping between the primary bag and the subsidiary bag of the double separation bag.
3. The PBMC collection bag clamped with a kocher and the double separation bag were joined with TSCD.
4. The PBMC liquid in the collection bag was transferred to the double separation bag.
5. A tube sealer was used for sealing (the tube was left long) to cut off the collection bag.

It was remembered that the bag with PBMC therein always had the tubes clamped with a klemme.

(2) Addition of ACD-A liquid (to prevent generation of aggregation)

(i) The bag-included weight of PBMC was measured.
(ii) The added volume of the ACD-A liquid is calculated (2% w/w relative to the PBMC content volume).
(iii) The double separation bag and the ACD-A liquid bag were connected with TSCD. A klemme was placed at the roots of the bags.
(iv) The calculated amount of ACD-A liquid was added while confirming the weight with a scale.
(v) Sealing was carried out with a tube sealer to cut off the ACD-A liquid bag at a position of 5 segments.
(vi) After sufficiently mixing the ACD-A liquid, the tube of the double separation bag was rolled several times with a roller bench to cut off one segment for CBC.
(vii) After cutting off the segment, the weight was measured to record the actual added volume of the ACD-A liquid.

(2. Blood count)

(1) Blood within the segment was transferred to a sample tube.
(2) CBC and machine classification were measured.
(3. Determination of the number of freeze bags/adjustment of content)

(1) Number of freeze bags

Number of freeze bags = 2
Bag-included weight after content adjustment (target) = 50 g + 35 g = 85 g

(2) Adjustment of content

(i) When the content is large centrifugation is carried out. 1200 rpm (370G), 12 min, 10°C, Acce19, Dece16. The whole bag was protected with an air cushion and set at a centrifugation device so that the bag would not twist.
(ii) After centrifugal separation, the PBMC bag is sandwiched at a separation stand and a supernatant part (PRP) is transferred to the subsidiary bag.

Be careful so that a nucleated cell of the precipitate would not enter.
The content cannot be seen unless the bag is set in a manner that the seal side would be at the back.
(iii) The PBMC bag was placed on a scale and the PRP is returned while measuring the weight to make an adjustment to a target weight.

(4. Disinfecting necessary articles)

(i) ACD-A liquid, separation bag (two sites of clamps were closed over the plastic bag), CP-1, albumin, separation bag (Kawasumi KBP-66DC), freeze bag (Nipro F-100), coupling tube (Kawasumi T-8), 20 ml syringe, alcohol cotton and the previously adjusted PBMC bag were disinfected one time with an ethanol spray (not placed inside a safety cabinet).
(ii) Wearing a yellow gown and hat
A yellow gown and a hat were worn for clean operation and attention was paid so as not to leave the adjustment room wearing them in order to maintain cleanliness.
(iii) Disinfecting necessary articles again
After the disinfected necessary articles above are all dried, attention was paid so as to place the disinfected necessary articles inside the safety cabinet while disinfecting again with an ethanol spray.
(iv) Wearing sterilized gloves

Sterilized gloves were worn to put a hand inside the safety cabinet and carry out operation.
Attention was paid so as not to take the hand out until the operation ends since the hand would not be sterile once the hand is taken out.

(5. Operation inside a safety cabinet)

(1) Preparation of cryodamage protection liquid

(i) CP-1 and albumin were punctured with an air needle after taking off the plastic cap and disinfecting the top part with alcohol cotton.
(ii) 20% albumin was taken in a 16 ml syringe.
(iii) 16 ml of 20% albumin was added in CP-1 for 50 ml (34 ml).
(iv) Inversion mixing was carried out in a manner so as not to create foam.

*The operation was carried out while cooling with a refrigerating agent in case of heat generation upon mixing CP-1 and albumin.

(2) Adding a cryodamage protection liquid

(i) A coupling tube was taken out from the bag and a clamp was closed.
(ii) A plastic needle (light yellow color) for transfusion port of a freeze tube was spiked into the PBMC bag.
(iii) A plastic needle (white color) exclusively for rubber of the coupling tube is spiked into the rubber cap of the cryodamage protection liquid set at a bottle holder.
(iv) The bottle holder of the cryodamage protection liquid was suspended on a hook of the safety cabinet and the clamp of the coupling tube was opened. Flow-through was promoted with a roller bench. Attention was paid so as not to dispose of the bottle holder.

While the bag is sandwiched by refrigerating agents to be cooled and mixed, a specified volume of cryodamage protection liquid was slowly put in (10 minutes with the total volume of 200 mL and 5 minutes with 100 mL were used as a standard).
Small amount of air was also put in for pushing out the liquid inside the tube in the later operation.
After putting in the cryodamage protection liquid, attention was paid to carry out the operation quickly while cooling or the cells would die.

(3) Transfusion to a freezing bag

(i) Each of the raw material number + "(1)" and raw material number + "(2)" was described in two freezing bags.
(ii) The ends of the tubes of the two freezing bags were cut with a tube sealer.
The PBMC bag and the two freezing bags were connected with TSCD.
(iii) The klemme of the freezing bag was opened to transfer PBMC to the freezing bag.
(iv) The air of the freezing bag was transferred to the original bag to perform air bleeding. Since air being mixed in would be a factor of bag breakage, air was bled as much as possible.
When there are 2 to 3 bags, innovations were made so that the volume would be equal as possible, but it was arranged to not to be time-consuming.
(v) PBMC of two segments was put back to the tube, the PBMC body was fixed between black lines with a tube sealer and two segments were made for CBC and for preservation.
(vi) CBC measurement was carried out.
(vii) The weight of the freezing bag was measured.
(viii) A bag is put into an aluminum protector one by one and sealed with a seal where the raw material number is described. The seal end was folded back so that the seal would be easily peeled upon thawing.

(6. Freezing)

(1) Freezing treatment was carried out in the following program with a program freezer (CRYOMED Freezer {Thermo SCIENTIFIC})

1. Wait at chamber = 2°C until Sample = 4°C
2. Adjust at 1°C/min until Sample = -4°C
3. Adjust at 60°C/min until Chamber = -70°C
4. Adjust at 20°C/min until Chamber = -15°C
5. Cool 1°C at a time until Sample = -40°C
6. Cool 1°C at a time until Sample = -80°C
7. End

(2) Preserving frozen specimen

Once freezing with a program freezer has ended, the protector with the freeze bag inside was taken out from the chamber to be put in and preserved in a liquid nitrogen tank.
Information was written on the "cell treatment department preserved specimen list (clinical study)" suspended

on the liquid nitrogen tank.

(Result)

A table showing the degree of monocyte purification and the like in a preparation is presented below (in the table, Mo represents monocyte, Ly represents lymphocyte, PBMC represents peripheral blood mononucleosis, Neut represents neutrophil, Ht represents hematocrit value, Plt represents platelet, and Pt represents patient).

Among the data described in the table below, each of the following expressions corresponds to the explanation described on the right.

ComTec: data of raw material collection and cryopreserving performed by a conventional method
Optia: data of raw material collection and cryopreserving performed by the method of the present disclosure
HD23: data of Example 1
HD24: data of Example 2
Apheresis collection specimen: specimen collected in the raw material collection step
Cryopreserving specimen: specimen right before freezing in the cryopreserving step (corresponding to (3)(v) of paragraph 0133)

[Table 1-1]

## Table of HD apheresis data comparison

| | Collection date | Donor ID | Treatment blood volume | Pt | | apheresis collection specimen | | | | | | | | | | | | |
|---|---|---|---|---|---|---|---|---|---|---|---|---|---|---|---|---|---|---|
| | | | (ml) | Mo(/μl) | Mo(%) | Sample volume (g) | | PBMC (10^8 cells) | Ly(/μl) | Ly(%) | Ly(10^8 cells) | Mo(/μl) | Mo(%) | Mo(10^8 cells) | Neut(/μl) | Neut(%) | Ht(%) | Plt (10^4/μl) |
| Com Tec | 2018/3/6 | HD5 | 5000 | 310 | 5.9 | 202.0 | | 58.7 | 25470 | 82.3 | 51.4 | 3590 | 11.6 | 7.3 | 1480 | 4.8 | - | - |
| | 2018/3/28 | HD6 | 6500 | 200 | 4.5 | 220.3 | | 61.3 | 23250 | 78.0 | 51.2 | 4590 | 15.4 | 10.1 | 1730 | 5.8 | - | - |
| | 2018/6/4 | HD7 | 5500 | 200 | 3.3 | 292.7 | | 119.9 | 35960 | 81.7 | 105.3 | 5020 | 11.4 | 14.7 | 2790 | 6.4 | 5.3 | 108.3 |
| | 2018/11/12 | HD8 | 5500 | 470 | 4.6 | 209.5 | | 82.7 | 32290 | 73.5 | 67.6 | 7180 | 16.3 | 15.0 | 3390 | 7.7 | 9.1 | 130.9 |
| | 2019/2/21 | HD10 | 7000 | 250 | 4.2 | 219.7 | | 70.4 | 28520 | 84.6 | 62.7 | 3520 | 10.4 | 7.7 | 1450 | 4.3 | 4.0 | 71.3 |
| | 2019/5/13 | HD15 | 6500 | 340 | 5.4 | 201.4 | | 31.4 | 13120 | 78.0 | 26.4 | 2460 | 14.6 | 5.0 | 1140 | 6.9 | 1.9 | 37.4 |
| | 2019/6/12 | HD17 | 8500 | 260 | 8.8 | 278.5 | | 59.2 | 17900 | 78.9 | 49.9 | 3340 | 14.7 | 9.3 | 1360 | 6.0 | 4.0 | 75.0 |
| | 2019/10/7 | HD20 | 7500 | 320 | 6.4 | 231.4 | | 67.6 | 24320 | 77.4 | 56.3 | 4880 | 15.5 | 11.3 | 1970 | 6.3 | 2.8 | 74.6 |
| | 2019/11/22 | HD22 | 7000 | 240 | 5.6 | 177.1 | | 48.6 | 23120 | 79.1 | 40.9 | 4320 | 14.8 | 7.7 | 1080 | 3.6 | 3.9 | 112.6 |
| Opta | 2020/1/27 | HD23 | 3280 | 270 | 3.9 | Collected blood volume 20 ml + Plasma 80 ml | (Only 1 cycle) | 5.4 | 2550 | 34.6 | 2.6 | 2810 | 38.1 | 2.8 | 1220 | 16.5 | 0.7 | 23.8 |
| | | | 4330 | | | Collected blood volume 20m + 20mll + plasma 60 ml | (2+3 cycles) | 16.0 | 11280 | 55.2 | 11.3 | 4760 | 23.3 | 4.8 | 2960 | 14.6 | 1.3 | 41.3 |
| | 2020/2/17 | HD24 | 7213 | 239 | 2.9 | 205.3 | | 24.8 | 7010 | 47.5 | 14.4 | 5070 | 34.4 | 10.4 | 1520 | 10.3 | 0.3 | 22.6 |

[Table 1-2]

| | Collection date | Donor ID | Treatment blood volume (ml) | Pt Mo(/μℓ) | Pt Mo(%) | Upon freezing-preserving Sample volume (g) | PBMC (10^8 | Ly(/μℓ) | Ly(%) | Ly(10^8 cells) | Mo(/μℓ) | Mo(%) | Mo(10^8 cells) | Neut(/μℓ) | Neut(%) | Ht(%) | Plt (10^4/μℓ) |
|---|---|---|---|---|---|---|---|---|---|---|---|---|---|---|---|---|---|
| Com Tec | 2018/3/6 | HD5 | 5000 | 310 | 5.9 | 101.6 | 49.8 | 42050 | 80.4 | 42.7 | 6930 | 13.3 | 7.0 | 2690 | 5.2 | 16.5 | 84.6 |
| | 2018/3/28 | HD6 | 6500 | 200 | 4.5 | 185.0 | 52.1 | 23660 | 78.4 | 43.8 | 4500 | 14.9 | 8.3 | 1660 | 5.6 | - | - |
| | 2018/6/4 | HD7 | 5500 | 200 | 3.3 | 99.7 | 56.1 | 48990 | 80.5 | 48.8 | 7240 | 11.9 | 7.2 | 4260 | 7.0 | 16.6 | 85.4 |
| | 2018/11/12 | HD8 | 5500 | 470 | 4.6 | 97.9 | 61.9 | 51560 | 73.6 | 50.5 | 11710 | 16.7 | 11.5 | 5390 | 7.7 | 26.8 | 74.3 |
| | 2019/2/21 | HD10 | 7000 | 250 | 4.2 | 93.9 | 57.9 | 53610 | 82.1 | 50.3 | 8090 | 12.4 | 7.6 | 3010 | 4.6 | 14.9 | 59.2 |
| | 2019/5/13 | HD15 | 6500 | 340 | 5.4 | 98.7 | 29.5 | 24660 | 76.6 | 24.3 | 5250 | 16.3 | 5.2 | 2150 | 6.7 | 6.8 | 51.6 |
| | 2019/6/12 | HD17 | 8500 | 260 | 8.8 | 101.9 | 59.2 | 49100 | 78.2 | 50.0 | 9000 | 14.3 | 9.2 | 4500 | 7.1 | 18.0 | 118.0 |
| | 2019/10/7 | HD20 | 7500 | 320 | 6.4 | 95.7 | 64.3 | 57600 | 79.8 | 55.1 | 9600 | 13.3 | 9.2 | 4900 | 6.8 | 10.0 | 104.0 |
| | 2019/11/22 | HD22 | 7000 | 240 | 5.6 | 97(ml) | 42.7 | 37310 | 79.7 | 36.2 | 6660 | 14.2 | 6.5 | 1930 | 4.2 | 10.8 | 101.6 |
| Opta | 2020/1/27 | HD23 | 3280<br>4330 | 270 | 3.9 | 96.9 | 20.0 | 12640 | 46.6 | 12.2 | 8010 | 29.5 | 7.8 | 4490 | 16.5 | 3.6 | 37.3 |
| | 2020/2/17 | HD24 | 7213 | 239 | 2.9 | 88.1 | 26.7 | 13960 | 40.8 | 13.5 | 13590 | 39.7 | 13.2 | 4240 | 12.4 | 0.7 | 28.4 |

As described above, in Example 1 (HD23), the monocyte purity became about 30% using the method of the present disclosure. In addition, the Ht (hematocrit value) and Plt (platelet) were further reduced compared to the conventional method.

In Example 1, the collection reference started from a normal value. (In the below-mentioned Table 2, AC represents ACD-A, Hct represents hematocrit value and TBV represents circulating blood volume.)

[Table 2]

MNC

Spectra Optia:
Software version: 11
Female 153 cm
Htc value: 40%

42 kg TBV: 2848 mL
Platelet : 310 E3/μL WBC: 7 E3/μL

| Treatment value | Initial value | Final value |
|---|---|---|
| Collection preference | 30 | 50 |
| Total blood treatment volume (mL) | 6600 | 7117 |
| Treatment time (minute) | 180 | 201 |
| Treatment TBV | 2.3 | 2.5 |
| AC (mL) | 600 | 653 |
| Blood collection (mL) | 7200 | 7770 |
| Plasma (mL) | 0 | 0 (AC 0) |
| Collection (mL) | 140 | 200 (AC 24) |
| Plasma inside collection bag (mL) | 0 | 140 (AC 17) |
| Collection step | 7 | 3 |
| Chamber flash (mL) | 16 | 16 |
| Chamber chase (mL) | 4 | 4 |

Water balance (mL)

| | |
|---|---|
| AC | 653 |
| Plasma | 0 |
| Collection | -60 |
| Plasma inside collection bag | -140 |
| Physiological saline that went around | -47 |
| Tube set | -3 |
| Rinse-back | 125 |
| Sum | 529 |
| Replenishment liquid | |
| Final water balance | |

AC to patient : 629 mL

From the result of this Example, it was confirmed that the monocyte purity that was about 10% increases up

to about 30% when combining separations by specific gravity and size compared to the case of performing apheresis (only specific gravity separation).

Furthermore, it was found that there is a tendency of the purity increasing when the spillover treatment is carried out.

As shown below, the purity further increased when also carrying out the cryoprotectant agent treatment, dead cell treatment and lymphocyte and platelet removal (OptiPrep) treatment.

(Example 2: Further improvement of apheresis)

Apheresis was carried out in the same manner as Example 1.

In Example 2, the same protocol as Example 1 was used but the collection preference value was set to be higher than the automatically set value by 20.

(Result)

It was discovered that the monocyte purity significantly increases by the method of the present disclosure (setting the collection preference value to be higher than the normal value and performing separations by specific gravity and size) (about 40%) (Table 1).

(Example 3: Treatment of DNase)

This Example performed test manufacturing of a Tax-specific dendritic cell using the cryopreserving raw material that underwent cryopreserving in Example 1, wherein products that did and did not undergo treatment of DNase were prepared.

(Protocol)

(Purpose)

- It is possible that the monocyte collection rate and the monocyte purity are decreased by an aggregate generated on the first day of the manufacturing step (Day 0). This example was performed while dividing into two groups, with presence or absence of DNase (pulmozyme) addition, and the ATL-DC collection rate and quality were comparatively examined.

(Raw material to be used)

The content volume is about 50 mL, half-scale of the normal manufacturing

<Day of collection and freezing> January 27, 2020

<Apheresis device> Spectra Optia (Terumo BCT)

<Donor> Healthy person (HD23)

<Collection condition>

- Mononucleosis collection (MNC collection) protocol was used.
- The used volume of ACD-A liquid was blood treatment volume: ACD-A liquid = 12:1.
- The treatment blood volume per cycle was determined for each cycle by the filled volume of blood cells into a chamber.
- The total blood count measurement was carried out during a cycle to determine the total blood treatment volume with the target of $1\times10^9$ monocytes . A collection specimen was added with plasma to prepare about 200 mL.

<Freezing condition>

- Performed in accordance with the method of cryopreserving a peripheral blood hematopoietic stem cell of a patient and a donor for hematopoietic stem cell transplantation in a routine medical care. (2% (w/w) of ACD-A liquid was added. Centrifugal separation was carried out to remove supernatant to prepare a content volume of 50 mL. 50 mL of CP-1/HSA mixing liquid was added to dispense into two frozen bags. A program freezer was used to cool to -80°C or lower to then preserve in a liquid nitrogen tank.)

<Total blood count data upon collection/freezing>

[Table 3]

| HD23 | Upon donor screening | | Item collected in the first cycle | | Item collected in second to third cycles | | Totoal of all three cycles | | Raw material before freezing | | Total of 2 bags | | HD23-aph200127-1 | | HD23-aph200127-2 | |
|---|---|---|---|---|---|---|---|---|---|---|---|---|---|---|---|---|
| Content | | | 100 | mL | 100 | mL | 200 | mL | | | 96.9 | g | 48.3 | g | 48.6 | g |
| WBC | 7010 | /μl | 7370 | /μl | 20420 | /μl | 2.78 | x10^9cells | 27140 | /μl | 2.63 | x10^9 cells | 1.31 | x10^9cells | 1.32 | x10^9 cells |
| PBMC (Lymp+Mono) | 2490 | /μl | 5300 | /μl | 16040 | /μl | 2.13 | x10^9cells | 20650 | /μl | 2.00 | x10^9 cells | 1.00 | x10^9cells | 1.00 | x10^9cells |
| Neutrophil | 4270 | /μl | 1230 | /μl | 2960 | /μl | 0.42 | x10^9cells | 4490 | /μl | 0.44 | x10^9 cells | 0.22 | x10^9cells | 0.22 | x10^9cells |
| Monocyte | 270 | /μl | 2770 | /μl | 4760 | /μl | 0.75 | x10^9cells | 8010 | /μl | 0.78 | x10^9cells | 0.39 | x10^9cells | 0.39 | x10^9cells |
| Lymphocyte | 2220 | /μl | 2530 | /μl | 11280 | /μl | 1.38 | x10^9cells | 12640 | /μl | 1.22 | x10^9 cells | 0.61 | x10^9 cells | 0.61 | x10^9cells |
| HCT | 40.5 | % | 0.7 | % | 1.3 | % | | | 3.6 | % | | | | | | |
| Plt | 31 | 10,000 /μl | 23.6 | 10,000 /μl | 41.3 | 10,000 /μl | | | 37.3 | 10,000 /μl | | | | | | |

(Method)

The raw material thawing-washing step was divided into two groups of DNase addition present and DNase addition absence as shown below and then the two groups of cells separately underwent the Tax-specific dendritic cell test manufacturing step. A part of the manufacturing step (raw material thawing-washing step) in the present Example is shown in Figure **12.** The detailed description of the method is as described in Example 4.

Furthermore, in this Example, pH adjustment of a medium used in the thawing-washing step (neutralization of acid by gas exchange under an atmosphere) was not performed. Pipetting was carried out using a 10 mL pipette for sufficient suspension of a cell prior to Opti-Prep overlay and prior to flask seeding.

(Result)

Based on the result (Table 4 and Table 5), there was no significant difference in the DC yield, live cell ratio and surface characteristics of each of what was obtained under both conditions of present and absent DNase treatment, and it was considered possible to manufacture a DC that can be administered.

(Example 4: Improvement of monocyte purification using a cryoprotectant agent)

In this Example, the improvement of monocyte purification when using a cryoprotectant agent was studied.

(Material and method)

(Preparation of a reagent)

A reagent was prepared in accordance with the following.

Day 0

((0) Preparation of a reagent)

Required amount

| | |
|---|---|
| RPMI1640: | 500 ml bottle 5 |
| PBS: | 500 ml bottle 1,<br>106.5ml for OptiPrep dilution |
| 25% HSA: | 90 ml |
| ACD-A liquid: | 60 ml |
| OptiPrep: | 30 ml |

(Preparation of a reagent)

As described below, a reagent required for the steps (1) thawing/washing and (2) separation and culturing was adjusted.

*Attention was paid so that the following reagents would be sterilely adjusted in a clean bench.

(Preparation of Medium (1) (for washing))

Medium (1) (for washing): 5.5% ACD-A/1% HSA/RPMI1640
After adding 30 ml of ACD-A liquid to a bottle of 500 ml of RPMI1640, 20 ml of 25% HSA was added.
ACD-A liquid 30 ml
RPMI1640 500 ml/Total 530 ml × 2
↓
25 % HSA 20 ml/total 550 ml × 2
↓
The 500 ml bottle with RPMI1640 therein was transferred, sealed and transported as shown below.

(1) 560ml → for 38°C
(2) 540ml → for room temperature

(Preparation of Medium (2) (for washing/culturing)) Medium (2) (for washing/culturing): 1% HSA/RPMI1640 20 ml of 25% HSA was added to a bottle of 500 ml of RPMI1640.

25% HSA 20 ml
RPMI1640 500 ml/Total 520 ml × 2
↓
One was kept at room temperature for washing and the other was kept cool at 4°C for separation.

(Preparation of separation liquid (specific gravity: 1.075))

An OptiPrep specific gravity liquid was adjusted in a 225 ml centrifuge tube as shown below.

*Before making the adjustment, the OptiPrep was returned to room temperature.

Specif gravity: 1.075 → PBS 106.5 ml + OptiPrep 30 ml
*Since OptiPrep has high viscosity, aliquoting should be carried out accurately with care.
*Pipetting is to be carried out so that there would be carried out accurately with care. no remaining OptiPrep inside the pipette.
↓
Inversion mixing was carried in a gentle manner and the liquid is dispensed into eight 50 ml centrifuge tubes with 15 ml each.

(Preparation of PBS for washing)

PBS for washing: 0.5% HSA/PBS (kept cool at 4°C)
10 ml of 25% HSA was added to a bottle of 500 ml of PBS.
25% HSA 10 ml
PBS 500 ml/Total 510 ml × 1

(RPMI1640 for flask washing)

A bottle of 500 ml of RPMI1640 was kept warm at 37°C (inside an incubator) upon initiation of incubation of a flask.

((1) The step of thawing/washing of a frozen specimen: pulmozynme treatment present)

The treatment method of using a cryoprotectant agent is described below.

Day 0

(1. Preparation of device)

Water bath was warmed to 38°C.
Setting of a centrifugation device (confirmation of the centrifugation feature setting) was carried out (also creation of balance).
Installation of a cell counter

(2. Preparation of centrifuge tube)

Five 225 ml centrifuge tubes were prepared with the labels "A", "B", "C", "(9)S" and "(9)P" and one 50 mL centrifuge tube was prepared with the label "D".

A bottle for waste liquid was also prepared separately.

(3. Preparation of reagent)

The Medium (1) (560 ml bottle) prepared in the step above was dispensed as shown below.

Medium(1): 5.5% ACD-A/1% HSA/RPMI1640
Two 225 ml centrifuge tubes (A and B) with 180 mL each
One 225 ml centrifuge tube (C) with 160 ml
One 50 ml centrifuge tube (D) with 40 ml (for co-washing)
Dispensing was carried out and the Water bath was warmed.

The remaining bottle (540 ml) was kept at room temperature.

Medium(2): 1% HSA/RPMI1640

One was kept at room temperature for washing and the other one was kept cool at 4°C for separation.

(4. Sample tube preparation)

An Eppendorf tube used for sampling and counting was prepared.

(5. Preparation of Medium 5.5% ACD-A)

All of the Medium (1) (A, B, C and D) that had been kept warm were taken out.

(6. Preparation of frozen specimen)

The frozen bag (frozen apheresis specimen) was taken out from liquid nitrogen.

(7. Thawing of frozen specimen)

The frozen bag was quickly thawed with a 38°C Water bath (about 2 minutes).

(8. Cell collection for the first time)

The frozen bag was put inside a clean bench and a bag spike and a 50 ml syringe were connected for collection in a manner that does not render the connection part unclean (so as to float in the air). (First time)

(9. Cell dispensation, Wash for the first time)

The first collection liquid volume was measured, divided into three equal amounts and dispensed into 225 ml centrifuge tubes (A, B and C: Medium (1)).
First collection liquid volume: 40 ml (about 13 ml each)

(10. Cell dispensation, Wash for the first time)

An 18G needle was connected to a 50 mL syringe and a 50 ml centrifuge tube (D) was used to aliquot 22 ml of the Medium (1).

(11. Cell collection for the second time)

Connection to the frozen bag was carried out again and the cells remaining in the bag was washed and collected. (Second time)

(12. Cell collection for the second time)

Second collection liquid volume was measured and added to a 225 ml centrifuge syringe (C).
Second collection liquid volume: 24 ml (it is calculated that the centrifuge tube C is added with about 15 ml of raw material)

(13. Cell collection for the second time)

A lid was put on a centrifuge tube and the centrifuge tube was inverted and mixed several times.

(14. Specimen centrifugal separation of a specimen for the first time)

Centrifugal separation (20°C, 350 × g, 5 minutes, brake 2) was carried out.

(15. Removal of supernatant)

Supernatant was removed (50 ml pipette) . Waste liquid was disposed of into a waste liquid bottle.
At this time, attention was paid so that about 10 ml of supernatant would remain in order to reduce cell loss.

(16. Cell suspension)

The bottom of the centrifuge tube was contacted with a tube stand and slid to the side to loosen the pellet.
The operations thereafter were carried out separately with the different conditions as shown below.

225 ml centrifuge tube A → Pulmozyme treatment present
225 ml centrifuge tubes B and C → Pulmozyme treatment absent

(Pulmozyme treatment present)

The step with the Pulmozyme treatment present was continued to carry out below.

(17. 225 ml centrifuge tube A) (Pulmozyme treatment present)
20 ml (25 ml pipette) of room temperature Medium (1) was added to carry out suspension.

(18. Wash for the second time, 5.5% ACD-A) (Pulmozyme treatment present)

Dilution with a room temperature Medium (1) was carried out in a measuring cylinder up to 200 ml (50 ml pipette) and inversion mixing was carried out about three times.
The presence/absence of an aggregate is confirmed herein and it was confirmed that there is no aggregate.

(Regardless of the presence/absence of an aggregate, the Pulmozyme treatment of cell suspension liquid after the centrifugation/supernatant removal from step 21 is carried out)

(19. Centrifugal separation of a specimen for the second time) (Pulmozyme treatment present)

Centrifugal separation (20°C, 300 × g, 5 minutes, break **2)** was carried out.

(20. Removal of supernatant) (Pulmozyme treatment present)

Supernatant was removed so that the remaining liquid would be about 5 ml (50 ml pipette).
The waste liquid was disposed of in a waste liquid bottle.

(21. Cell suspension) (Pulmozyme treatment present)

The bottom of the centrifuge tube was contacted with a tube stand and slid to the side to loosen the pellet.

(21.1. Pulmozyme treatment) (Pulmozyme treatment present)

The remaining liquid was used to suspend the cell (10 ml pipette) to measure the suspension liquid volume.
Suspension liquid volume: 2.5 ml
→ 5 ml - suspension liquid volume: 2.5 ml = added volume: 2.5 ml
(When there is not enough, the added amount of the Medium (1) is added)
(21.2. Pulmozyme treatment) (Pulmozyme treatment present)
1 ml of Pulmozyme (2.5 mg/2.5 ml) was added.
(21.3. Pulmozyme treatment) (Pulmozyme treatment present)
Reaction was caused for 15 minutes while shaking the tube.

(22. Wash for the third time, 1% HSA/RPMI) (Pulmozyme treatment present)

20 ml of the room temperature Medium (2) (25 ml pipette) was added to carry out suspension.
175 ml of the room temperature Medium (2) was added to carry out dilution in a measuring cylinder up to 200 ml (50 ml pipette) and inversion mixing was carried out about three times .

(23. Sampling) (Pulmozyme treatment present)

A suspension liquid was sampled (sample (8)P) (only counting the number of cells).
→ Aliquot 10 μl into an Eppendorf tube (for × 5 dilution) .

(24. Centrifugal separation of a specimen for the third time) (Pulmozyme treatment present)

Centrifugal separation (20°C, 300 × g, 5 minutes, break 2) was carried out.

(25.1. Counting cells) (Pulmozyme treatment present)

The number of cells in a sample (8)P was counted during centrifugal separation.
The result was recorded on the cell count record table and the number of cells was calculated.
Number of live cells in the sample (8)P: $3.0 \times 10^8$...{A}

(25.2. Preparation of reagent/device) (Pulmozyme treatment present)

After termination of the centrifugation of step 24, the setting of the break of the centrifugation device was turned OFF and set to 4°C for cooling.

(25.3. Medium preparation, 1% HSA/RPMI) (Pulmozyme treatment present)

The cold Medium (2) (500 ml bottle) that had been cooled at 4°C was prepared.

(26. Removal and sampling of supernatant) (Pulmozyme treatment present)

Supernatant was removed (50 ml pipette).

(Herein, more supernatant may be removed than the wash for the first time (about 5 ml))

→ Supernatant was collected in a 225 ml centrifuge tube ((9)P) (only FCM) .

(27. Cell suspension) (Pulmozyme treatment present)

The bottom of the centrifuge tube was contacted with a tube stand and slid to the side to loosen the pellet.
The remaining liquid was used to suspend the cell (10 ml pipette) to measure the suspension liquid volume.
Suspension liquid volume: 4.0 ml...{B}

(28. Calculation of suspension liquid volume) (Pulmozyme treatment present)

From the result of counting, the suspension liquid volume of the cell was calculated with the following formula and the cell concentration was adjusted.

(1) Since the cell count of (8)P was less than 4×10^8, the suspension liquid volume was set at 20 ml.
(2) {C:20ml} - {B:4.0ml} = added amount: 16.0 ml...{E}

(29. Preparation of suspension liquid) (Pulmozyme treatment present)

The added volume of 16.0 ml of the cool Medium (2) was added to the cell suspension liquid of step 27 (cell suspension) and a 10 ml pipette was used to carry out pipetting.

(30. Sampling) (Pulmozyme treatment present)

The cell suspension liquid was sampled (sample (10)P) (counting the number of cells, for FCM).
→ Aliquot 0.5 ml into a 15 ml centrifuge tube (for × 20 dilution).
The number of 50 ml centrifuge tubes necessary for separation at an Optiprep was confirmed (number of separation tubes: 1)
↓
Proceed to the step of "(2) Adjustment of immature dendritic cell"
During the time until overlay is carried out, the cell suspension liquid was left at 4°C.

(Pulmozyme treatment absent)

From step 17, the steps were continued with the Pulmozyme treatment absent in the following manner.

(17.225 ml centrifuge tubes B and C) (Pulmozyme treatment absent)
20 ml (25 ml pipette) of the room temperature Medium (1) was added to carry out suspension.

Suspension liquid of two centrifuge tubes (B and C) was collected into one 225 ml centrifuge tube (C). Upon doing so, 20 ml of the room temperature Medium (1) was used to co-wash the centrifuge tube.

(18. Wash for the second time, 5.5% ACD-A) (Pulmozyme treatment absent)

Dilution with a room temperature Medium (1) was carried out in a measuring cylinder up to 200 ml (50 ml pipette) and inversion mixing was carried out about three times.

(19. Centrifugal separation of a specimen for the second time) (Pulmozyme treatment absent)

Centrifugal separation (20°C, 300 × g, 5 minutes, break 2) is carried out.

(20. Removal of supernatant) (Pulmozyme treatment absent)

Supernatant was removed (50 ml pipette) . The waste liquid was disposed of in a waste liquid bottle.
Supernatant was removed so that the remaining liquid would be about 5 ml (50 ml pipette).

(21.1. Cell suspension, 1% HSA/RPMI) (Pulmozyme treatment absent)

The bottom of the centrifuge tube was contacted with a tube stand and slid to the side to loosen the pellet.

(21.2. Cell suspension, 1% HSA/RPMI) (Pulmozyme treatment absent)

20 ml of the room temperature Medium (2) (25 ml pipette) was added to carry out suspension.
The suspension liquid volume was measured. Suspension liquid volume: 27.5 ml

(21.3. Cell suspension 1% HSA/RPMI) (Pulmozyme treatment absent)

120 ml of the room temperature Medium (2) was added (50 ml pipette) to confirm the presence/absence of an aggregate.

(Aggregate: absent)

(22. Wash for the third time, 1% HSA/RPMI) (Pulmozyme treatment absent)

52.5 ml of the room temperature Medium (2) was added to carry out dilution in a measuring cylinder up to 200 ml (50 ml pipette) to suspend a cell by pipetting.

(23. Sampling) (Pulmozyme treatment absent)

Suspension liquid was sampled (sample (8)S) (only counting the number of cells).
→ Aliquot 10 μl into an Eppendorf tube (for × 5 dilution)

(24. Centrifugal separation of a specimen for the third time) (Pulmozyme treatment absent)

Centrifugal separation (20°C, 300 × g, 5 minutes, break 2) was carried out.

(25.1. Counting cells) (Pulmozyme treatment absent)

The number of cells in the sample (8)S was counted during centrifugal separation.
The result was recorded on the cell count record table and the number of cells was calculated.
Live cell count in the sample (8)S: $5.0 \times 10^8$...{8S}

(25.2. Preparation of reagent/device) (Pulmozyme treatment absent)

After termination of the centrifugation of step 24, the setting of the break of the centrifugation device is turned OFF and set to 4°C for cooling.

(25.3. Medium preparation 1% HSA/RPMI) (Pulmozyme treatment absent)

A cool Medium (2) (500 ml bottle) that had been cooled at 4°C was prepared.

(26. Removal and sampling of supernatant) (Pulmozyme treatment absent)

Supernatant was removed so that the remaining liquid would be about 5 ml (50 ml pipette).
→ The supernatant was collected in a 225 ml centrifuge tube ((9)S-1) (FCM) .

(27. Cell suspension) (Pulmozyme treatment absent)

20 ml of the cool Medium (2) (25 ml pipette) was added to carry out suspension.
The suspension liquid volume was measured.
Suspension liquid volume: 28 ml...{B}

(28. Calculation of suspension liquid volume) (Pulmozyme treatment absent)

From the result of counting, the suspension liquid volume of the cell was calculated with the following formula and the cell concentration was adjusted. (Adjustment was made so that the cell concentration would be the same as "Pulmozyme treatment present".)

(1) {8S: 50 × 10^7}/{1.5 × 10^7/ml} = 33 ml...{C : cell suspension liquid total volume}
(2) {C: 33 ml} - {B: 28 ml} = added volume: 5 ml...{E}

(29. Preparation of suspension liquid) (Pulmozyme treatment absent)

Added volume of 5 ml of the cool Medium (2) was added to the cell suspension liquid of step 27 (cell suspension) and a 10 ml pipette was used to carry out pipetting.

(Treatment of aggregate)

Since a large aggregate was recognized in step 29, the following operation was carried out.
20 ml of the room temperature Medium (2) was added to a 50 ml centrifuge tube. A 10 ml pipette was used to collect only the aggregate which was transferred to the 50 ml centrifuge tube and the aggregate was suspended and loosened.
The tube was left for about 30 seconds to wait the remaining aggregate to sink naturally.
Supernatant was collected so that the large aggregate would not enter and the aggregate was put back to the cell suspension liquid of step **29.**

(30. Sampling) (Pulmozyme treatment absent)

Cell suspension liquid was sampled (sample (10)) (counting the number of cells, for FCM).
→ Aliquot 0.5 ml into a 15 ml centrifuge tube (for × 20 dilution)
The number of 50 ml centrifuge tubes necessary for separation with an Optiprep was calculated.
{C: 33 ml}/20 ml = number of separation tubes: 2
(After overlaying 20 ml in the first bottle, the remaining liquid was diluted in a measuring cylinder up to 20 ml for overlay in the second bottle)
↓
Proceed to the step of "(2) Adjustment of immature dendritic cell"

(Result)

In this Example, the monocyte purity that was about 30% right after apheresis increased to about 50% after the thawing-washing step by utilizing a cryoprotectant agent (CP-1) . Furthermore, after the specific gravity centrifugal separation step, the monocyte purity increased up to about 70% (Table 4) .

[Table 4]

Collected/freeze-preserved apheresis specimen (used device: Optia)

Raw material cell count

| | CBC before raw material freezing | | | | Collected liquid volume after thawing | PBMC count | Monocyte count |
|---|---|---|---|---|---|---|---|
| | Mono+Lymp (μl) | Mono(μl) | Hct (%) | Plt (10,000/μl) | | (CBC Mono+Lymp) x liquid volume | (CBC Mono) x liquid volume |
| Dnase absent | | | | | | | |
| Raw material | 20650 | 8010 | 3.6 | 37.3 | 28 | 5.78.E+08 | 2.24.E+08 |
| Dnase present | | | | | | | |
| Raw material | 20650 | 8010 | 3.6 | 37.3 | 13 | 2.68.E+08 | 1.04.E+08 |

Result

| | | Visual Observation (Turk) | Visual Observation (Trypan blue) | | Auto cell counter (Trypan blue) | | FCM-positive rate | | Turk cell count × FCM-positive rate | |
|---|---|---|---|---|---|---|---|---|---|---|
| | | Total cell count | Live cell count | Live cell rate | Live cell count | Live cell rate | CD3+ | CD14+ | CD3-positive cell count | CD14-positive cell count |
| Dnase absent | | | | | | | | | | |
| Freezing-washing | Sample 8 | 5.00.E+08 | 6.84.E+08 | 97% | 7.73.E+08 | 90% | | | | |
| | Sample 9 | | | | | | 60.0% | 7.8% | | |
| | Sample 10 | 4.79.E+08 | 4.03.E+08 | 95.3% | 5.10.E+08 | 90% | 27.5% | 50.3% | 1.32.E+08 | 2.41.E+08 |
| After Optiprep | Monocyte layer | 1.68.E+08 | 1.93.E+08 | 96.6% | 2.21.E+08 | 94% | 5.9% | 71.8% | 9.85.E+06 | 1.21.E+08 |
| | Lymphocyte layer | 1.62.E+08 | 1.32.E+08 | 97.1% | 1.46.E+08 | 80% | 54.9% | 19.1% | 8.89.E+07 | 3.09.E+07 |
| Before Tax pulse | DC | | 2.29.E+07 | 87.1% | 2.12.E+07 | 89% | | | | |
| Before final freezing | DC | | 2.38.E+07 | 96.3% | 2.26.E+07 | 95% | | | | |
| Dnase present | | | | | | | | | | |
| Freezing-washing | Sample 8 | 3.00.E+08 | 2.70.E+08 | 90% | 4.57.E+08 | 89% | | | | |
| | Sample 9 | | | | | | 57.7% | 15.4% | | |
| | Sample 10 | 2.40.E+08 | 1.40.E+08 | 97.3% | 2.29.E+08 | 95% | 24.5% | 53.3% | 5.87.E+07 | 1.28.E+08 |
| After Optiprep | Monocyte layer | 9.71.E+07 | 1.31.E+08 | 92.2% | 1.60.E+08 | 100% | 4.1% | 76.2% | 3.97.E+06 | 7.40.E+07 |
| | Lymphocyte layer | 6.30.E+07 | 6.00.E+07 | 95.6% | 6.59.E+07 | 86% | 59.2% | 15.6% | 3.73.E+07 | 9.84.E+06 |
| Before Tax pulse | DC | | 1.20.E+07 | 94.7% | 8.17.E+06 | 88% | | | | |
| Before final freezing | DC | | 1.04.E+07 | 98.9% | 6.20.E+06 | 90% | | | | |

(thin box) Gating to 5 to 22 μm

(thick box) Gating to 7 to 22 μm

Samples 8、9、10・・・ Cell collected after second wash, supernatant removed after third wash and cell collected after third wash, respectively

*A sample after thawing-washing and OptiPrep was gated into 5 to 22 μm and a sample before Tax pulsing and before final freezing was gated into 7 to 22 μm.

An aggregate was generated in the manufacturing step of Day 0 in a group without DNase addition, whereas an aggregate was not generated after the adding treatment in a group with DNase addition. Furthermore, there was not explicit difference between the two groups regarding adhesion of a monocyte to a flask on Day 0 and the purity/surface character of DC collected on Day 7.

Under this collection condition using a Spectra Optia, a monocyte which is a target cell can be sufficiently collected and lymphocytes, red blood cells, platelets, or the like being mixed in were able to be reduced. It is considered that the reduction of these cells being mixed in, which are not targeted, would lead to improvement in ATL-DC collection rate and quality.

In this examination, pH adjustment of washing liquid used in the thawing-washing step was not performed, but it was possible to achieve the yield of DC that can be administered in the case of conversion into full-scale manufacturing and the purity of DC was good (Table 5).

[Table 5]

Day7 **Collected DC "before final ferezing" surface marker**

| DNase **absent** | | | |
|---|---|---|---|
| | DC gate /Live cells | Lymp gate/Live cells | |
| | 91.2-91.9% | 4.3-4.7% | |
| | /DC gate | /Lymp gate | /Live cells |
| CD11c | 98.3-99% | 11.0% | 90.5-90.9% |
| CD14 | 0.4% | 0.9% | 0.4% |
| HLA-DR | 99.8-99.9% | 20.1-26.1% | 92.6-92.9% |
| CD40 | 99.6% | 20.5% | 91.8% |
| CD83 | 84.8% | 11.5% | 77.9% |
| CD80 | 99.9% | 23.9% | 92.2% |
| CD86 | 99.9% | 19.8% | 92.0% |
| CCR7 | 0.4% | 1.4% | 0.5% |
| CD4 | 17.0% | 61.2% | 18.4% |
| CD25 | 78.5% | 25.5% | 72.8% |
| CD3 | 1.54-1.7% | 67.8-68.1% | 4.6-4.8 |
| CD19 | 1.2% | 16.2% | 1.8% |
| CD56 | 1.4% | 2.8% | 1.4% |

| DNase **present** | | | |
|---|---|---|---|
| | DC gate /Live cells | Lymp gate/Live cells | |
| | 8.9-89.5% | 5.4-5.7% | |
| | /DC gate | /Lymp gate | /Live cells |
| CD11c | 95.7-96.5% | 11-14.8% | 85.7-86.6% |
| CD14 | 0.5% | 0.9% | 0.5% |
| HLA-DR | 99.5-99.7% | 30.3-33.2% | 90.4-90.9% |
| CD40 | 99.4% | 24.1% | 90.3% |
| CD83 | 58.6% | 12.8% | 53.2% |
| CD80 | 99.8% | 26.1% | 90.8% |
| CD86 | 99.6% | 19.0% | 90.2% |
| CCR7 | 0.5% | 1.7% | 0.5% |
| CD4 | 6.3% | 59.8% | 8.9% |
| CD25 | 80.8% | 21.2% | 73.5% |
| CD3 | 0.8-1.7% | 67.2-69.8% | 4.6-5.3% |
| CD19 | 0.8% | 12.1% | 1.4% |
| CD56 | 1.0% | 2.7% | 1.0% |

(Example 5: Day 0 monocyte/lymphocyte analysis)

This Example was carried out in the following manner.

(Staining)

1. Suspension was carried out with 1% FBS/PBS to make adjustment to about 1 × 10^7/mL.
2. An antibody was dispensed to a FACS Tube and cell suspension liquid was added 100 μL each.
3. Incubation was carried out for 20 min on ice while shielding light.
4. Cool PBS was added 4 mL each to carry out centrifugation of 440 g for 6 min at 4°C.
5. Supernatant was removed with a decant and a cell was suspended with a suitable volume of 1% FBS/PBS (250 μL or more).
6. After storing on ice while shielding light until measurement, the liquid was exposed to flow cytometry. Staining with an antibody was carried out as shown in Figure **13.**

(Result)

The result is shown in Figures **1** to **4.** The figures show the result of Day 0 monocyte/lymphocyte analysis of a test specimen wherein the Pulmozyme treatment is present in Figures **1** to **2** and the Pulmozyme treatment is absent in Figures **3** to **4.** Figures **1** and **3** are a preparation before OptiPrep and Figures **2** and **4** are a preparation after OptiPrep.

(Example 6: DC analysis of specimen before final freezing)

This Example was carried out in the following manner.

(Antibody dispensation)

1) Labels "P, S" (two patterns) to "1 to 9" (nine patterns) were made and attached to eighteen FCM tubes for staining.
2) An antibody mix of each Stain was made for nine FCM tubes for making an antibody mix "Ab 1 to 9".
3) A small vortex was made for mixing.
4) An antibody of each Stain is dispensed to each tube.
5) Light was shield for storage at 4°C until use.

(Staining)

1) The collected cell suspension liquid (about 2 × 10^6 cells) was received.
2) 1% FBS/PBS was added to render the total volume 10ml for mixing.
3) Centrifugal separation (4°C, 400 g, 5 minutes) was carried out.
4) Supernatant was removed.
5) 700 μl of 1% FBS/PBS was added to carry out pipetting and suspension of the pellet.
6) Mixing was carried out in a vortex.
7) 50 μl of cell suspension liquid was dispensed in each FCM tube in which an antibody had been dispensed.
8) Mixing was carried out in a vortex.
9) Incubation was carried out for 30 minutes at 4°C while shielding light (a small vortex was made at the time point of 15 minutes) .
10) 2 ml of 1% FBS/PBS was added for mixing in a vortex.
11) Centrifugal separation (4°C, 400 g, 5 minutes) was carried out.
12) Supernatant was removed by decantation.
13) 1% FBS/PBS was added 400 μl each for mixing in a vortex.
14) Light was shield for storage at 4°C until measurement.

(Result)

Figures **5** to **7** confirm the feature of the dendritic cell obtained using various antibodies with the Pulmozyme treatment present. Figures **5** to **7** show FCM using an antibody relative to each marker.

Figures **8** to **10** confirm the feature of the dendritic cell obtained using various antibodies with the Pulmozyme treatment absent. Figures **8** to **10** show FCM using an antibody for each marker.

(Example 7: Flow until manufacturing of pulse cell)

The ATL-DC manufacturing step flow is shown below.

A part of the flow below is as discussed above and the differentiation of a dendritic cell and after are shown in a concise manner.

(Protocol)

Day 0

(1) Thawing-washing step

- A cryopreserved specimen is thawed with a 38°C thermostatic tank.
- The washing step using ACD-A/HSA containing RPMI-1640 (2 to 3 times)
- The washing step using HSA containing RPMI-1640 (0 to 1 time)
- Suspension of an aggregate by Pulmozyme in the washing step
- The step of making cell suspension liquid using HSA containing RPMI-1640

(2)Specific gravity centrifugal separation step

- Monocyte/lymphocyte separation step using OptiPrep
- The step of making monocyte suspension liquid using HSA containing RPMI-1640

(3) Adhesion treatment step, initiate culturing

- Leave for 120 minutes in the presence of AZT in a T-175 flask
- The step of collecting non-adhered cells and washing adhered cells
- Step of differentiating into dendritic cell: culture for 5 days using HSA containing RPMI-1640 added with GM-CSF, IL-4 and AZT Day 5
- KLH pulse step
- RPMI-1640 containing KLH, TNF-$\alpha$, GM-CSF, AZT and HSA is added to carry out culturing for one day

Day6

- OK432 () treatment step:
- OK432 which is picibanil/anti-malignant tumor haemolytic streptococcus formulation is added in the culturing liquid to carry out culturing for another day

Day7

- Step of collecting mature dendritic cell

- Tax peptide pulse step (room temperature 22 ± 3°C, for 90 minutes)
- The step of washing by centrifugation (twice)

This enables obtainment of a Tax peptide pulse dendritic cell.

- Final formulating step:

  - Preparation of cell density (1 × $10^7$ cells/mL)
  - Mix with cell cryopreserving liquid (5 × $10^6$ cells/mL)
  - Filling to cryo tube or the like (2.5 × $10^6$ cells/0.5 mL/tube)
  - Labelling to a cryo tube (cryopreserved cell) and ample of the added thawing agent (physiological saline)
  - Preservation of a formulation: cryopreserved cell (≤ -80°C) Physiological saline (room temperature)

Final formulation (cryopreserved cell + added thawing agent) is obtained.
The following is carried out upon shipping.
Standard test (manufacturing organization):
Live cell count, live cell rate, surface character analysis (FCM), sterile test, endotoxin, mycoplasma (PCR method)

(Result)

Accordingly, it was confirmed that a Tax peptide pulse dendritic cell is manufactured.

(Example 8: Feature analysis and MLR of obtained cell)

Mix lymphocyte reaction (MLR) was carried out to confirm the function of the dendritic cell (P2Exp21 DC product (S/P = Pulmozyme absent/present) obtained in the Example above. In the MLR analysis, co-culturing with homologous CD4-positive cell was carried out to assess the lymphocyte stimulation ability.
The procedure of MLR is as shown below.

(Material and method)

{Required articles}

15mL conical tube
FACS tube with a cap
blue tube
2/5/10 mL pipette
PBS
PBS/FBS: PBS/2% FBS, PBS/1% FBS
RPMI
R10: RPMI/10% FBS
96 well U bottom plate (Nunc Cat#163320)
BD IMag Human CD4 T Lymphocyte Enrichment Set-DM(BD Biosciences, Cat#557939)
CFSE (Sigma Aldrich, Cat #150347594)

*Adjusting-dispensing to 1 mM is carried out for cryopreserving at -20°C. Freezing-thawing is carried out four times and then the cell is disposed of (tally marks are written on the lid)

V450-Anti CD3 Ab (BD Horizon, Cat#560365)
APC Cy7-Anti CD4 Ab (BioLegend, Cat#300517)
Lymphoprep
CELLBANKER1
Micropipette (2, 10, 20, 100, 200, 1000 μL)
Chip with a filter (10, 20, 100, 200, 1000 μL)
Pipette aid

{Required device}

Centrifugation device with ensured quality
Hemocytometer counter
Inverted microscope

(Separation of Allogeneic CD4 cell)

<Separation of PBMC>

1) 10 mL of a healthy person's peripheral blood that had been collected using heparin was separated into PBMC with a specific gravity centrifugal separation method using Lymphoprep.

<Separation of CD4 cell>

1) 1% FBS/2 mM EDTA/PBS...{A} was prepared in the following manner.

PBS 16 mL + BSA 160 µL + EDTA 64 µL (0.5 M)

2) {A} was added to the PBMC so as to achieve 10 × 10^6/mL and suspension was carried out with P1000 for transfer to a FACS tube with a lid.
3) 5 µL/1 × 10^6 of Biotinylated Human T Lymphocyte Enrichment Cocktail were added for pipetting with P1000 and incubation was carried out for 15 min with RT.
4) {A} was added to carry out dilution in a measuring cylinder up to 4 mL to carry out centrifugation (700 g, 5 min, break 1) and remove supernatant (using a P1000chip).
5) 1 mL of {A} was added with P1000 to carry out suspension and 3 mL of {A} was further added.
6) Centrifugation (700 g, 5 min, break 1) was carried out and supernatant was removed (using a P1000 chip).
7) Steps 5) to 6) were performed again.
8) 5 µL/1 × 10^6 of BD IMag Streptavidin Particle Plus-DM were added after vortex.
9) Suspension (use P200 if not loosened) was carried out with P1000 and incubation was carried out for 30 min with RT.
The following steps were carried out while paying attention particularly so as not to create foam.
10) 1.6 mL of {A} was added (adjust to 2 mL, so as to be in the range of 20 to 80 × 10^6/mL) (tube {1}).
11) The tube {1} is left for 6 min to a Magnet.
12) A 2 mL pipette was used to collect supernatant to a new tube {2} so as not to contact the bottom of the tube {1}.
13) 2 mL of {A} was added to the tube {1} to carry out suspension and the tubes {1} and {2} were left for 6 min to a Magnet.
14) 2 mL pipette was used to collect supernatant from tube {2} to 15 mL tube and then the supernatant of the tube {1} was collected to tube {2}.
15) The tube {2} was left for 6 min at a Magnet and the 15 mL tube was left on ice.
16) A 2 mL pipette was used to collect supernatant from the tube {2} to a 15 mL tube.
17) Dilution was carried out with R10 (RPMI/10% FBS) in a measuring cylinder up to 14 mL.
18) After centrifugation (700 g, 5 min), supernatant was removed.
17) Suspension was carried out to 10 mL of RPMI and the number of cells was counted.
18) After centrifugation (700 g, 5min), supernatant was removed.
19) Separation was carried out on the day before MLR setup or earlier to freeze all cells. Adjustment was made to 5 × 10^6/mL with CELLBANKER1 for dispensation of 5 × 10^6/tube each to put in a bicelle for preservation at -80°C.

(MLR Day 1)

<Thawing of DC formulation>

1) Thawing of a DC formulation and counting of the number of dendritic cells were carried out in accordance with the preparation of an ATL-DC-101 final administration formulation (given that PBS is used instead of physiological saline).
2) Based on the number of the counted dendritic cells, R10 (RPMI/10% FBS) was used to adjust about 0.3 mL of 2 × 10^5/mL DC cell suspension liquid (A). The remaining DC was used for staining a cell surface antigen.
The cell suspension liquid loosely underwent a vortex and then aliquoting carried out as shown below.
3) 0.5 mL of 2 × 10^4/mL DC cell suspension liquid (B) was prepared from the DC cell suspension liquid (A).
4) 0.5 mL of 2 × 10^3/mL DC cell suspension liquid (C) was prepared from the DC cell suspension liquid (B).
5) **As shown in the drawing below, 100 μL of each of the cell suspension liquids (A), (B) and (C) were put into F-7, 8, E-7, 8, D-7, 8 of a 96 well U bottom plate. In addition, 100 μL of R10 was put into C-5, 7, 8, D-5, E-5, F-5.

[Chemical 1]

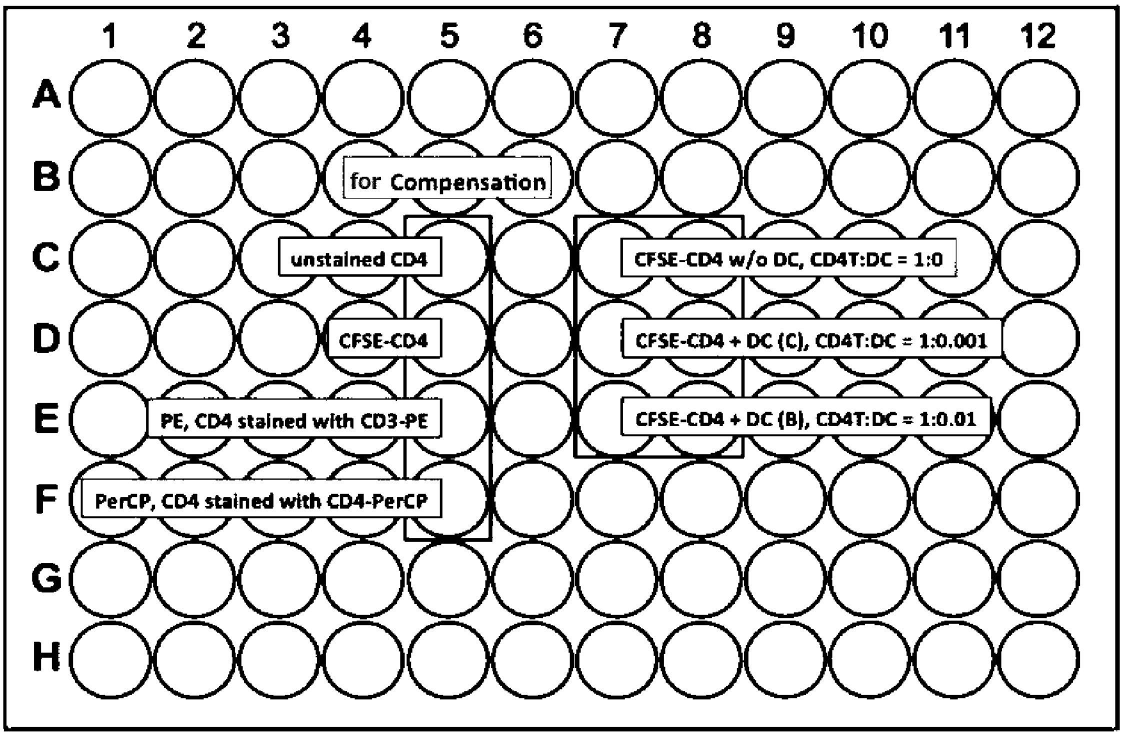


6) In order to prevent evaporation, Milli-Q water was added 200 μL each to the outermost well (36well) of the 96well plate.
7) The plate was cooled at 4°C. (Hereafter, attention was paid so that water drop of the lid would not touch the Buffer when taking out.)

**Another set of same plate was made for microscopy. There is no need for a set for doublet or compensation.

<CD4 cell thawing and CFSE label>

1) 14 mL of R10 was put into a 15 mL conical tube and warmed in a 37°C water bath.

2) After the R10 had been warmed up, a CD4 cell freezing tube was quickly melted in the 37°C water bath.
3) Immediately after the thawing, the cell liquid in the tube was transferred to a warmed R10, the cap was firmly closed and the liquid was inverted and stirred.
4) Centrifugation (700 g, 5 min, RT)
5) After sucking supernatant with an aspiration pipette, 13 mL of R10 was used to suspend the cell.
6) Centrifugation (700 g, 5 min, RT).
7) After sucking supernatant with an aspiration pipette, 10 mL of R10 was used to suspend the cell.
8) The number of cells was counted. (cell suspension: trypan blue = 1:1)
9) Centrifugation (700 g, 5 min, RT)
10) After sucking supernatant with a pipette, R10 was used to adjust the cell to 2 × 10^6/mL.
11) Cell suspension liquid was added to C-5, E-5 and F-5 of the plate 100 μL each to cool the platelet again at 4°C.
12) All of the remaining cells were transferred to an FACS tube with a cap.
13) Centrifugation (700 g, 5 min, RT)
14) Supernatant was sucked with a pipette.
15) The cell was suspended with 4mL of PBS.
16) Steps 13) to 15) were repeated twice more. (No need to transfer to a new FACS tube with a cap.)
17) Centrifugation (700 g, 5 min, RT)
18) Supernatant was sucked with a P1000 chip.
19) The cell was suspended with 0.4 mL of PBS so as to achieve 0.5 to 1 × 10^7 cells/mL.
20) The electricity of the clean bench was turned off.
21) 2 μL of CFSE (1 mM) was added and the tube was shaken for sufficient mixing (final conc 5 μM).
21) The cell was warmed with an incubator (37°C, 15 min).
22) The cell underwent simple vortex and 3.6 mL of R10 was added to achieve a total of 4 mL.
23) Centrifugation (700 g, 5 min, RT)
24) Supernatant was sucked with a P1000 chip.
25) Simple vortex was made and 4 mL of R10 was added.
26) Steps 23) to 25) were repeated twice more.
27) Centrifugation (700 g, 5 min, RT)
28) Supernatant was sucked with a P1000 chip.
29) The cell was adjusted with R10 to 2 × 10^6/mL.
30) Cells were added to D-5, C-7, 8, D-7, 8, E-7, 8, F-7, 8 of the plate 100 μL each (pipetting is unnecessary).
31) The plate was shield from light with an aluminum foil (not sealed).
31) Incubation was carried out for 4 days at 37°C.

(MLR Day 5)

<Collection/washing, FCM analysis>

1) 1% FBS/PBS was dispensed to seven blue tubes 3 mL each.
2) The cell of each tube was collected with a 96 well plate to a blue tube. Doublet was collected into one. (P200 was used to carry out firm suspension for collection. Attention was paid so as not to create foam.)
3) After centrifugation (700 g, 5 min, RT), supernatant was removed with a decant and a vortex was made.
4) 1% FBS/PBS was added 4 mL each.
5) Steps3) and 4) were repeated again.
6) After centrifugation (700 g, 5 min, RT), supernatant was removed with a decant and a vortex was made.
7) 1% FBS/PBS was added 100 μL each.
8) An antibody was added to each and a vortex was made. (Antibody added volume: V450-CD3 Ab 5 μL, APC Cy7-CD4 Ab 1 μL)
9) Light was shield for 20 min at 4°C (a tube stand was placed on ice).
10) 4 mL of 1% FBS/PBS was added to carry out centrifugation of 440 g for 5 min at 4°C.
11) Supernatant was removed with a decant and a vortex was made.
12) Steps 9) and 10) were repeated again.
13) Suspension was carried out with 450 μL of 1% FBS/PBS and a vortex was made.
14) FCM analysis was performed.

(Used specimen)

DC:

P2Exp14 CE-DC190625 (2.5 × 10^6 cells/tube × 1 tube)
P2Exp21-S-DC200129 (2.5 × 10^6 cells/tube × 1 tube)
P2Exp21-P-DC200129 (2.5 × 10^6 cells/tube × 1 tube)
CD4 cells: HD-CD4-200124 (4.8 × 10^6 cells/tube × 2 tubes)

(Result)

- P2EXP21 DC products (S/P) were both MLR reaction positive. In addition, it was recognized that the reaction intensity tended to be slightly higher for S, but the difference was not explicit.

[Table 6]

MLR

| DC | Freezing date | Thawing date | Preservnation period (day) | Count of number of cells 7-22um Total cell count (x10^6 cells) | Live cell count (x10^6 cells) | Live cell rate (%) |
|---|---|---|---|---|---|---|
| P2Exp14-CE DC190625 | 6/25/19 | 2/6/20 | 226 | 2.04 | 1.79 | 88 |
| P2Exp21-S-DC200129 | 1/29/20 | 2/6/20 | 8 | 2.03 | 1.94 | 96 |
| P2Exp21-P-DC200129 | 1/29/20 | 2/6/20 | 8 | 2.02 | 1.82 | 90 |

| CD4+T | Freezing date | Thawing date | Preservnation period (day) | Count of number of cells Total cell count (x10^6 cells) | Live cell count (x10^6 cells) | Live cell rate (%) |
|---|---|---|---|---|---|---|
| CD4-191016 (After CFSE labeling) | 10/16/19 | 2/6/20 | 113 | 13.8 | - | 93.3 |

* After freezing, 0.6×10^6 CD4 cells (counted by visual observation) were seeded and CFSE labeling to the remaining cells were performed.

(Result)

The result is shown in Figure 11.

As shown, regardless of the presence/absence of the DNase treatment, a CD4-positive cell with weakened CFSE was present in the DC obtained with the method of the present disclosure. Thus, it was shown that there is CD4-positive cell stimulation ability.

Figure 11 shows the function test result of a dendritic cell product of the samples below.

1) P2Exp14 CE-DC190625 (apheresis raw material by a conventional method) (used as a positive control: a dendritic cell product that had been manufactured from an apheresis raw material using a conventional method and a product that passed the function test previously)
2) P2Exp21-S-DC200129 (apheresis raw material by Optia (method of the present disclosure), DNase treatment absent)
3) P2Exp21-P-DC200129 (apheresis raw material by Optia (method of the present disclosure), DNase treatment present)

It has been understood from Figure 11 that all dendritic cell products 1), 2) and 3) have the CD4+T cell stimulation ability in a dendritic cell concentration-dependent manner. There was no explicit difference in the function of the dendritic cell due to the presence/absence of DNase.

Accordingly, it has been proven that the Tax peptide pulse dendritic cell manufactured with the monocyte obtained with the method of the present disclosure is functional.

(Example 9: Preparation flow from P1 ATL-DC final product formulation to administration)

A final product formulation (cryopreserved cell + added thawing liquid) undergoes shipping judgement test: live cell count, sterile test, endotoxin, and mycoplasma (PCR method), and is then administered to a patient.

Upon administration, the cells are thawed to prepare cell suspension liquid for subcutaneous administration and administration of $5 \times 10^6$ cells per dose is carried out three times every two weeks.

Test within step (CPC): (final centrifugation supernatant)
sterile test, endotoxin
are carried out.

(Example 10: Monocyte collection and lymphocyte removal)

The same protocol as Example 1 was used in Example 10, but the collection preference value was changed as shown below.

0 to 1 hour after initiation of apheresis: collection preference value + 30

1 to 3 hours after initiation of apheresis: collection preference value + 20

Collection was carried out under this condition, wherein a monocyte with high purity was obtained (Table 7). Since the obtainment of a monocyte with high purity reduced the subject's lymphocyte removal rate, it was attempted to add apheresis (lymphocyte removal mode (automatically set preference value (30 to 50))) at an automatically set preference value of a secondary chamber separation system (MNC) program after the monocyte collection. Specifically, the preference was changed back to the automatically set value after monocyte collection to carry out collection.

[Table 7]

| Donor ID | | | | HD10 | HD25 | HD04 |
|---|---|---|---|---|---|---|
| Collected specimen | Manufacturing raw material | WBC | x10^8 cells | 31.1 | 17.6 | 23.4 |
| | | Neutrophil | x10^8 cells | 3.0 | 2.0 | 1.6 |
| | | Monocyte | x10^8 cells | 13.8 | 7.9 | 8.5 |
| | | Lymphocyte | x10^8 cells | 12.6 | 6.6 | 12.3 |
| | | HCT | % | 1.8 | 0.1 | 0.2 |
| | | Plt | 10,000/μL | 97.5 | 17.7 | 16.9 |
| | Ly removal | WBC | x10^8 cells | 20.6 | 19.5 | 12.1 |
| | | Neutrophil | x10^8 cells | 1.0 | 1.3 | 0.5 |
| | | Monocyte | x10^8 cells | 2.6 | 4.5 | 1.9 |
| | | Lymphocyte | x10^8 cells | 16.7 | 13.2 | 9.5 |
| | | HCT | % | 2.5 | 1.9 | 0.4 |
| | | Plt | 10,000/μL | 156.1 | 103.7 | 46.2 |

(Result)

Addition of this step enabled removal of lymphocytes equal to the number of lymphocytes that was able to be removed upon conventional collection.

(Example 11: Treatment)

When the condition employed in Example 10 is used, the condition is used as apheresis upon collection of a raw material of a dendritic cell, wherein the purpose is to collect a monocyte that is the raw material of a dendritic cell by a centrifugal separation method, which is also for treatment purpose (Therapeutic cytapheresis).

In this regard, this condition is utilized to remove an immune cell that negatively controls antitumor immunity such as Treg. A dendritic cell administered thereafter can present an antigen to a naive T lymphocyte that newly proliferated under cytokine environment produced to maintain homeostasis.

In this Example, a lymphocyte removal mode (automatically set preference value (30 to 50)) is combined to enable apheresis upon raw material collection to collect a monocyte with high purity by a centrifugal separation method, which enabled removal of lymphocytes at level $10^9$ which is the level achieved in a normal treatment apheresis (Table 8).

[Table 8]

| | | | | | |
|---|---|---|---|---|---|
| Ly removal | WBC | x10^8cells | 20.6 | 19.5 | 12.1 |
| | Neutrophil | x10^8 cells | 1.0 | 1.3 | 0.5 |
| | Monocyte | x10^8 cells | 2.6 | 4.5 | 1.9 |
| | Lymphocyte | x10^8 cells | 16.7 | 13.2 | 9.5 |
| | HCT | % | 2.5 | 1.9 | 0.4 |
| | Plt | 10,000/μL | 156.1 | 103.7 | 46.2 |

The lymphocyte removal in Table 8 does not include lymphocyte removal by the monocyte collection step, and the added number of lymphocytes of the monocyte collection step and the lymphocyte removal step is as shown below.

HD10: 29.3×10^8
HD25: 19.8×10^8
HD04: 21.8×10^8

The monocyte fractions and platelet fractions increase when a value is set higher than an automatically set value and the neutrophils and red blood cell fractions increase by setting the value low, which is considered to reduce the lymphocyte removal rate. In addition, the lymphocyte removal after the monocyte collection step would be collection after the density gradient centrifugal separation layer has stabilized, which is thus considered preferable to be performed for 20 minutes or longer (30 minutes or longer if possible).

Data showing comparison in the monocyte fractionation (taking out) step and the lymphocyte fractionation (taking out) step is shown below.

[Table 9]

| Donor ID | | | HD10 | HD25 | HD04 |
|---|---|---|---|---|---|
| Mo fractionation | Monocyte | x10^8 cells | 13.8 | 7.9 | 8.5 |
| | Lymphocyte | x10^8 cells | 12.6 | 6.6 | 12.3 |
| | Monocyte/lymphocyte | | 1.10 | 1.20 | 0.69 |
| Ly fractionation | Monocyte | x10^8 cells | 2.6 | 4.5 | 1.9 |
| | Lymphocyte | x10^8 cells | 16.7 | 13.2 | 9.5 |
| | Monocyte/lymphocyte | | 0.16 | 0.34 | 0.20 |

As a result, removal of a suppressive immune cell such as Treg, which configures an immunotolerant state of a cancer patient, and also induction of activation of antitumor immunity by a lymphocyte or cytokine newly produced by the maintained homeostasis of an organism can be expected.

(Note)

Although the present disclosure has been exemplified using a preferable embodiment of the present invention as described above, it is understood that the scope of the present disclosure should be interpreted by the Claims alone. It is also understood that any patent, any patent application, and any references cited herein should be incorporated herein by reference in the same manner as the contents are specifically described herein. The present application claims priority to Japanese Patent Application No. 2020-27595 filed on February 20, 2020 and Japanese Patent Application No. 2020-81523 filed on May 1, 2020. The entire content thereof is incorporated herein by reference.

[Industrial Applicability]

The technique provided in the present disclosure can be utilized in the business of manufacturing a therapeutic drug for adult T-cell leukemia and the like.

## Claims

**1.** A method of producing a monocyte preparation from a blood preparation, comprising:

1) the step of obtaining a blood preparation from a subject; and
2) the step of enriching a monocyte based on specific gravity and cell size in the blood preparation.

**2.** The method of claim 1, wherein the step 2) enriches a monocyte based on specific gravity and then enriches a monocyte based on cell size in the blood preparation.

**3.** A method of producing a monocyte preparation from a blood preparation, comprising:

1) the step of obtaining a blood preparation with a blood cell concentration lower than a predetermined value from a subject; and
2) the step of enriching a monocyte based on specific gravity and/or cell size in the blood preparation.

**4.** The method of claim 3, wherein the predetermined value is a value for taking a buffy coat.

**5.** The method of claim 3, wherein the blood cell concentration is adjusted while referring to a collection preference.

**6.** The method of claim 5, wherein the collection preference is set at a value higher than 60 - {(0.2 $\times$ white blood cell count (1000/$\mu$l)) + (0.08 $\times$ platelet count (1000/$\mu$l)}.

7. The method of claim 5 or 6, wherein the collection preference is set at 60 - {(0.2 × white blood cell count (1000/μl)) + (0.08 × platelet count (1000/μl)} + 10 to 20.

8. The method of any one of claims 5 to 7, wherein a predetermined value of the collection preference is 30 to 50.

9. The method of any one of claims 5 to 8, wherein the collection preference is set to be higher than a predetermined value by 10 to 30.

10. The method of claim 9, wherein the collection preference is set to be higher than a predetermined value by 20 to 30.

11. The method of any one of claims 5 to 10, wherein the collection preference is set at 40 to 80.

12. The method of any one of claims 1 to 11, wherein the enriching step comprises causing a spillover.

13. A method of producing a monocyte preparation from a blood preparation, comprising:

1) the step of obtaining a blood preparation from a subject; and
2) the step of enriching a monocyte based on specific gravity and/or cell size from the blood preparation, wherein the enriching step comprises causing a spillover of a lymphocyte fraction so as to remove a lymphocyte fraction.

14. The method of claim 13, wherein, when a value of a treatment blood volume (reference treatment blood volume) at a time point of when a red blood cell is continuously overflowing from a chamber is 1 as a spillover, the spillover is performed with a treatment blood volume set at a value greater than a reference treatment blood volume.

15. The method of claim 14, wherein the treatment blood volume is at least 1.2-fold of the reference treatment blood volume.

16. The method of any one of claims 1 to 15, **characterized in that** the monocyte preparation is directly obtained from a blood cell preparation obtained from the subject.

17. The method of any one of claims 1 to 16, wherein the enrichment is carried out in 5 hours or less after the blood preparation is obtained from the subject.

18. The method of any one of claims 1 to 17, wherein the separation by cell size is carried out in 3 hours or less after the separation by specific gravity.

19. The method of any one of claims 1 to 18, **characterized in that** the monocyte preparation comprises a monocyte with a concentration that is about 30% or greater with respect to all nucleated cells.

20. A method of producing a monocyte preparation from a blood preparation, comprising:

1) the step of obtaining a blood preparation from a subject;
2) the step of adding a cryoprotectant agent to the blood preparation; and
3) the step of separating the blood preparation added with the cryoprotectant agent by specific gravity to obtain a fraction that is heavier than a lymphocyte.

21. The method of claim 20, wherein the blood preparation had went through apheresis separation.

22. The method of claim 20 or 21, wherein the cryoprotectant agent comprises a component selected from the group consisting of hydroxyethyl starch, sucrose, lactose, glucose, mannitol, trehalose, dimethyl sulfoxide (DMSO), glycerol, polyethylene glycol, propylene glycol, polyvinyl pyrrolidone, sorbitol and dextran.

23. The method of any one of claims 20 to 22, **characterized in that** the enrichment is carried out after cryopreserving.

24. The method of any one of claims 20 to 23, **characterized in that** the separation by specific gravity is centrifugal separation with a specific gravity of 1.074 to 1.076.

25. The method of any one of claims 20 to 24, wherein the separation by specific gravity is carried out under following

centrifugal separation conditions:

First centrifugal separation
225 mL centrifuge tube, 300 × g to 400 × g, 5 minutes, room temperature
Second to third centrifugal separations
225 mL centrifuge tube, 250 × g to 350 × g, 5 minutes, room temperature

**26.** The method of any one of claims 20 to 25, where the enrichment is carried out after carrying out the apheresis treatment of any one of claims 1 to 19.

**27.** A method of producing a monocyte preparation from a blood preparation, comprising:

1) the step of obtaining a monocyte preparation comprising a cryoprotectant agent from an obtained blood preparation; and
2) the step of washing the monocyte preparation with a washing liquid comprising 10% or less of a blood preservation liquid A liquid (ACD-A liquid) containing sodium citrate hydrate.

**28.** The method of claim 27, wherein the ACD-A liquid is present at 6% or less.

**29.** The method of claim 28, wherein the ACD-A liquid is present at 5.5%.

**30.** The method of any one of claims 20 to 29, comprising washing of the blood preparation with a washing liquid having a composition of 5.5% ACD-A liquid/1% human serum albumin (HSA)/RPMI-1640.

**31.** The method of any one of claims 1 to 30, further comprising practice of a dead cell removal treatment.

**32.** The method of claim 31, wherein the dead cell removal treatment comprises removal of also a lymphocyte and a platelet.

**33.** The method of claim 31 or 32, wherein the dead cell removal treatment comprises specific gravity centrifugal separation.

**34.** The method of claim 33, **characterized in that** the specific gravity centrifugal separation is carried out using a density medium Iodixanol aqueous solution.

**35.** The method of claim 34, wherein the Iodixanol aqueous solution is provided at 60 w/v%.

**36.** The method of any one of claims 1 to 35, wherein the enrichment is carried out with or without addition of DNase.

**37.** The method of claim 36, wherein the DNase comprises Pulmozyme.

**38.** A method of producing a Tax-specific dendritic cell, comprising:

the step of inducing a dendritic cell from a monocyte preparation obtained with the method of any one of claims 1 to 37; and
the step of pulsing a Tax antigen peptide with respect to the induced dendritic cell.

**39.** A method of producing a product for regenerative medicine or the like, comprising the step of filling the Tax-specific dendritic cell produced with the method of claim 38 that had been suspended in a cryoprotectant liquid into a cryo tube to render a product for regenerative medicine or the like.

**40.** A product for regenerative medicine or the like, which is produced with the method of claim 39.

**41.** A system of producing a monocyte-containing substance, comprising at least one of:

an apheresis apparatus having a function of adjusting a blood cell concentration;
a means for realizing a spillover;
a size separation part that size-separates a cell;

a cryoprotectant agent feeding part that feeds a cryoprotectant agent;
a monocyte separation part that carries out separation of a monocyte by specific gravity; and
a dead cell removal means.

**42.** The system of claim 41, further comprising at least one of a separation part by specific gravity, a cryopreserving part that cryopreserves a cell, a thawing-washing part that carries out thawing-washing, and a specific gravity centrifugal separation part that carries out specific gravity centrifugal separation.

**43.** A method of treating an adult T-cell leukemia (ATL) patient, comprising:

1) the step of obtaining a blood preparation form the patient;
2) the step of measuring a blood cell concentration in the blood preparation; and
3) the step of obtaining a blood preparation with a blood cell concentration equivalent to a predetermined value from the patient when the blood cell concentration is lower than a predetermined value,

wherein the patient is a patient receiving apheresis in order to obtain a dendritic cell formulation for treating the ATL.

**44.** The method of claim 43, wherein the predetermined value is a value for taking a buffy coat.

**45.** The method of claim 43, wherein the blood cell concentration is adjusted while referring to a collection preference.

**46.** The method of claim 45, wherein the collection preference is set at a value higher than 60 - { (0.2 × white blood cell count (1000/μl)) + (0.08 × platelet count (1000/μl)}.

**47.** The method of claim 45 or 46, wherein the collection preference is set at 60 - {(0.2 × white blood cell count (1000/μl)) + (0.08 × platelet count (1000/μl)} + 10 to 30.

**48.** The method of any one of claims 45 to 47, wherein a predetermined value of the collection preference is 30 to 50.

**49.** The method of any one of claims 45 to 48, wherein the collection preference is set to be higher than a predetermined value by 10 to 30.

**50.** The method of claim 49, wherein the collection preference is set to be higher than a predetermined value by 20 to 30.

**51.** The method of any one of claims 45 to 50, wherein the collection preference is at 40 to 80.

**52.** The method of any one of claims 43 to 51, wherein the step of obtaining a blood preparation comprises the step of taking out a monocyte and the step of taking out a lymphocyte.

**53.** The method of claim 52, wherein the step of taking out a monocyte comprises at least one characteristic of any one or more of claims 1 to 36.

**54.** The method of claims 52 to 53, wherein the step of taking out a lymphocyte comprises at least one characteristic of any one or more of claims 43 to 51 and is performed for at least 20 minutes or longer when performed following the step of taking out a monocyte.

**55.** The method of claim 54, wherein the step of taking out a lymphocyte is performed for 30 minutes or longer.

**56.** The method of any one of claims 52 to 55, wherein the step of taking out a lymphocyte is performed with the collection preference set at 30 to 50.

**57.** A program that has a computer execute a method of treating an adult T-cell leukemia (ATL) patient, the method comprising:

1) the step of obtaining a blood preparation from the patient;
2) the step of measuring a blood cell concentration in the blood preparation; and
3) the step of obtaining a blood preparation with a blood cell concentration equivalent to a predetermined value from the patient when the blood cell concentration is lower than a predetermined value,

wherein the patient is a patient receiving apheresis in order to obtain a dendritic cell formulation for treating the ATL.

**58.** The program of claim 57, wherein the predetermined value is a value for taking a buffy coat.

**59.** The program of claim 57, wherein the blood cell concentration is adjusted while referring to a collection preference.

**60.** The program of claim 59, wherein the collection preference is set at a value higher than 60 - { (0.2 × white blood cell count (1000/μl)) + (0.08 × platelet count (1000/μl)}.

**61.** The program of claims 59 to 60, wherein the collection preference is set at 60 - {(0.2 × white blood cell count (1000/μl)) + (0.08 × platelet count (1000/μl)} + 10 to 30.

**62.** The program of any one of claims 59 to 61, wherein a predetermined value of the collection preference is 30 to 50.

**63.** The program of any one of claims 59 to 62, wherein the collection preference is set to be higher than a predetermined value by 10 to 30.

**64.** The program of claim 63, the collection preference is set to be higher than a predetermined value by 20 to 30.

**65.** The program of any one of claims 59 to 64, wherein the collection preference is set at 40 to 80.

**66.** The program of any one of claims 57 to 65, wherein the step of obtaining a blood preparation comprises the step of taking out a monocyte and the step of taking out a lymphocyte.

**67.** The program of claims 66, wherein the step of taking out a monocyte comprises at least one characteristic of any one or more of claims 1 to 36.

**68.** The program of claims 66 to 67, wherein the step of taking out lymphocyte comprises at least one characteristic of any one of claims 43 to 51 and is performed for at least 20 minutes or longer when performed following the step of taking out a monocyte.

**69.** The program of claim 68, wherein the step of taking out a lymphocyte is performed for 30 minutes or longer.

**70.** The program of any one of claims 66 to 69, wherein the step of taking out a lymphocyte is performed with the collection preference set at 30 to 50.

**71.** A record medium storing a program that has a computer execute a method of treating an adult T-cell leukemia (ATL) patient, the method comprising:

1) the step of obtaining a blood preparation from the patient;
2) the step of measuring a blood cell concentration in the blood preparation; and
3) the step of obtaining a blood preparation with a blood cell concentration equivalent to a predetermine value from the patient when the blood cell concentration is lower than a predetermined value,

wherein the patient is a patient receiving apheresis in order to obtain a dendritic cell formulation for treating the ATL.

**72.** The record medium of claim 71, wherein the predetermined value is a value for taking a buffy coat.

**73.** The record medium of claim 71, wherein the blood cell concentration is adjusted while referring to a collection preference.

**74.** The record medium of claim 73, wherein the collection preference is set at a value higher than 60 - {(0.2 × white blood cell count (1000/μl)) + (0.08 × platelet count (1000/μl)}.

**75.** The record medium of claim 73 or 74, wherein the collection preference is set at 60 - {(0.2 × white blood cell count (1000/μl)) + (0.08 × platelet count (1000/μl)} + 10 to 30.

**76.** The record medium of any one of claims 73 to 75, wherein a predetermined value of the collection preference is 30

to 50.

**77.** The record medium of any one of claims 73 to 76, wherein the collection preference is set to be higher than a predetermined value by 10 to 30.

**78.** The record medium of claim 77, wherein the collection preference is set to be higher than a predetermined value by 20 to 30.

**79.** The record medium of any one of claims 73 to 78, wherein the collection preference is set at 40 to 80.

**80.** The record medium of any one of claims 71 to 79, wherein the step of obtaining a blood preparation comprises the step of taking out a monocyte and the step of taking out lymphocyte.

**81.** The record medium of claim 80, wherein the step of taking out a monocyte comprises at least one characteristic of any one or more of claims 1 to 36.

**82.** The record medium of claim 80 or 81, wherein the step of taking out a lymphocyte comprises at least one characteristic of any one or more of claims 43 to 51 and is performed for at least 20 minutes or longer when performed following the step of taking out a monocyte.

**83.** The record medium of claim 82, wherein the step of taking out a lymphocyte is performed for 30 minutes or longer.

**84.** The record medium of any one of claims 80 to 83, wherein the step of taking out a lymphocyte is performed with the collection preference set at 30 to 50.

**85.** A system for treating an adult T-cell leukemia (ATL) patient, comprising:

1) a preparation obtainment part that obtains a blood preparation from the patient,
2) a measurement part that measures a blood cell concentration in the blood preparation; and
3) an equivalent preparation obtainment part that obtains a blood preparation with a blood cell concentration that is equivalent to a predetermine value from the patient when the blood cell concentration is lower than a predetermined value,

wherein the patient is a patient receiving apheresis in order to obtain a dendritic cell formulation for treating the ATL.

**86.** The system of claim 85, wherein the predetermined value is a value for taking a buffy coat.

**87.** The system of claim 85, wherein the blood cell concentration is adjusted while referring to a collection preference.

**88.** The system of claim 87, wherein the collection preference is set at a value higher than 60 - { (0.2 $\times$ white blood cell count (1000/$\mu$l)) + (0.08 $\times$ platelet count (1000/$\mu$l)}.

**89.** The system of claim 87 or 88, wherein the collection preference is set at 60 - {(0.2 $\times$ white blood cell count (1000/$\mu$l)) + (0.08 $\times$ platelet count (1000/$\mu$l)} + 10 to 30.

**90.** The system of any one of claims 87 to 89, wherein a predetermined value of the collection preference is 30 to 50.

**91.** The system of any one of claims 87 to 90, wherein the collection preference is set to be higher than a predetermined value by 10 to 30.

**92.** The system of claim 91, wherein the collection preference is set to be higher than a predetermined value by 20 to 30.

**93.** The system of any one of claims 87 to 92, wherein the collection preference is set at 40 to 80.

**94.** The system of any one of claims 85 to 93, wherein the step of obtaining a blood preparation comprises the step of taking out a monocyte and the step of taking out lymphocyte.

**95.** The system of claim 94, wherein the step of taking out a monocyte comprises at least one characteristic of any one

or more of claims 1 to 36.

**96.** The system of claim 94 or 95, wherein the step of taking out a lymphocyte comprises at least one characteristic of any one or more of claims 43 to 51 and is performed for at least 20 minutes or longer when performed following the step of taking out a monocyte.

**97.** The system of claim 96, wherein the step of taking out a lymphocyte is performed for 30 minutes or longer.

**98.** The system of any one of claims 94 to 97, wherein the step of taking out a lymphocyte is performed with the collection preference set at 30 to 50.

**99.** An operation method of a system for treating an adult T-cell leukemia (ATL) patient, the system comprising:

1) a preparation obtainment part that obtains a blood preparation from the patient;
2) a measurement part that measures a blood cell concentration in the blood preparation; and
3) an equivalent preparation obtainment part that obtains a blood preparation with a blood cell concentration equivalent to a predetermined value from the patient when the blood cell concentration is lower than a predetermined value,

wherein the method comprises:

1) the step of obtaining a blood preparation from the patient;
2) the step of measuring a blood cell concentration in the blood preparation; and
3) the step of obtaining a blood preparation with a blood cell concentration equivalent to a predetermined value from the patient when the blood cell concentration is lower than a predetermined value,

wherein the patient is a patient receiving apheresis in order to obtain a dendritic cell formulation for treating the ATL.

**100.** The method of claim 99, wherein the predetermined value is a value for taking a buffy coat.

**101.** The method of claim 99, wherein the blood cell concentration is adjusted while referring to a collection preference.

**102.** The method of claim 101, wherein the collection preference is set at a value higher than 60 - {(0.2 × white blood cell count (1000/μl)) + (0.08 × platelet count (1000/μl)}.

**103.** The method of claim 101 or 102, wherein the collection preference is set at 60 - {(0.2 × white blood cell count (1000/μl)) + (0.08 × platelet count (1000/μl)} + 10 to 30.

**104.** The method of any one of claims 101 to 103, wherein a predetermined value of the collection preference is 30 to 50.

**105.** The method of any one of claims 101 to 104, wherein the collection preference is set to be higher than a predetermined value by 10 to 30.

**106.** The method of claim 105, wherein the collection preference is set to be higher than a predetermined value by 20 to 30.

**107.** The method of any one of claims 101 to 106, wherein the collection preference is set at 40 to 80.

**108.** The method of any one of claims 99 to 107, wherein the step of obtaining a blood preparation comprises the step of taking out a monocyte and the step of taking out a lymphocyte.

**109.** The method of claim 108, wherein the step of taking out a monocyte comprises at least one characteristic recited in any one or more of claims 1 to 36.

**110.** The method of claim 108 or 109, wherein the step of taking out a lymphocyte comprises at least one characteristic recited in any one or more of claims 43 to 51 and is performed for at least 20 minutes when performed following the step of taking out a monocyte.

**111.** The method of claim 110, wherein the step of taking out a lymphocyte is performed for 30 minutes or longer.

**112.** The method of any one of claims 108 to 111, wherein the step of taking out a lymphocyte is performed with the collection preference set at 30 to 50.

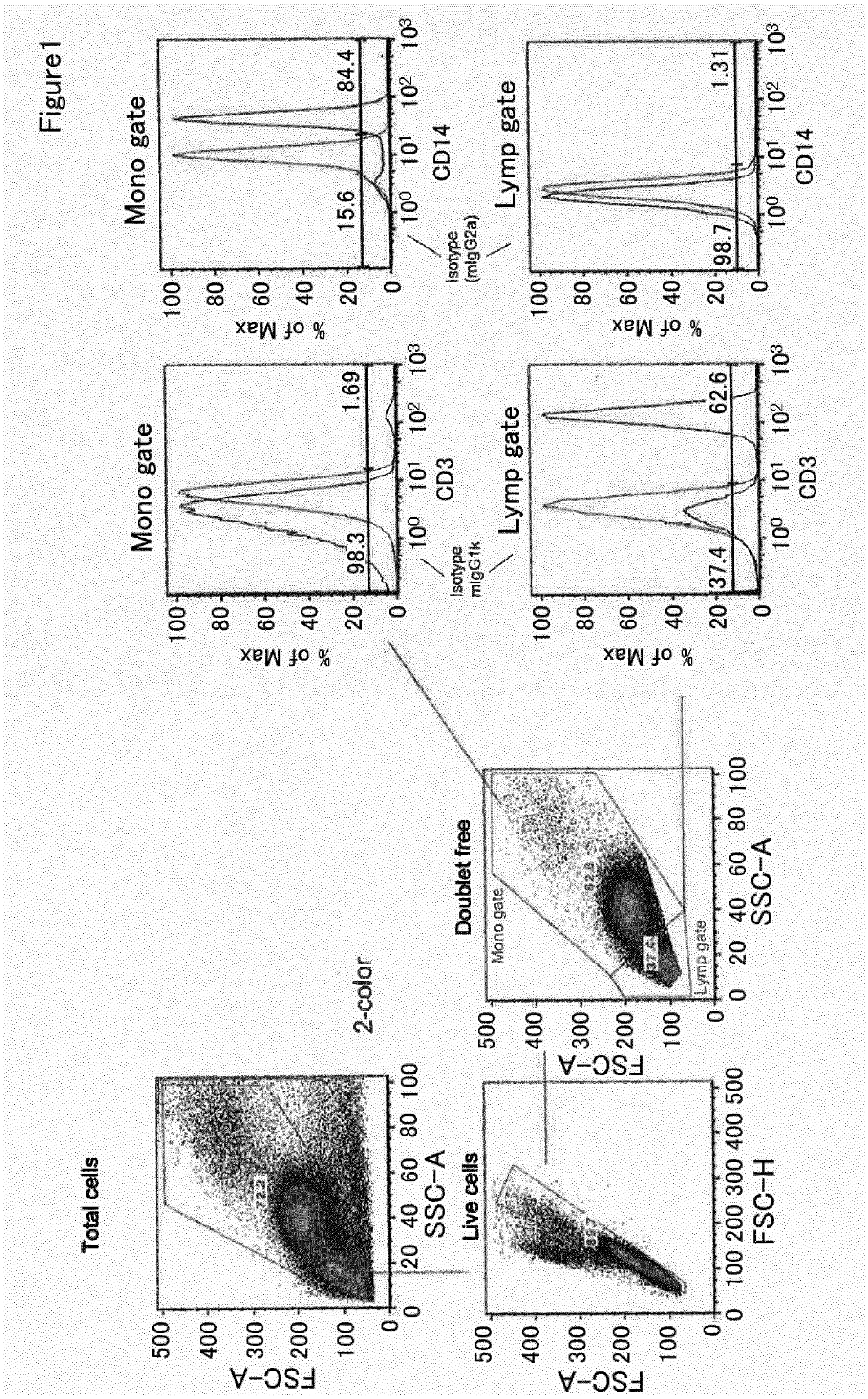
Figure1
2-color
Total cells
72.2
FSC-A
SSC-A
Live cells
89.7
FSC-H
Doublet free
Mono gate
62.8
37.4
Lymp gate
Mono gate
Lymp gate
% of Max
CD3
98.3
1.69
37.4
62.6
Isotype
mIgG1k
CD14
15.6
84.4
98.7
1.31
Isotype
(mIgG2a)

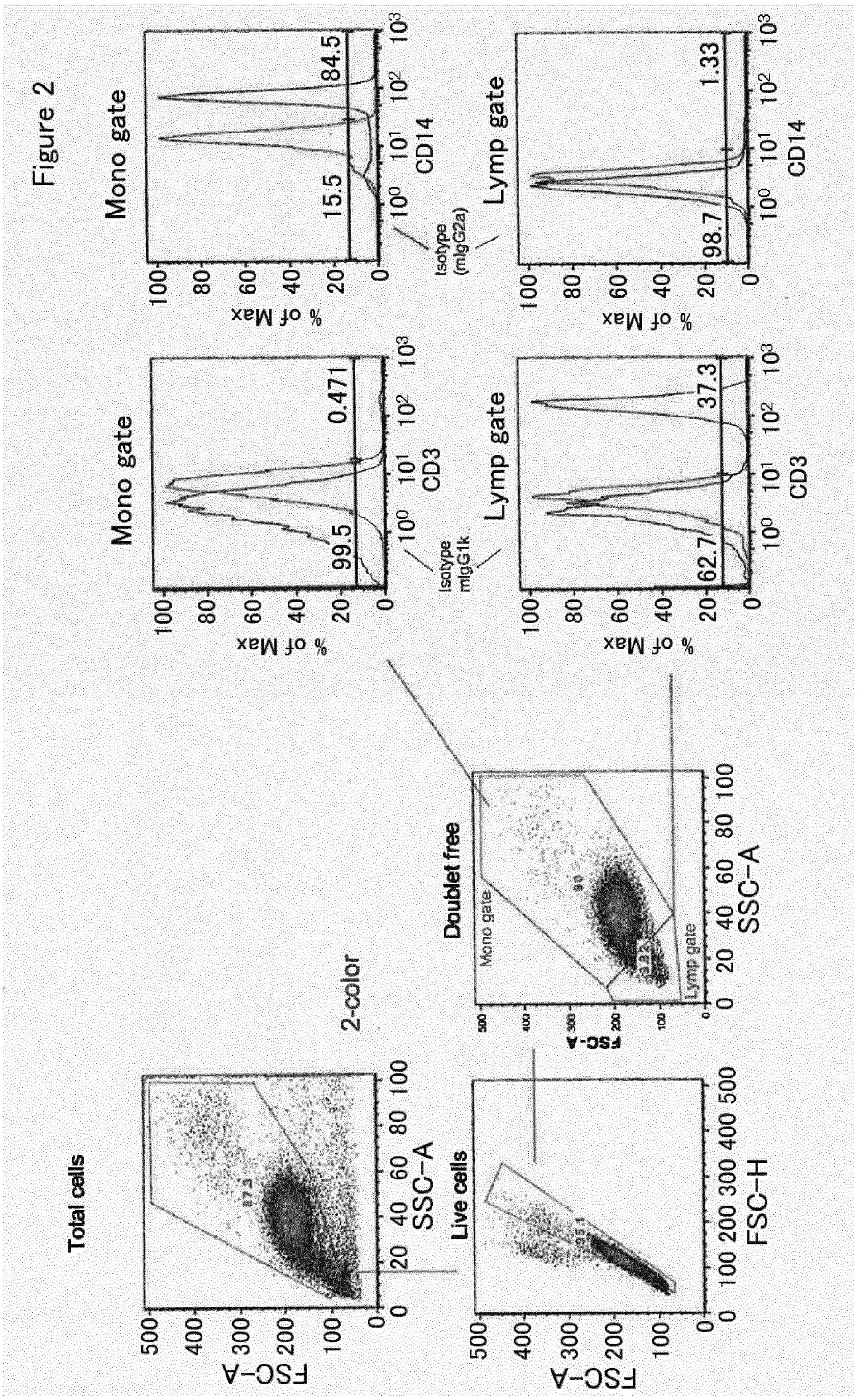
Figure 2
Total cells
FSC-A
SSC-A
87.3
Live cells
FSC-H
95.1
2-color
Doublet free
Mono gate
Lymp gate
90
9.92
Mono gate
% of Max
CD3
99.5
0.471
Isotype mIgG1k
CD14
15.5
84.5
Isotype (mIgG2a)
Lymp gate
62.7
37.3
98.7
1.33

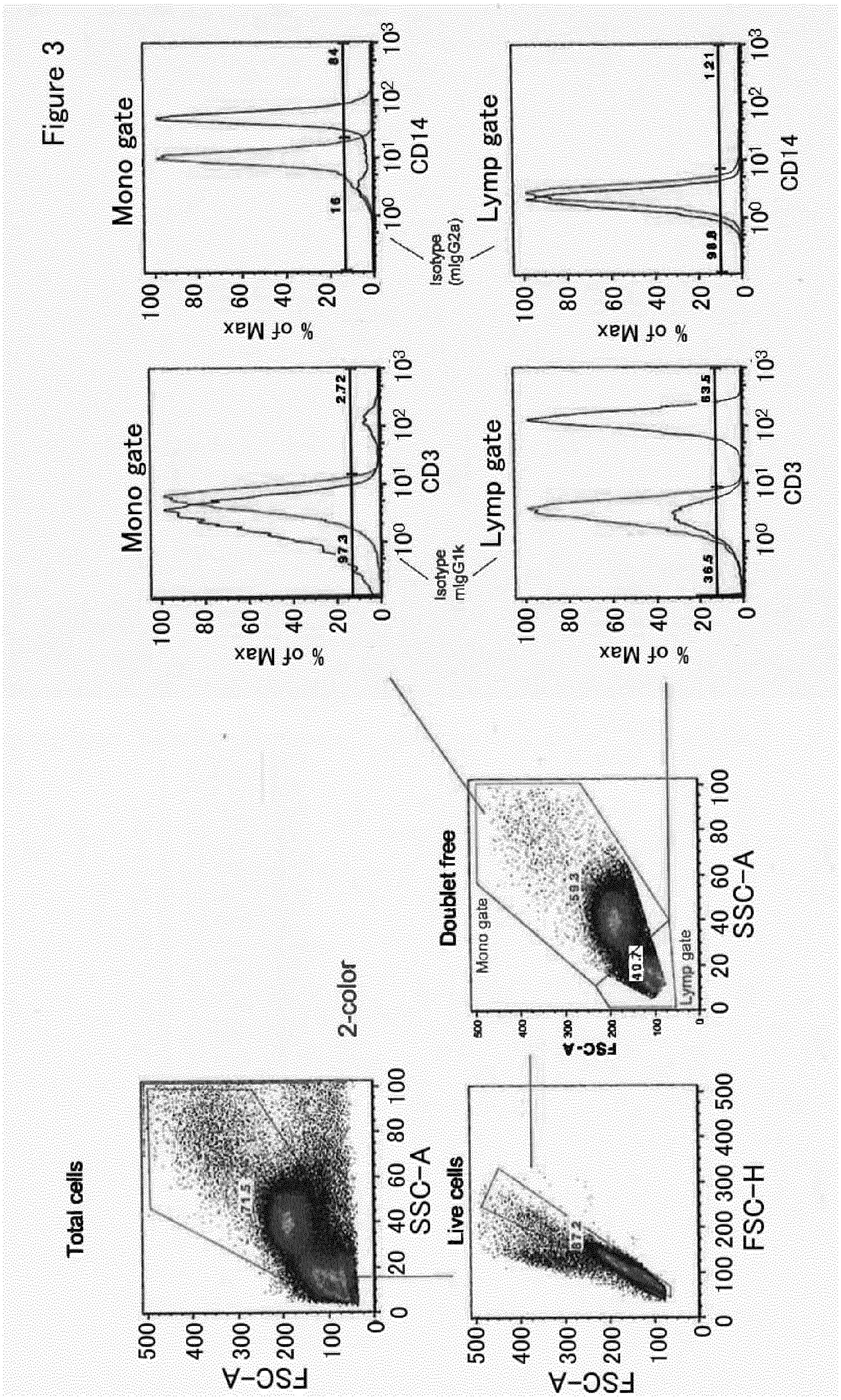
Total cells
FSC-A
SSC-A
71.5
Live cells
FSC-H
87.2
2-color
Doublet free
Mono gate
Lymp gate
59.3
40.7
Mono gate
Lymp gate
% of Max
CD3
CD14
Isotype mIgG1k
Isotype (mIgG2a)
97.3
2.72
16
84
36.5
63.5
98.8
1.21

Figure 3

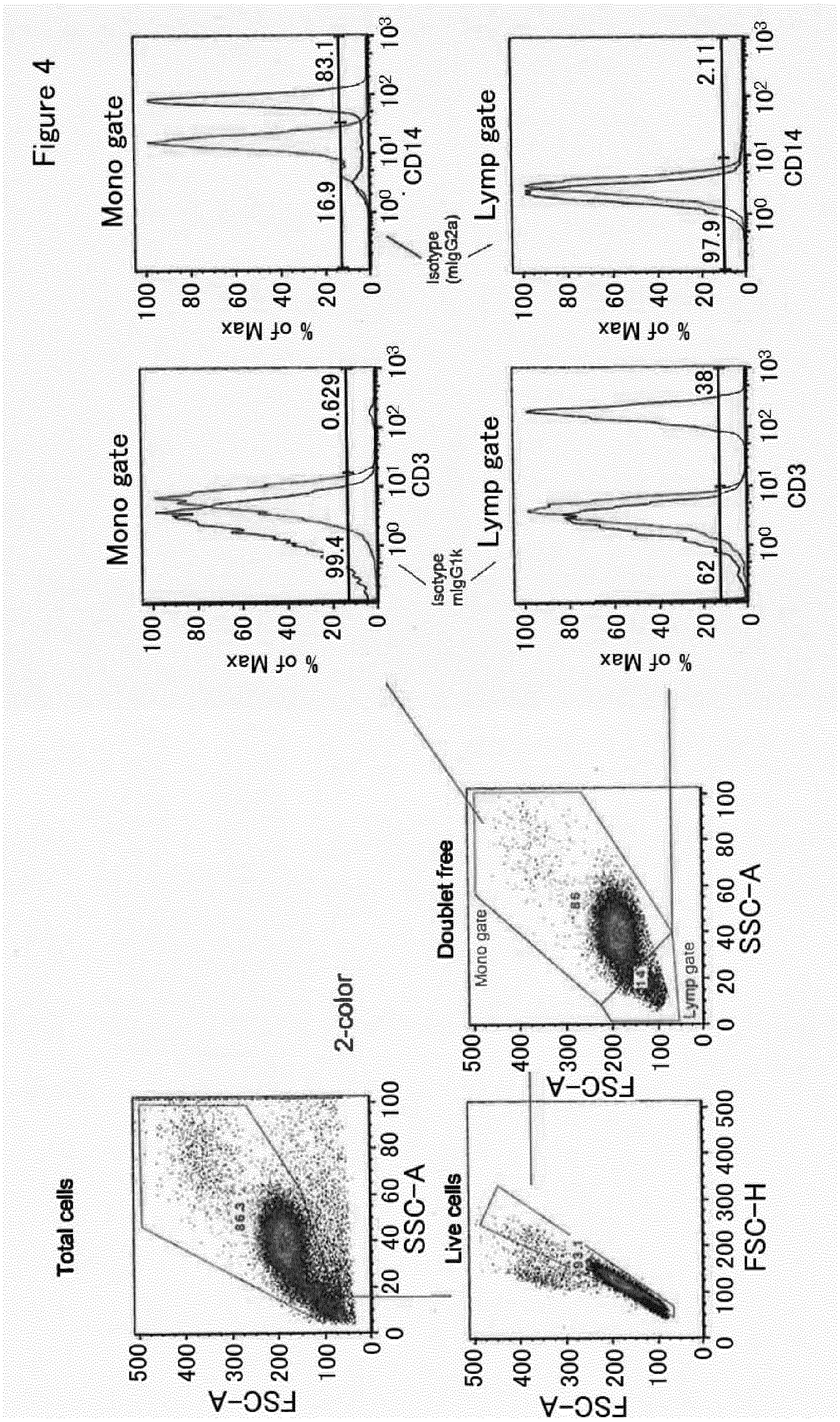
Figure 4
2-color
Total cells
86.3
FSC-A
SSC-A
Live cells
93.1
FSC-H
Doublet free
Mono gate
86
14
Lymp gate
Mono gate
% of Max
CD3
99.4
0.629
Isotype mIgG1k
Mono gate
CD14
16.9
83.1
Isotype (mIgG2a)
Lymp gate
CD3
62
38
Lymp gate
CD14
97.9
2.11

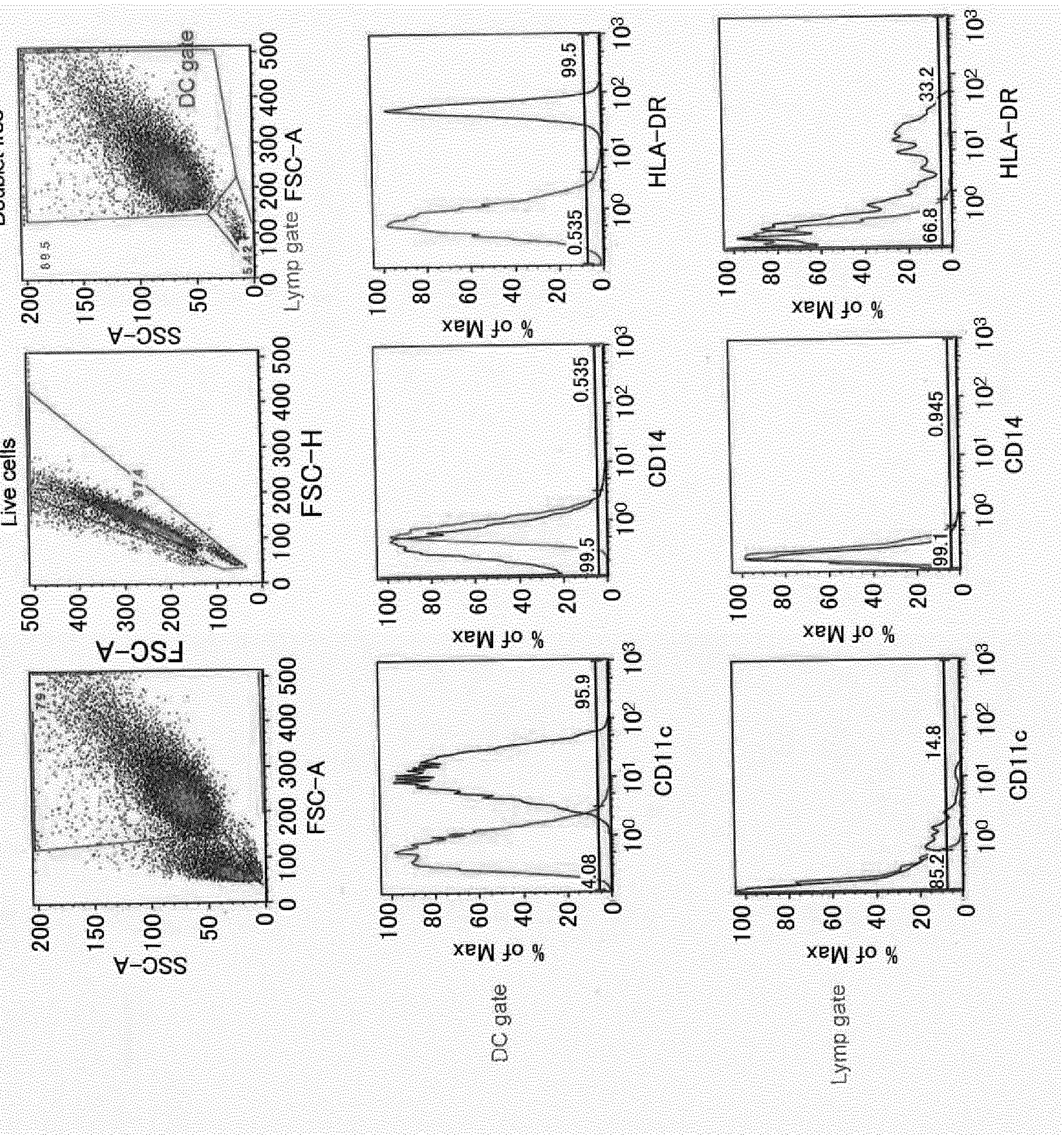

Live cells
Doublet free
DC gate
Lymp gate
SSC-A
FSC-A
FSC-H
% of Max
CD11c
CD14
HLA-DR
79.1
97.4
89.5
5.42
4.08
95.9
99.5
0.535
99.5
0.535
85.2
14.8
99.1
0.945
66.8
33.2


Figure 5

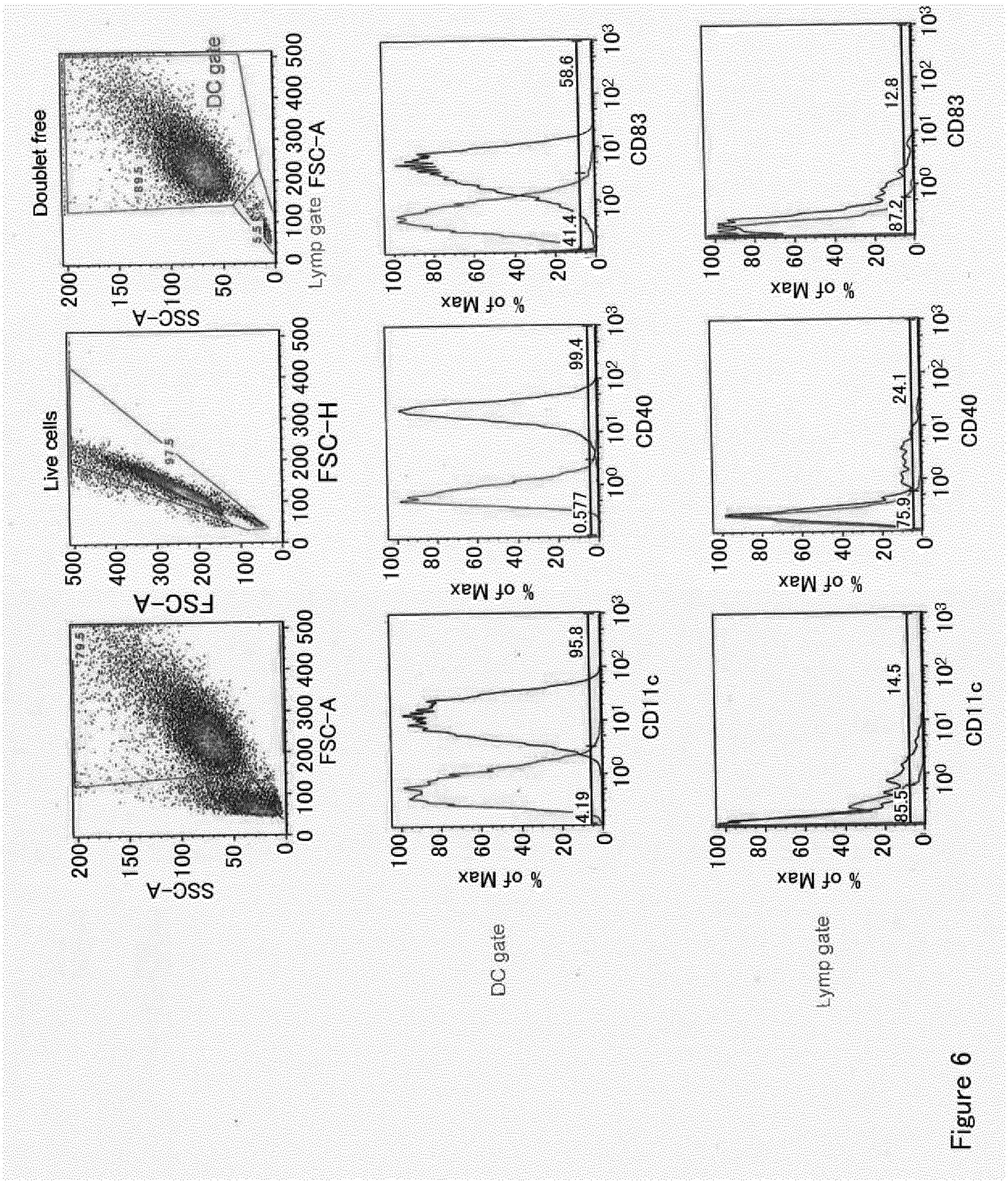
SSC-A
FSC-A
79.5
Live cells
FSC-A
FSC-H
97.5
Doublet free
SSC-A
FSC-A
DC gate
Lymp gate
89.5
5.5
DC gate
% of Max
CD11c
4.19
95.8
CD40
0.577
99.4
CD83
41.4
58.6
Lymp gate
CD11c
85.5
14.5
CD40
75.9
24.1
CD83
87.2
12.8

Figure 6

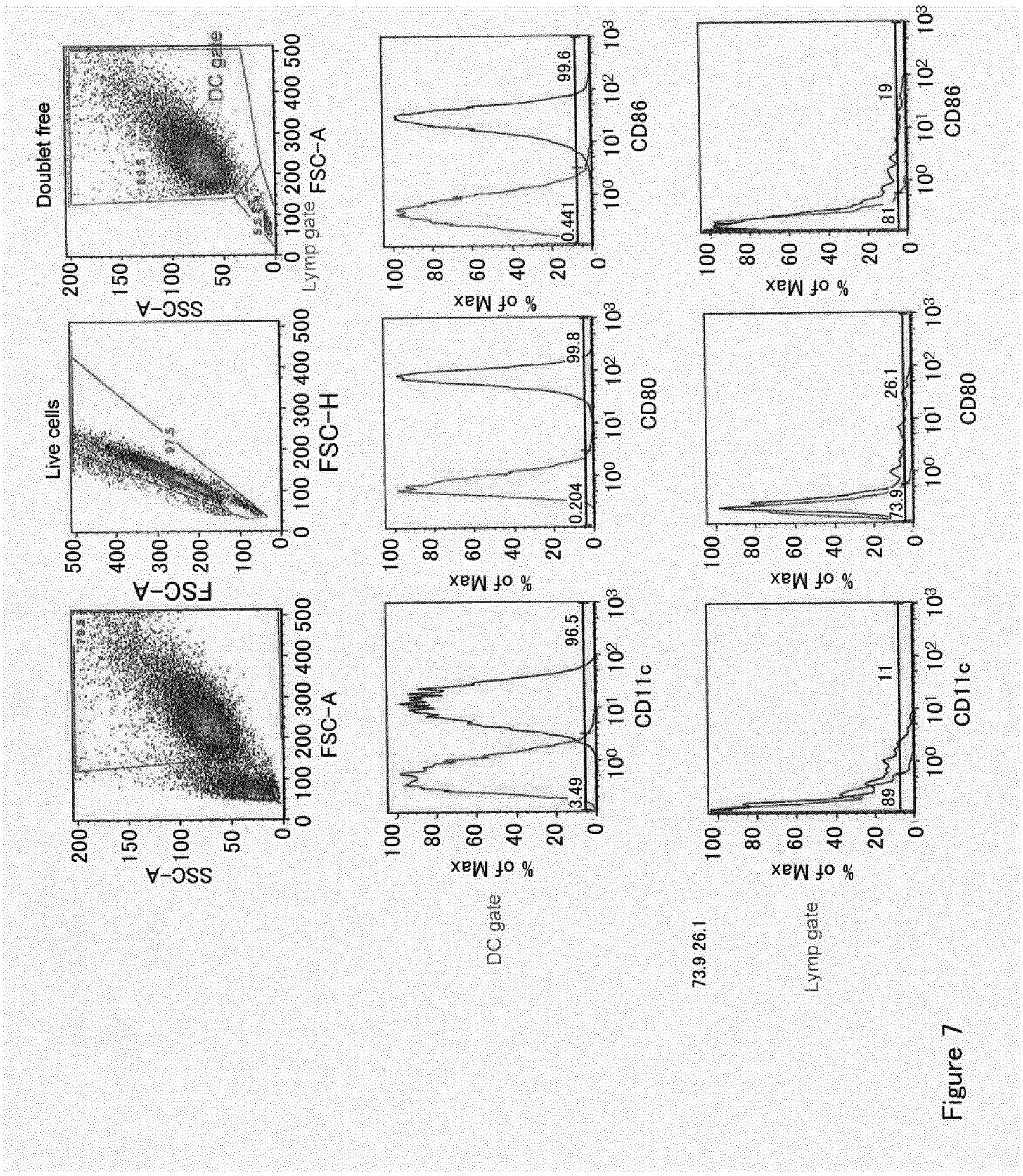
Live cells
Doublet free
DC gate
Lymp gate
SSC-A
FSC-A
FSC-H
97.5
% of Max
CD11c
CD80
CD86
3.49
96.5
0.204
99.8
0.441
99.6
89
11
73.9
26.1
81
19
73.9 26.1

Figure 7

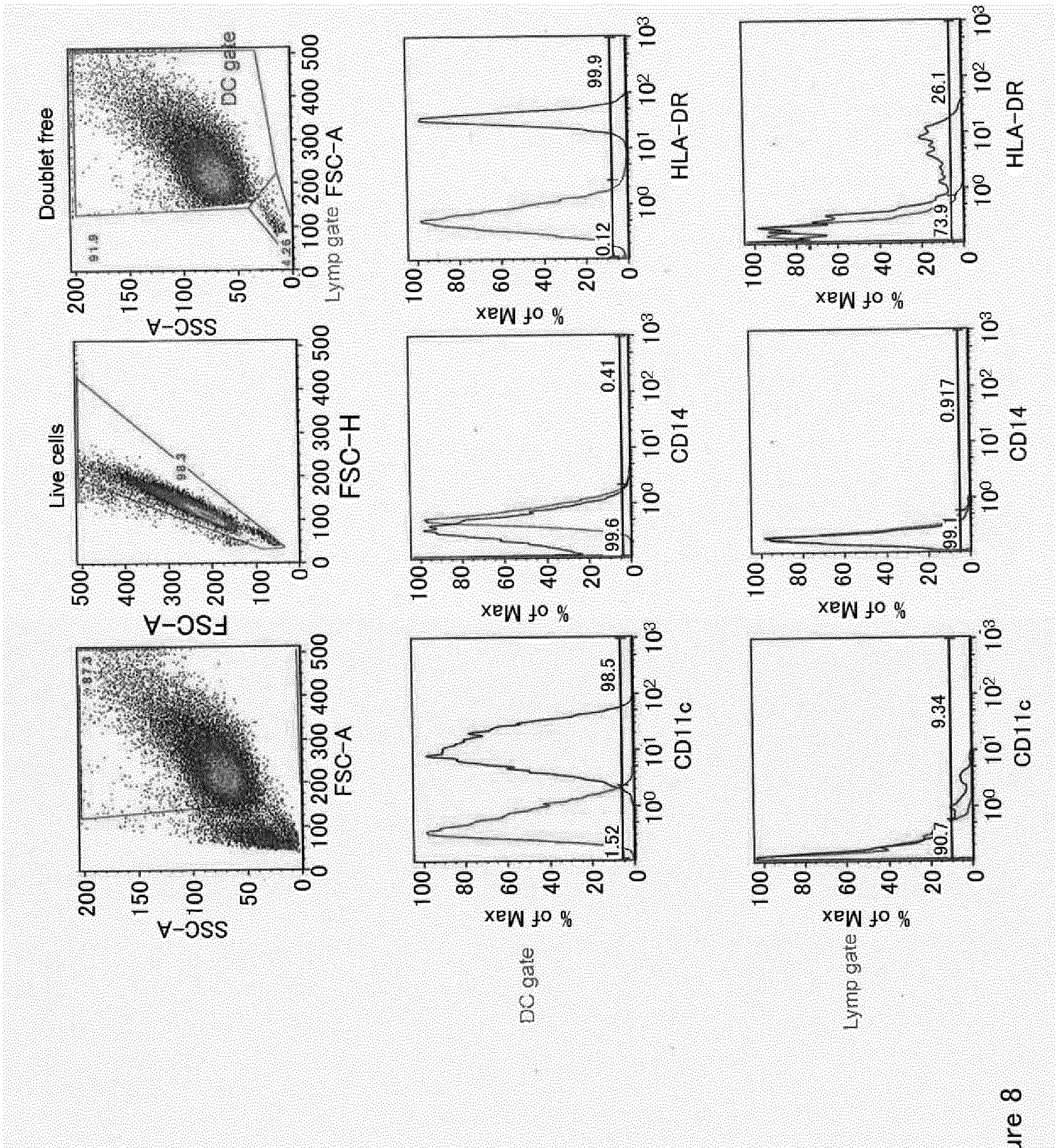
SSC-A
FSC-A
Live cells
FSC-A
FSC-H
98.3
Doublet free
SSC-A
FSC-A
91.9
4.26
DC gate
Lymp gate
DC gate
% of Max
CD11c
1.52
98.5
CD14
99.6
0.41
HLA-DR
0.12
99.9
Lymp gate
% of Max
CD11c
90.7
9.34
CD14
99.1
0.917
HLA-DR
73.9
26.1

Figure 8

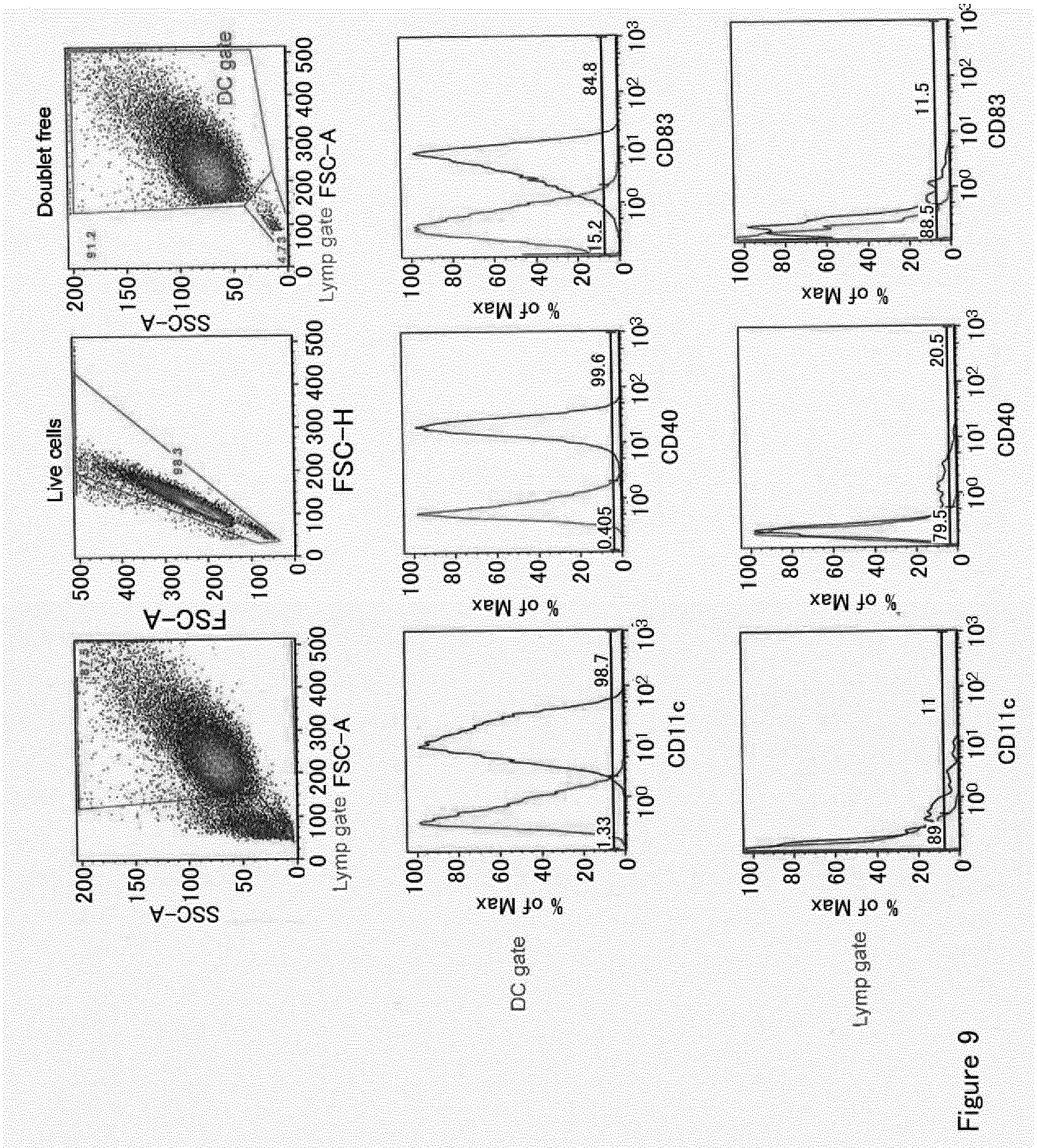
Doublet free
Live cells
DC gate
Lymp gate
SSC-A
FSC-A
FSC-H
91.2
4.73
98.3
87.5
% of Max
CD11c
CD40
CD83
1.33
98.7
0.405
99.6
15.2
84.8
89
11
79.5
20.5
88.5
11.5

Figure 9

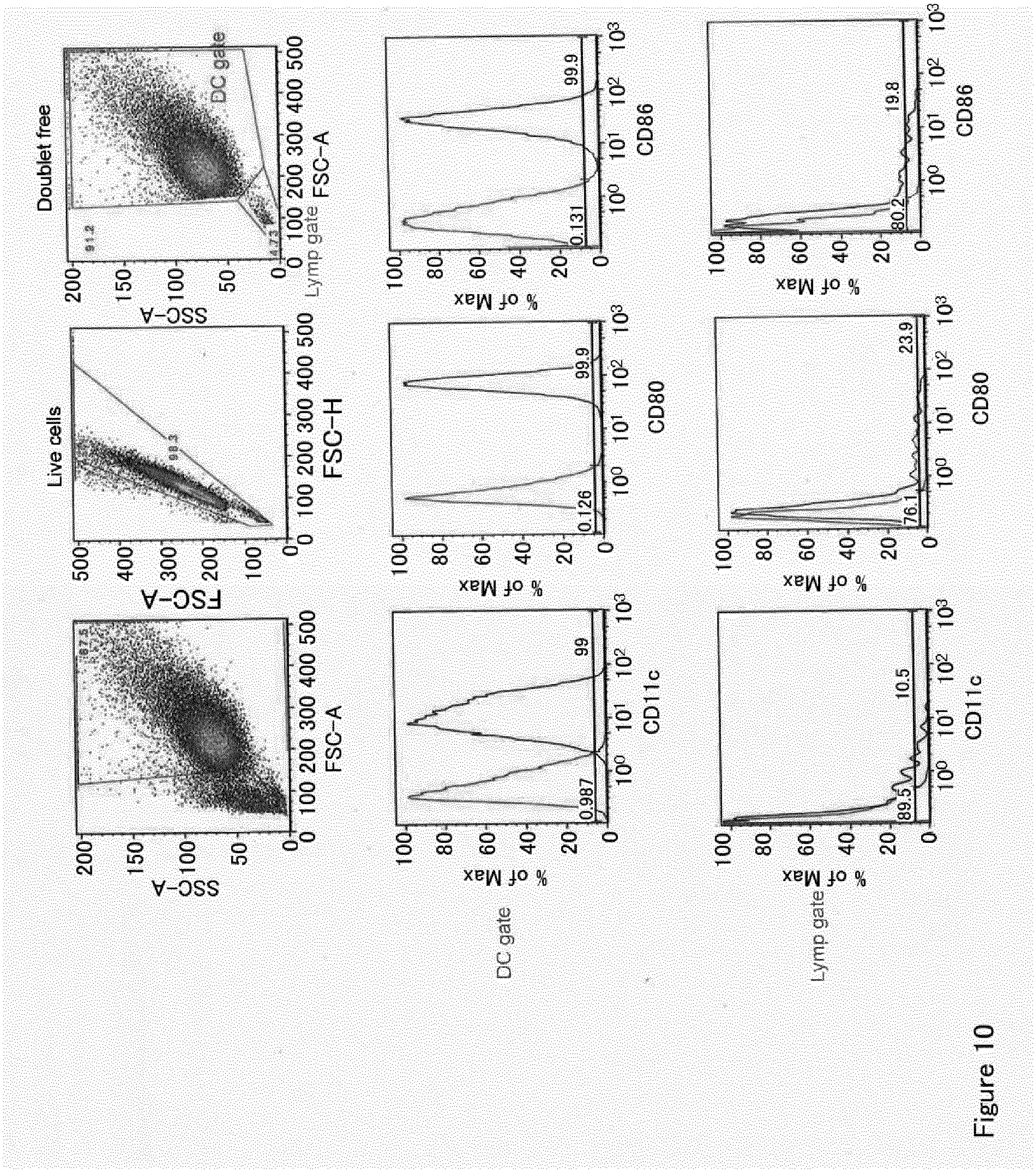
Live cells
Doublet free
DC gate
Lymp gate
SSC-A
FSC-A
FSC-H
% of Max
CD11c
CD80
CD86
98.3
91.2
4.73
0.987
99
0.126
99.9
0.131
99.9
89.5
10.5
76.1
23.9
80.2
19.8

Figure 10

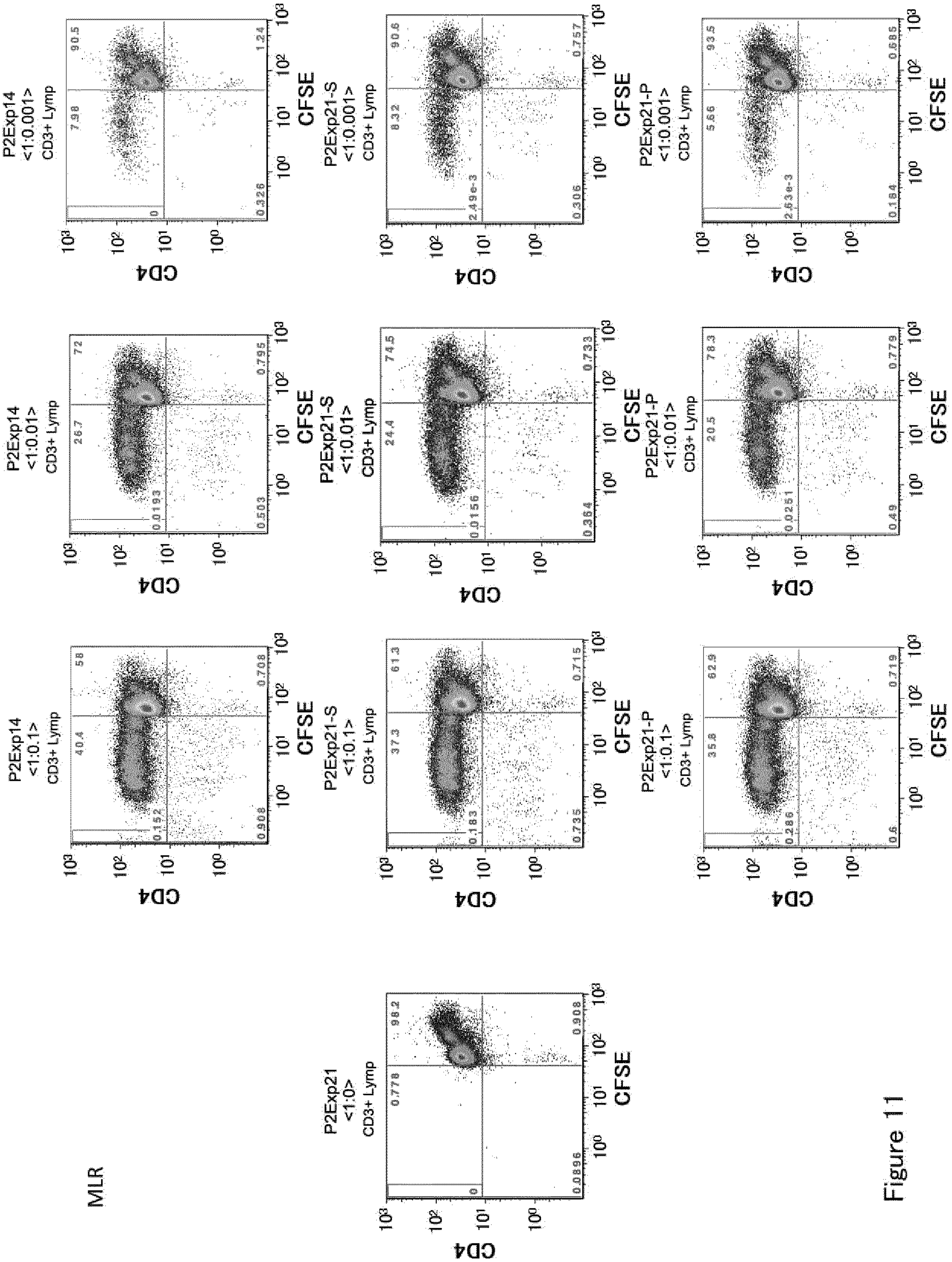
MLR
P2Exp21
<1:0>
CD3+ Lymp
P2Exp14
<1:0.1>
CD3+ Lymp
P2Exp14
<1:0.01>
CD3+ Lymp
P2Exp14
<1:0.001>
CD3+ Lymp
P2Exp21-S
<1:0.1>
CD3+ Lymp
P2Exp21-S
<1:0.01>
CD3+ Lymp
P2Exp21-S
<1:0.001>
CD3+ Lymp
P2Exp21-P
<1:0.1>
CD3+ Lymp
P2Exp21-P
<1:0.01>
CD3+ Lymp
P2Exp21-P
<1:0.001>
CD3+ Lymp
CD4
CFSE

Figure 11

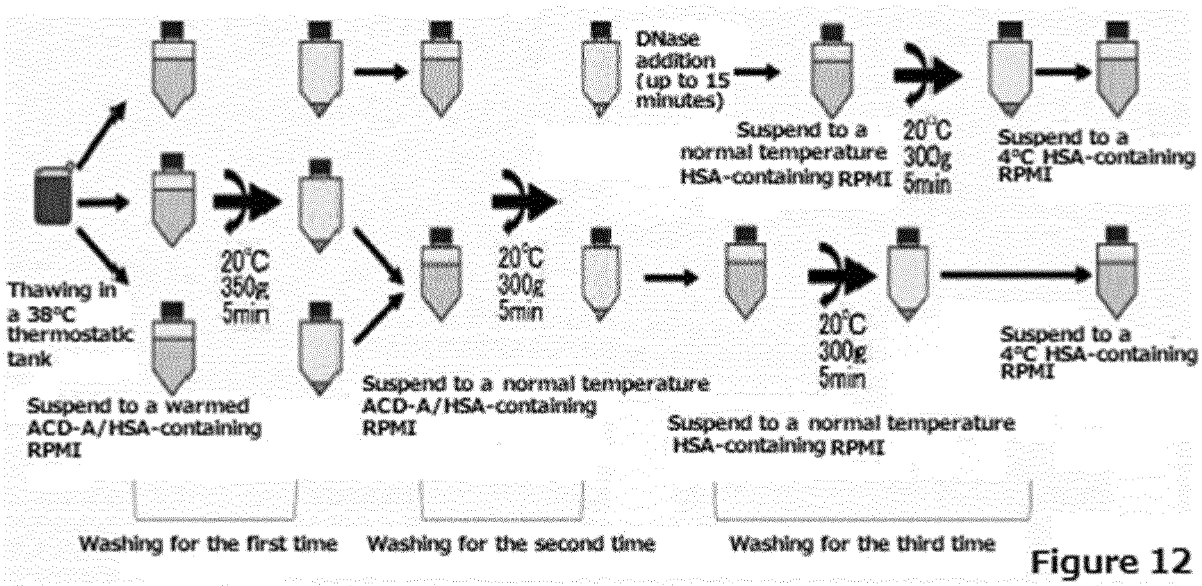


Figure 12

Figure 13

★ Control (isotype)

2 color

| | PE / FL2 | Per-CP / FL4 | |
|---|---|---|---|
| | mIgG2a(k) | mIgG1(k) | |
| | BD Pharm | BD Pharm | |
| | 555574 | 559425 | Stain Buffer |
| | 5 μL | 10 μL | 20 μL |
| 5 tubes ⇒ | 27.5 μL | 55 μL | 110 μL |

➡ Dispensing 35 μL

★ Stain (CD3 / CD14)

2 color

| | PE / FL2 | Per-CP / FL4 | |
|---|---|---|---|
| | CD14 | CD3 | |
| | BD Pharm | BD Pharm | |
| | 555398 | 552851 | Stain Buffer |
| | 5 μL | 10 μL | 20 μL |
| 5 tubes ⇒ | 27.5 μL | 55 μL | 110 μL |

➡ 35 μL dispensatoin

Figure 14

Manufacturing step of a denderitic cell vaccine (comprising ALT-DC-101) (raw material collection to manufacturing Day 0)

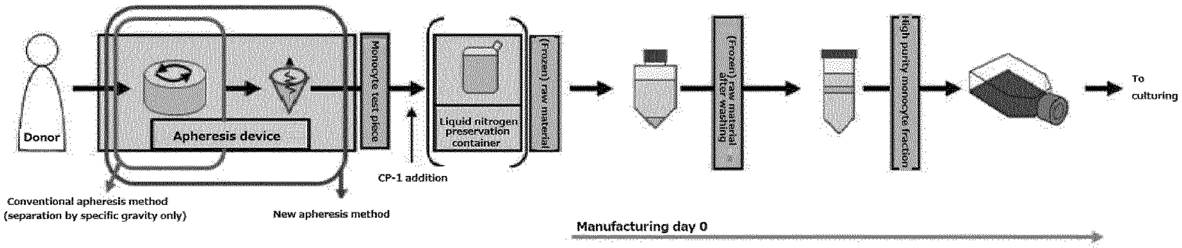


| | | | | | | |
|---|---|---|---|---|---|---|
| Step | Raw material collection step | | Freezing-preserving step | (Thawing) washing step | Specific gravity centrifugal separation step | Adherence treatment step |
| | Separation part by specific gravity | Separation part by specific gravity and size | | | | |
| Main purpose of step | · PBMC collection | · Monocyte enrichment<br>· Removal of lymphocyte and platelet | · Cell freeze-preservation | · Monocyte enrichment*<br>· Removal of lymphocyte*, red blood cell, platelet, dead cell, plasma and freezing-protecting agent | · Monocyte enrichment<br>· Removal of lymphocyte, red blood cell, platelet and dead cell | · Removal of non-adhered cell (lumphocyte and the like) |
| Effect | By collecting a portion where blood cell concentration is low in a buffy coat (set the collection preference high), less red blood cells and neutrophils are mixed in and monocyte concentration can be improved. | A monocyte fraction with a large size is collected by using this separation part. In addition, by causing a spillover even after red blood cell detection, less plateles and lymphocytes are mixed in and monocyte concentration can be improved. | | It is presumed that the specific gravities of a monocyte and a lymphocyte are reversed after adding DMSO, and monocyte concentration can be improved only by centrifugal separation under a specific condition (without using specific gravity liquid). | While the specific gravity of a cell is heavier than normal after adding DMSO, monocyte separation can be carried out by setting high specific gravity for the specific gravity liquid (Optiprep) in the specific gravity centrifugal separation method. | |

*Unexpectedly discovered matter

**INTERNATIONAL SEARCH REPORT**

International application No. PCT/JP2021/006464

A. CLASSIFICATION OF SUBJECT MATTER

C12N 5/0786(2010.01)i; A61K 9/10(2006.01)i; A61K 35/15(2015.01)i; A61K 35/19(2015.01)i; A61K 47/10(2006.01)i; A61K 47/20(2006.01)i; A61K 47/26(2006.01)i; A61K 47/32(2006.01)i; A61K 47/36(2006.01)i; A61P 7/00(2006.01)i; A61P 35/02(2006.01)i; C12M 1/00(2006.01)i; C12M 1/34(2006.01)i; C12M 3/00(2006.01)i; C12N 5/0784(2010.01)i; C12Q 1/04(2006.01)i

FI: C12N5/0786; A61K9/10; A61K35/15; A61K35/15 A; A61K35/15 Z; A61K35/19; A61K47/10; A61K47/20; A61K47/26; A61K47/32; A61K47/36; A61P7/00; A61P35/02; C12M1/00 A; C12M1/34 A; C12M3/00 Z; C12N5/0784; C12Q1/04

According to International Patent Classification (IPC) or to both national classification and IPC

B. FIELDS SEARCHED

Minimum documentation searched (classification system followed by classification symbols)
C12N5/0786; A61K9/10; A61K35/15; A61K35/19; A61K47/10; A61K47/20; A61K47/26; A61K47/32; A61K47/36; A61P7/00; A61P35/02; C12M1/00; C12M1/34; C12M3/00; C12N5/0784; C12Q1/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus/MEDLINE/EMBASE/BIOSIS (STN); Japio-GPG/FX; AGRICOLA (STN); FSTA (STN); SCISEARCH (STN); TOXCENTER (STN)

C. DOCUMENTS CONSIDERED TO BE RELEVANT

| Category* | Citation of document, with indication, where appropriate, of the relevant passages | Relevant to claim No. |
|---|---|---|
| X<br>A | 伊藤誠他，末梢血幹細阻採取における Spectra Optia MNC モードと CMNC モードの後方視的検討，日本輸血細胞治療学会誌，20 December 2018, vol. 64, no. 6, pp. 742-751, doi:10.3925/jjtc.64.742, pp. 742-745, 749, (ITO, Makoto et al., "FUNCTIONAL COMPARISON BETWEEN Spectra Optia® MNC AND CMNC MODES FOR PERIPHERAL BLOOD STEM CELL COLLECTION", Japanese Journal of Transfusion and Cell Therapy) | 1-3, 13, 16-19, 41, 42<br>4-12, 14, 15, 20-40, 43-112 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 April 2021 (21.04.2021) | 11 May 2021 (11.05.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

INTERNATIONAL SEARCH REPORT

International application No. PCT/JP2021/006464

C (Continuation). DOCUMENTS CONSIDERED TO BE RELEVANT

| Category* | Citation of document, with indication, where appropriate, of the relevant passages | Relevant to claim No. |
|---|---|---|
| X<br>A | 末廣陽子，他，ATL に対する Tax 標的樹状細胞ワクチン療法：第 I 相臨床研究長期追跡結果，第 2 回日本 HTLV-1 学会学術集会，14 October 2016, p. 45, 0-44 entire text, non-official translation (SUEHIRO, Yoko et al., "Tax-targeted dendritic cell vaccine therapy with ATL: Long-term follow-up result from the phase 1 clinical trial", Academic Conference of the 2nd Japanese Society of HTLV-1 and Associated Diseases) | 38-40<br>1-37, 41-112 |
| X<br>A | 末廣陽子，Tax 抗原を標的にした成人 T 細胞白血病・リンパ腫に対する樹状細胞ワクチン療法，日本臨牀，February 2017, vol. 75, no. 2, pp. 295-300, pp. 295-299, (SUEHIRO, Yoko, "Tax-targeted DC vaccine for ATL", NIPPON RINSHO) | 38-40<br>1-37, 41-112 |
| X<br>A | 末廣陽子，成人 T 細胞白血病／リンパ腫に対する新規治療アプローチ －Tax 標的樹状細胞ワクチン療法（第 1 相試験），医学のあゆみ，30 November 2019, vol. 271, no. 9, pp. 923-928, pp. 923-928, non-official translation (SUEHIRO, Yoko, "New therapeutic approach to adult T-cell leukemia and lymphoma - Tax-targeted dendritic cell vaccine therapy (phase 1 study)", Journal of clinical and experimental medicine) | 38-40<br>1-37, 41-112 |
| A | JP 4-028369 A (ASAHI MEDICAL KK) 30 January 1992 (1992-01-30) entire text, all drawings | 1-112 |

Form PCT/ISA/210 (continuation of second sheet) (January 2015)

**INTERNATIONAL SEARCH REPORT**
Information on patent family members

International application No.
PCT/JP2021/006464

| Patent Documents referred in the Report | Publication Date | Patent Family | Publication Date |
|---|---|---|---|
| JP 4-028369 A | 30 Jan. 1992 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008022702 A
- JP 2014133712 A
- WO 2004092373 A
- JP 2020027595 A
- JP 2020081523 A


### Non-patent literature cited in the description


- **MIYAZAKI et al.** *Blood,* 2013, vol. 121, 4894-4901
- **CANCER.** *Science,* 2012, vol. 103, 150-153